# EUROPEAN PATENT SPECIFICATION

(11) **EP 0 388 232 B1**
(45) Date of publication and mention of the grant of the patent: **12.01.2005**
(21) Application number: 90302866.0
(22) Date of filing: 16.03.1990
(51) Int. Cl.: C12N 15/51, A61K 39/29, G01N 33/576, C12Q 1/70

(54) **NANBV diagnostics and vaccines**
NANBV-Diagnostika und Vakzine
Diagnostics et vaccins de NANBV

(30) Priority: 17.03.1989 US 325338; 20.04.1989 US 341334; 18.05.1989 US 355002
(43) Date of publication of application: 19.09.1990
(60) Divisional application: 00109602.3
(73) Proprietor: CHIRON CORPORATION, Emeryville, California 94608 (US)
(72) Inventor: Houghton, Michael, Danville, California 94526 (US); Choo, Qui-Lim, El Cerrito, California 94530 (US); Kuo, George, San Francisco, California 94122 (US)
(74) Representative: Voelker, Ingeborg Carla Emmy

(56) References cited:
- EP-A- 0 293 274
- EP-A- 0 318 216
- WO-A-90/00597
- CHEMICAL ABSTRACTS, vol. 112, no. 1, 01 January 1990, Columbus, OH (US); T. ARIMA et al., p. 209, no. 1980n
- CHEMICAL ABSTRACTS, vol. 112, no. 7, 12 February 1990, Columbus, OH (US); T. ARIMA et al., p. 169, no. 49584p
- CHEMICAL ABSTRACTS, vol. 112, no. 11, 12 March 1990, Columbus, OH (US); T. ARIMA et al., p. 441, no. 95311v
- NUCLEIC ACIDS RESEARCH, vol. 17, no. 24, 1989, IRL Press; Y. KUBO et al., pp. 10367-10372

## Description

### Technical Field

The invention relates to materials and methodologies for managing the spread of non-A, non-B hepatitis virus (NANBV) infection. More specifically, it relates to polynucleotides derived from the genome of an etiologic agent of NANBH, hepatitis C virus (HCV), to polypeptides encoded therein, and to antibodies directed to the polypeptides. These reagents are useful as screening agents for HCV and its infection, and as protective agents against the disease.

### References Cited in the Application

Barr et al. (1986), Biotechniques 4:428.
Botstein (1979), Gene 8:17.
Brinton, M.A. (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press), p.327-374.
Broach (1981) in: Molecular Biology of the Yeast Saccharomyces, Vol. 1, p.445, Cold Spring Harbor Press.
Broach et al. (1983), Meth. Enz. 101:307.
Chang et al. (1977), Nature 198:1056.
Chirgwin et al. (1979), Biochemistry 18:5294.
Chomczynski and Sacchi (1987), Analytical Biochemistry 162:156.
Clewell et al. (1969), Proc. Natl. Acad. Sci. USA 62:1159. Clewell (1972), J. Bacteriol. 110:667.
Cohen (1972), Proc. Natl. Acad. Sci. USA 69:2110.
Cousens et al. (1987), gene 61:265.
De Boer et al. (1983), Proc. Natl. Acad. Sci. USA 292:128. Dreesman et al. (1985), J. Infect. Disease 151:761. Feinstone, S.M. and Hoofnagle, J.H. (1984), New Engl. J. Med. 311:185.
Fields & Knipe (1986), FUNDAMENTAL VIROLOGY (Raven Press, N.Y.).
Fiers et al. (1978), Nature 273:113.
Gerety, R.J. et al., in VIRAL HEPATITIS AND LIVER DISEASE (Vyas, B.N., Dienstag, J.L., and Hoofnagle, J.H., eds,
Grune and Stratton, Inc., 1984) pp 23-47.
Goeddel et al. (1980), Nucleic Acids Res. 8:4057.
Graham and Van der Eb (1978), Virology 52:546.
Grunstein and Hogness (1975), Proc. Natl. Acad. Sci. USA 73:3961.
Grych et al. (1985), Nature 316:74.
Gubler and Hoffman (1983), Gene 25:263.
Hahn (1988) Virology 162:167.
Hammerling et al. (1981), MONOCLONAL ANTIBODIES AND T-CELL HYBRIDOMAS.
Han (1987), Biochemistry 26:1617.
Helfman (1983), Proc. Natl. Acad. Sci. USA 80:31.
Hess et al. (1968), J. Adv. Enzyme Reg 7:149.
Hinnen et al. (1978), Proc. Natl. Acad. Sci. 75:1929. Hitzeman et al. (1980), J. Biol. Chem. 255:2073.
Holland et al. (1978), Biochemistry 17:4900.
Holland (1981), J. Biol. Chem. 256: 1385.
Houghton et al. (1981), Nucleic Acids Res. 9:247
Hunyh, T.V. et al. (1985) in DNA CLONING TECHNIQUES; A PRACTICAL APPROACH (D. Glover, Ed., IRL Press, Oxford, U.K.) pp. 49-78.
Immun. Rev. (1982) 62:185.
Iwarson (1987), British Medical J. 295:946.
Kennett et al. (1980) MONOCLONAL ANTIBODIES.
Kyte and Doolittle (1982), J. Mol. Biol. 157:105-132.
Laemmli (1970), Nature 227, 680.
Lee et al. (1988), Science 239:1288.
Maniatis, T., et al. (1982) MOLECULAR CLONING; A LABORATORY MANUAL (Cold Spring Harbor Press, Cold Spring Harbor, N.Y.).
Mayer and Walker, eds. (1987), IMMUNOCHEMICAL METHODS IN CELL AND MOLECULAR BIOLOGY (Academic Press, London).
Maxam et al. (1980), Methods in Enzymology 65:499. MacNamara et al. (1984), Science 226:1325.
Messing et al. (1981), Nucleic Acids Res. 9:309.
Messing (1983), Methods in Enzymology 101:20-37.
METHODS IN ENZYMOLOGY (Academic Press).
Michelle et al., Int. Symposium on Viral Hepatitis.
Monath (1986) in THE VIRUSES: THE TOGAVIRADAE AND FLAVIVIRIDAE (Series eds. Fraenkel-Conrat and Wagner, vol. eds. Schlesinger and Schlesinger, Plenum Press), p.375-440.
Nagahuma et al. (1984), Anal. Biochem. 141:74.
Neurath et al. (1984), Science 224:392.
Nisonoff et al. (1981), Clin. Immunol. Immunopathol. 21:397-406.
Overby, L.R. (1985), Curr. Hepatol. 5:49.
Overby, L.R. (1986), Curr. Hepatol. 6:65.
Overby, L.R. (1987), Curr. Hepatol. 7:35.
Peleg (1969), Nature 221:193.
Pfefferkorn and Shapiro (1974), in COMPREHENSIVE VIROLOGY, Vol. 2 (Fraenkel-Conrat & Wagner, eds., Plenum, N.Y.) pp. 171-230.
Prince, A.M. (1983), Annu. Rev. Microbiol. 37:217.
Rice et al. (1985), Science 229:726.
Rice et al. (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press), p.279-328.
Roehrig (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press)
Sadler et al. (1980), Gene 8, 279.
Saiki et al. (1986), Nature 324: 163.
Saiki et al. (1988), Science 239:487.
Sanger et al. (1977), Proc. Natl. Acad. Sci. USA 74:5463.
Schlesinger et al. (1986), J. Virol. 60:1153.
Schreier, M., et al. (1980) HYBRIDOMA TECHNIQUES
Scopes (1984), PROTEIN PURIFICATION, PRINCIPLES AND
PRACTICE, SECOND EDITION (Springer-Verlag, N.Y.).
Shimatake et al. (1981), Nature 292:128.
Sippel (1973), Eur. J. Biochem. 37:31.
Steimer et al. (1986), J. Viral. 58:9.
Stollar (1980), in THE TOGAVIRUSES (R.W. Schlesinger, ed., Academic Press, N.Y.), pp. 584-622.
Sumiyoshi et al. (1987), Virology 161:497.
Taylor et al. (1976), Biochem. Biophys. Acta 442:324.
Towbin et al. (1979), Proc. Natl. Acad. Sci. USA 76, 4350.
Tsu and Herzenberg (1980), in SELECTED METHODS IN CELLULAR IMMUNOLOGY (W.H. Freeman and Co.) pp. 373-391.
Vytdehaag et al. (1985), J. Immunol. 134:1225.
Valenzuela, P., et al. (1982), Nature 298:344.
Valenzuela, P., et al. (1984), in HEPATITIS B (Millman, I., et al., ed, Plenum Press) pp. 225-236.
Warner (1984), DNA 3:401.
Wu and Grossman (1987), Methods in Enzymology Vol. 154, RECOMBINANT DNA, Part E.
Wu (1987), Methods in Enzymology vol 155, RECOMBINANT DNA, part F.
Zoller (1982), Nucleic Acids Res. 10:6487.

### Cited Patents

EPO Pub. No. 318,216
PCT Pub. No. WO 89/04669
U.S. Patent No. 4,341,761
U.S. Patent No. 4,399,121
U.S. Patent No. 4,427,783
U.S. Patent No. 4,444,887
U.S. Patent No. 4,466,917
U.S. Patent No. 4,472,500
U.S. Patent No. 4,491,632
U.S. Patent No. 4,493,890

### Background Art

Non-A, Non-B hepatitis (NANBH) is a transmissible disease or family of diseases that are believed to be viral-induced, and that are distinguishable from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). NANBH was first identified in transfused individuals. Transmission from man to chimpanzee and serial passage in chimpanzees provided evidence that NANBH is due to a transmissible infectious agent or agents.

Epidemiologic evidence is suggestive that there may be three types of NANBH: the water-borne epidemic type; the blood or needle associated type; and the sporadically occurring (community acquired) type. However, the number of agents which may be the causative of NANBH are unknown.

Clinical diagnosis and identification of NANBH has been accomplished primarily by exclusion of other viral markers. Among the methods used to detect putative NANBV antigens and antibodies are agar-gel diffusion, counterimmunoelectrophoresis, immunofluorescence microscopy, immune electron microscopy, radioimmunoassay, and enzyme-linked immunosorbent assay. However, none of these assays has proved to be sufficiently sensitive, specific, and reproducible to be used as a diagnostic test for NANBH.

Previously there was neither clarity nor agreement as to the identity or specificity of the antigen antibody systems associated with agents of NANBH. This was due, at least in part, to the prior or co-infection of HBV with NANBV in individuals, and to the known complexity of the soluble and particulate antigens associated with HBV, as well as to the integration of HBV DNA into the genome of liver cells. In addition, there is the possibility that NANBH is caused by more than one infectious agent, as well as the possibility that NANBH has been mis-diagnosed. Moreover, it is unclear what the serological assays detect in the serum of patients with NANBH. It has been postulated that the agar-gel diffusion and counterimmunoelectrophoresis assays detect autoimmune responses or nonspecific protein interactions that sometimes occur between serum specimens, and that they do not represent specific NANBV antigen-antibody reactions. The immunofluorescence, and enzyme-linked immunosorbent, and radioimmunoassays appear to detect low levels of a rheumatoid-factor-like material that is frequently present in the serum of patients with NANBH as well as in patients with other hepatic and nonhepatic diseases. Some of the reactivity detected may represent antibody to host-determined cytoplasmic antigens.

There have been a number of candidate NANBV. See, for example the reviews by Prince (1983), Feinstone and Hoofnagle (1984), and Overby (1985, 1986, 1987) and the article by Iwarson (1987). However, there is no proof that any of these candidates represent the etiological agent of NANBH.

The demand for sensitive, specific methods for screening and identifying carriers of NANBV and NANBV contaminated blood or blood products is significant. Post-transfusion hepatitis (PTH) occurs in approximately 10% of transfused patients, and NANBH accounts for up to 90% of these cases. The major problem in this disease is the frequent progression to chronic liver damage (25-55%).

Patient care as well as the prevention of transmission of NANBH by blood and blood products or by close personal contact require reliable screening, diagnostic and prognostic tools to detect nucleic acids, antigens and antibodies related to NANBV. In addition, there is also a need for effective vaccines and immunotherapeutic therapeutic agents for the prevention and/or treatment of the disease.

Applicant discovered a new virus, the Hepatitis C virus (HCV), which has proven to be the major etiologic agent of blood-borne NANBH (BB-NANBH). Applicant's initial work, including a partial genomic sequence of the prototype HCV isolate, CDC/HCV1 (also called HCV1), is described in EPO Pub. No. 318,216 (published 31 May 1989) and PCT Pub. No. WO 89/04669 (published 1 June 1989). The disclosures of these patent applications, as well as any corresponding national patent applications, are incorporated herein by reference. These applications teach, inter alia, recombinant DNA methods of cloning and expressing HCV sequences, HCV polypeptides, HCV immunodiagnostic techniques, HCV probe diagnostic techniques, anti-HCV antibodies, and methods of isolating new hCV sequences, including sequences of new HCV isolates.

### Disclosure of the Invention

The present invention is based, in part, on new HCV sequences and polypeptides that are not disclosed in EPO Pub. No. 318,216, or in the PCT Pub. No. WO 89/04669. Included within the invention is the application of these new sequences and polypeptides in, inter alia, immunodiagnostics, probe diagnostics, anti-HCV antibody production, PCR technology and recombinant DNA technology. Included within the invention, also, are new immunoassays based upon the immunogenicity of HCV polypeptides disclosed herein. The new subject matter claimed herein, while developed using techniques described in, for example EPO Pub. No. 318,216, has a priority date which antecedes that publication, for any counterpart thereof. Thus, the invention provides novel compositions and methods useful for screening samples for HCV antigens and antibodies, and useful for treatment of HCV infections.

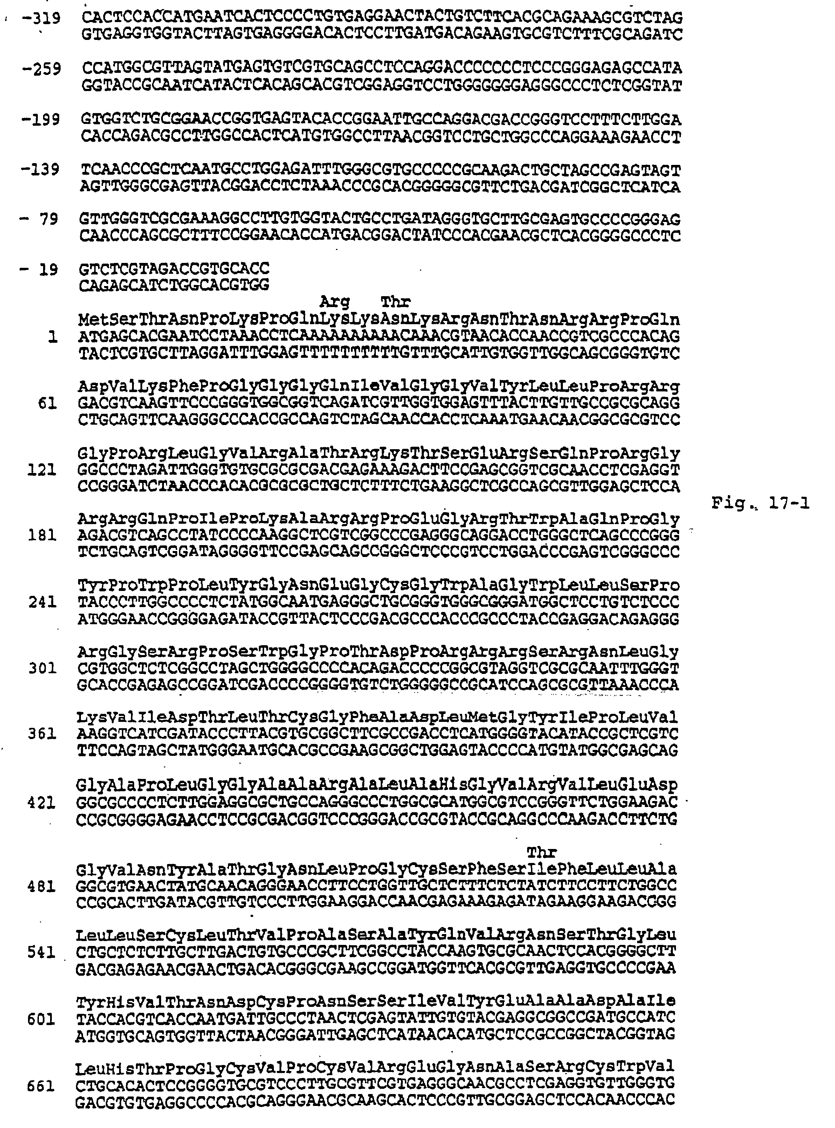

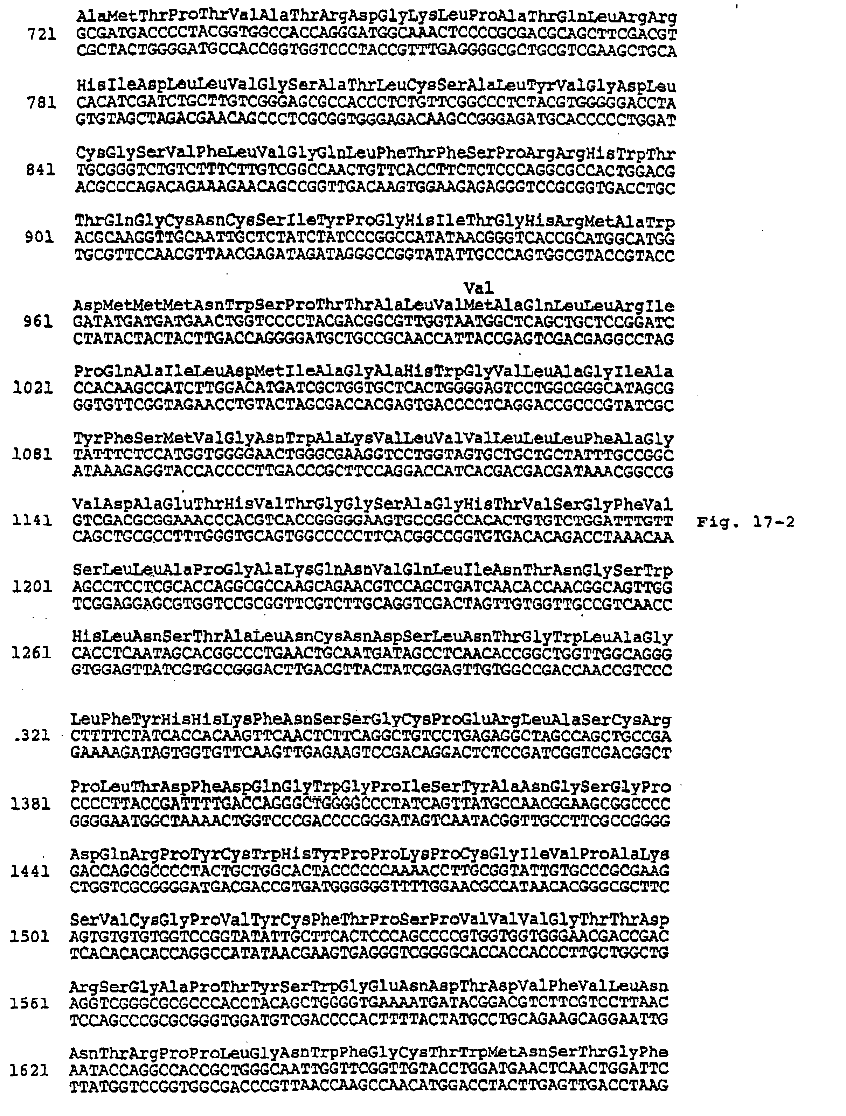

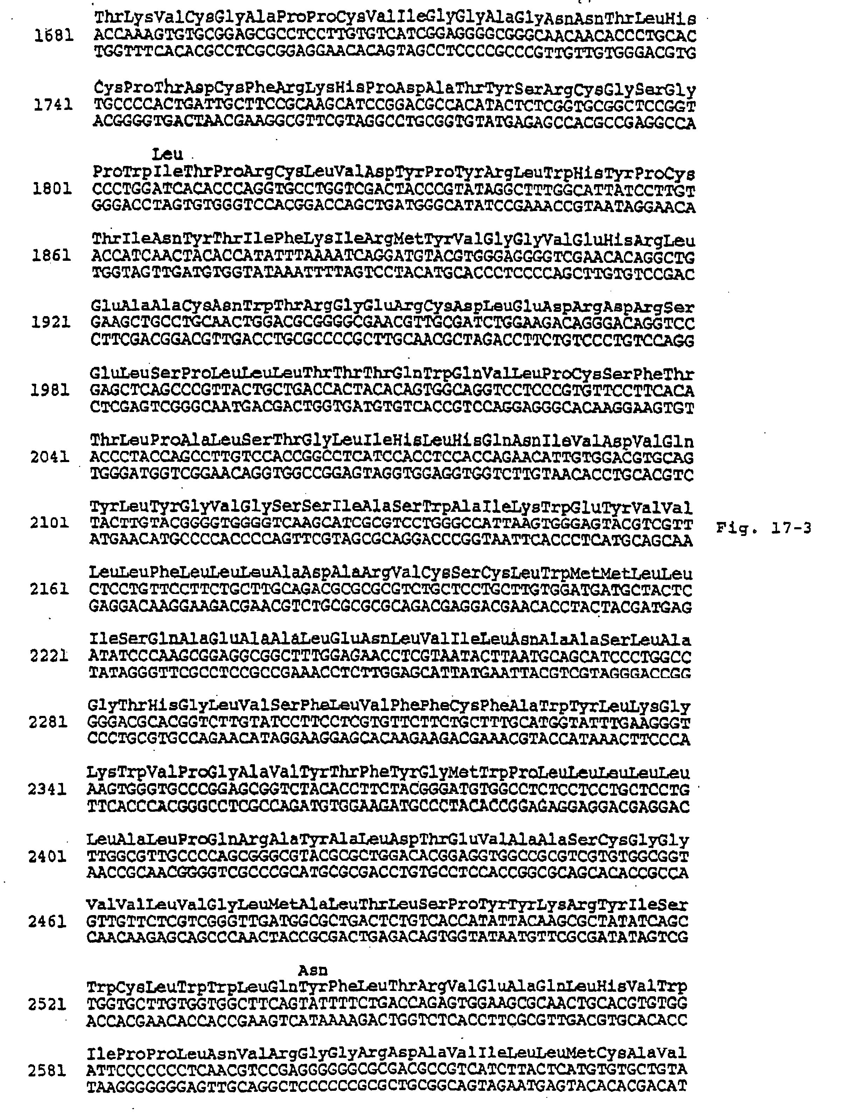

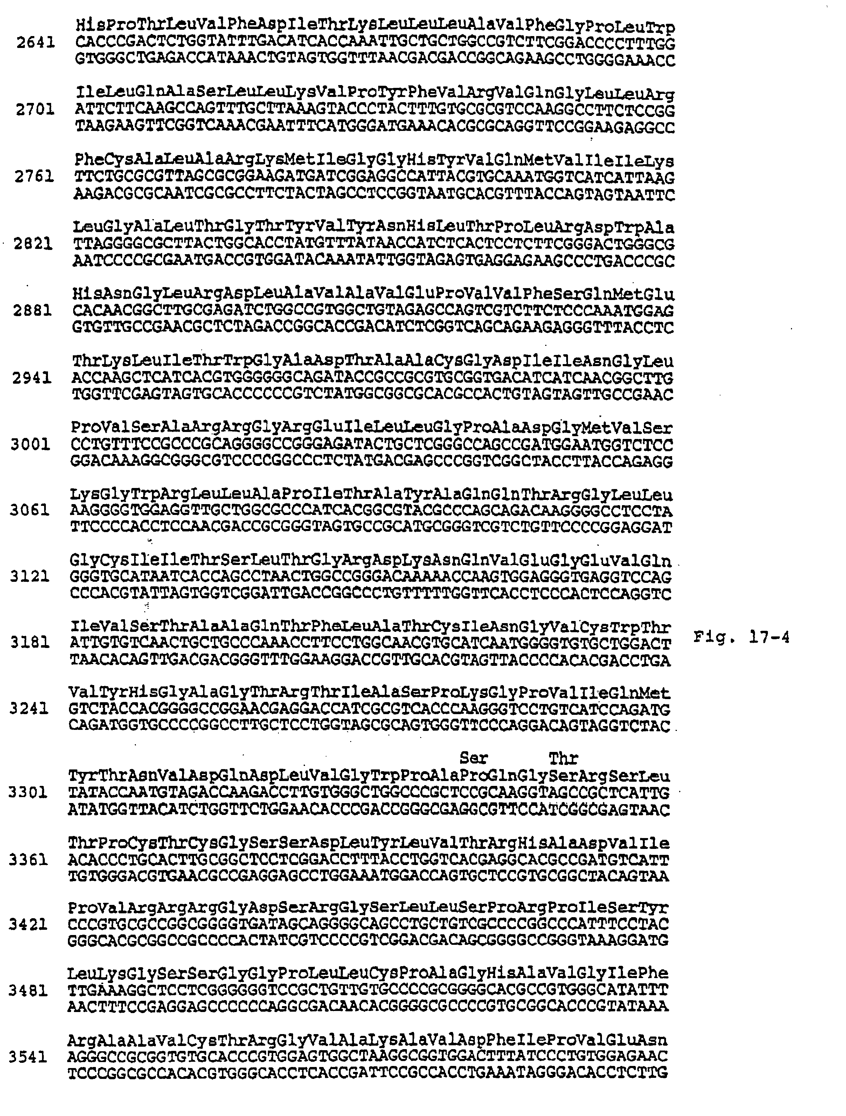

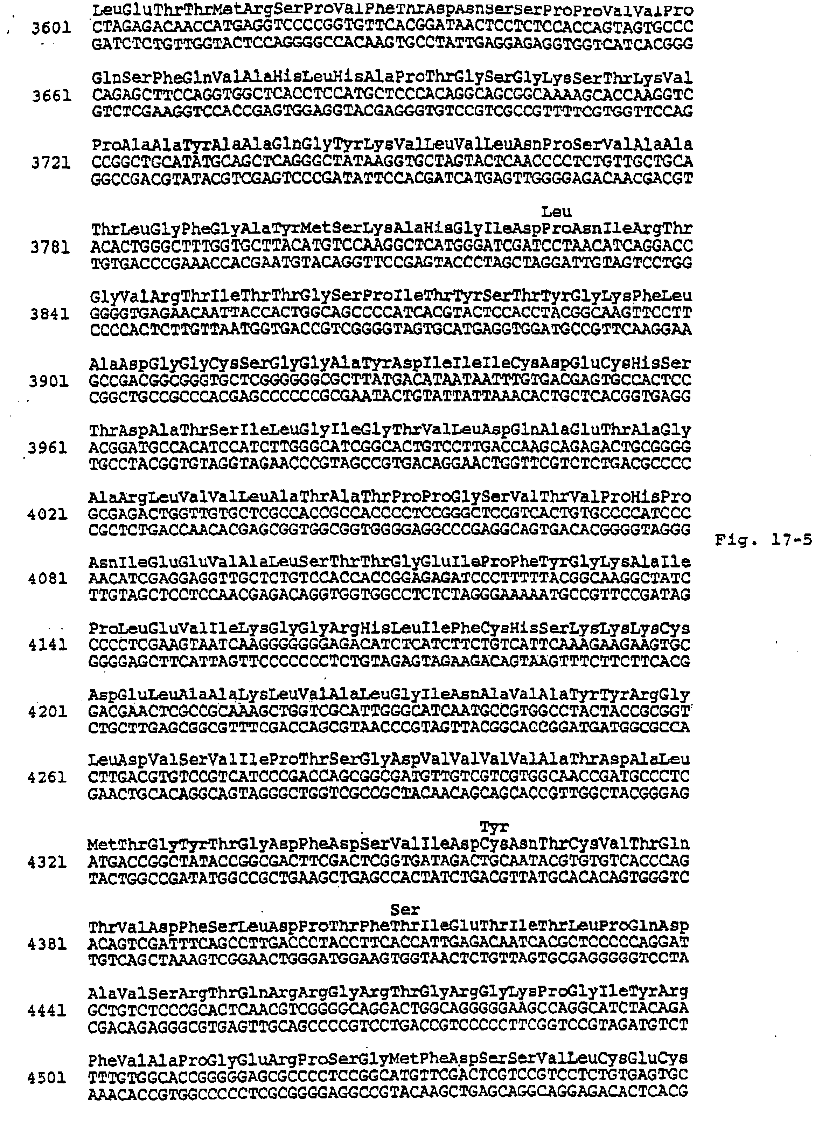

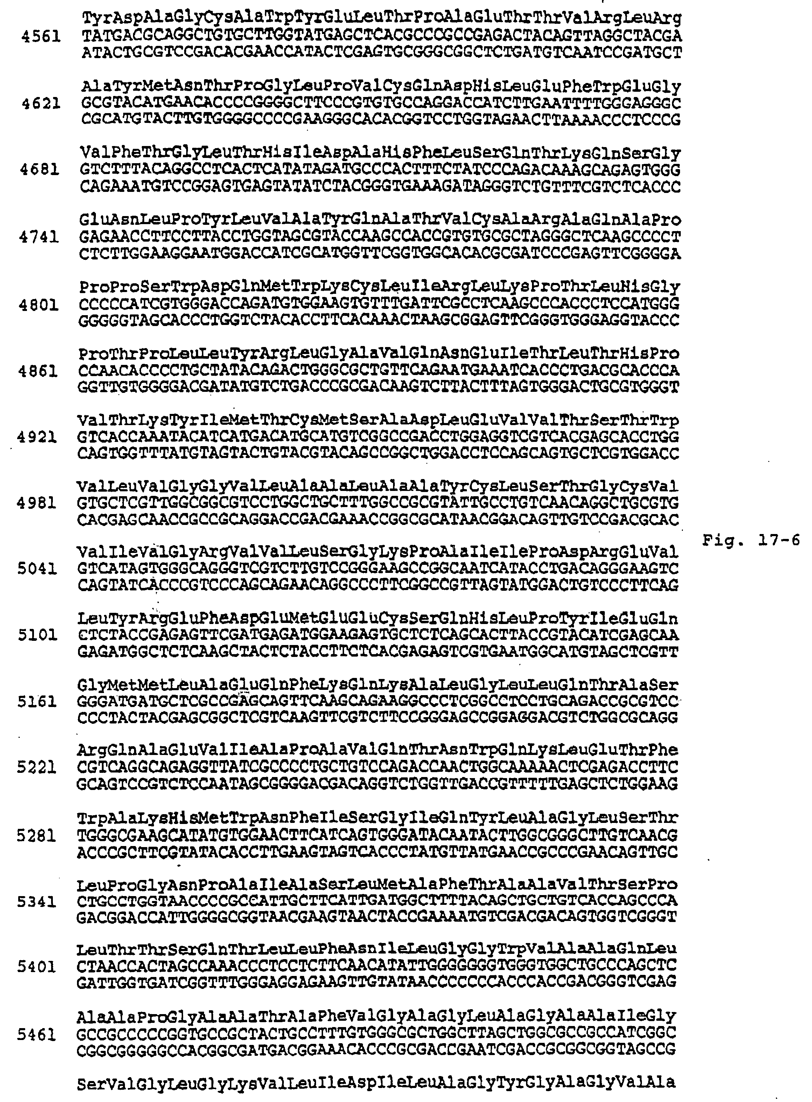

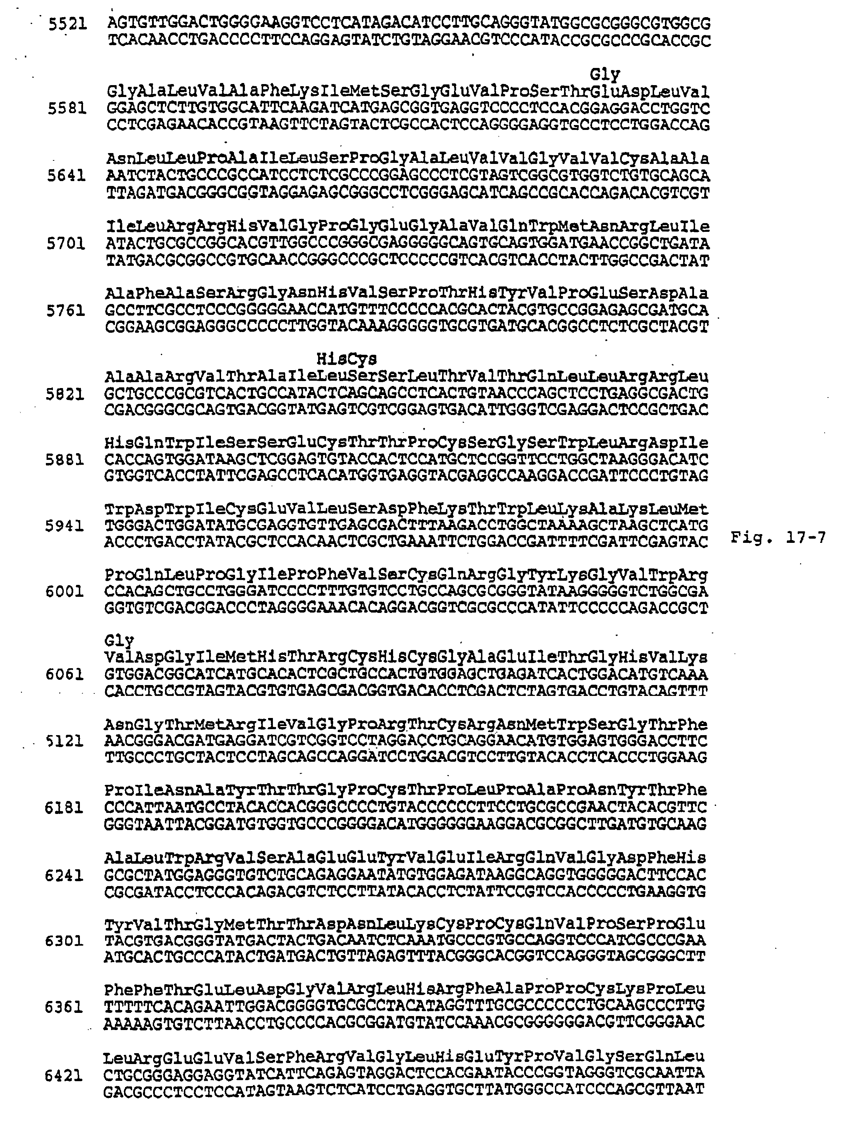

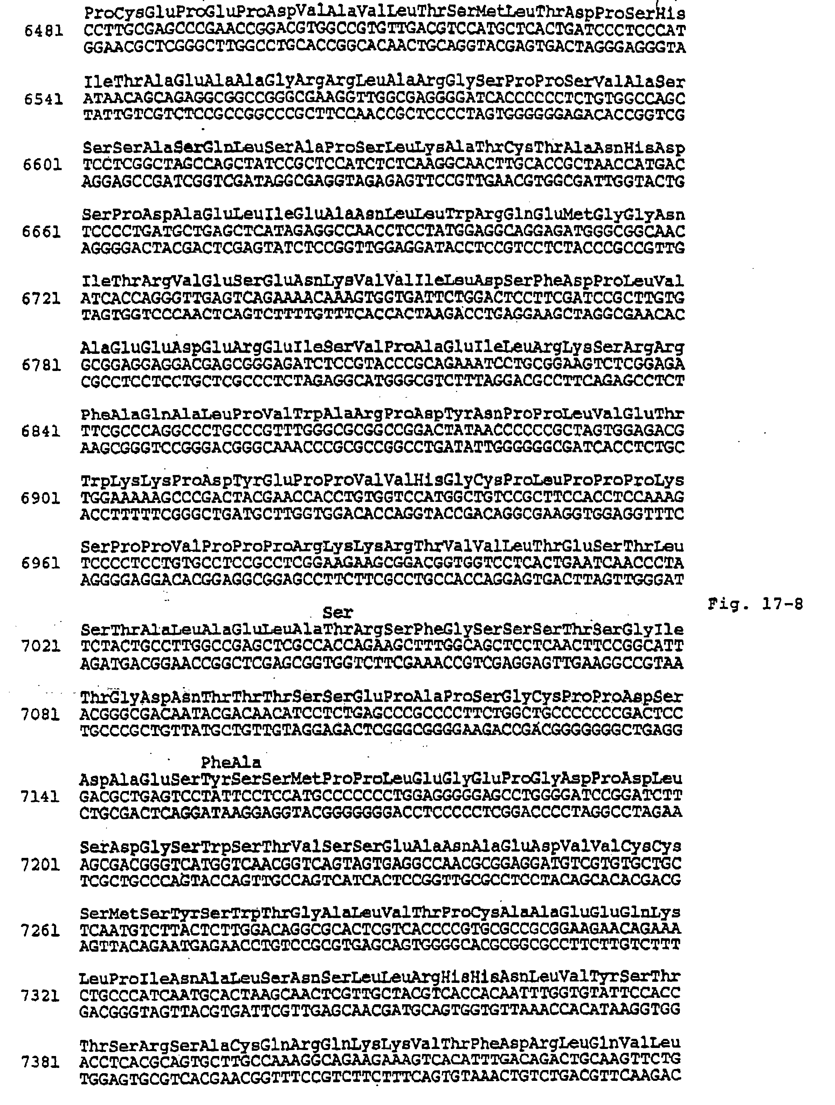

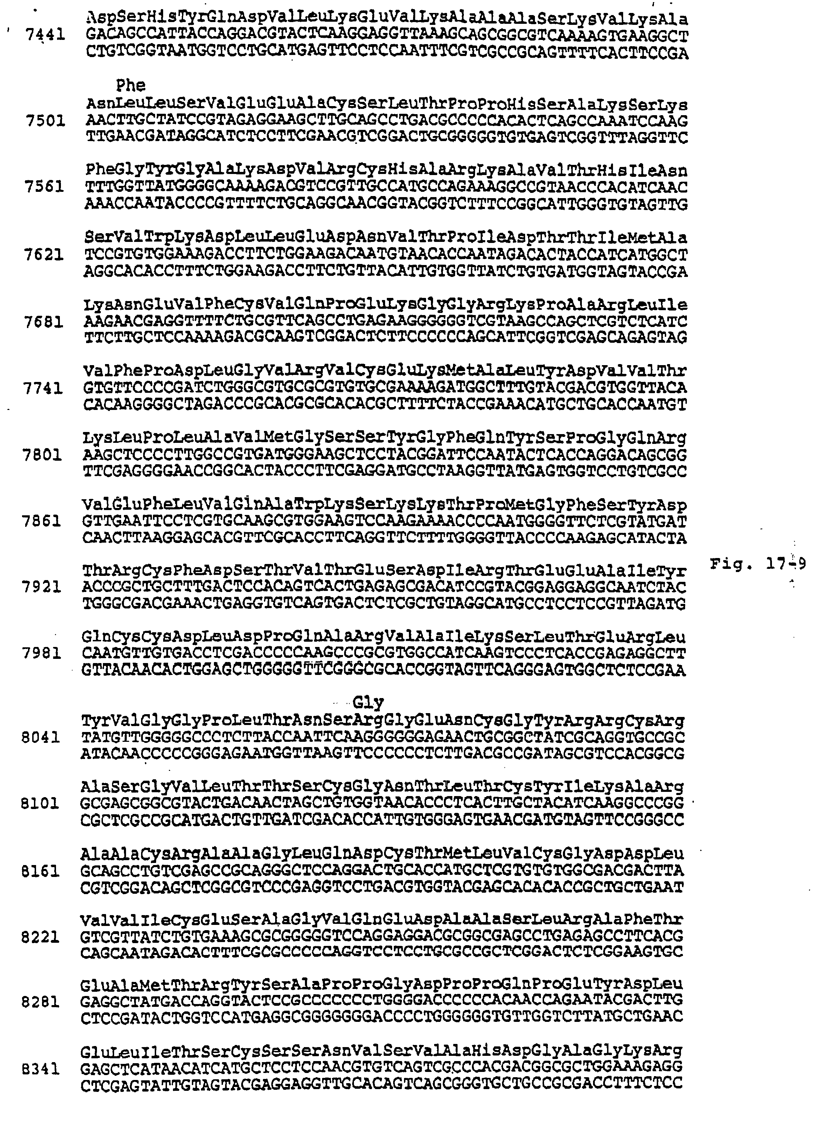

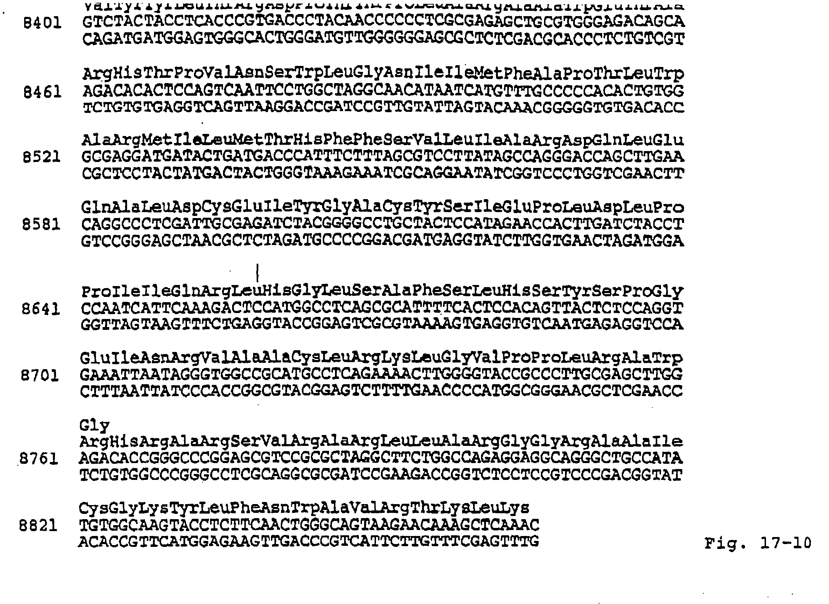

Accordingly, one aspect of the invention is based on characterisation of recombinant polynucleotides comprising sequences derived from HCV cDNA, wherein the HCV cDNA is in clone 13i, or clone 26j, or clone 59a, or clone 84a, or clone CA156e, or clone 167b, or clone pi14a, or clone CA216a, or clone CA290a, or clone ag30a, or clone 205a, or clone 18g, or clone 16jh, or wherein the HCV cDNA is of a sequence indicated by nucleotide numbers -319 to 1348 in Figure 17.

Another aspect of the invention is based on characterisation of purified polypeptides comprising an epitope encoded within HCV cDNA wherein the HCV cDNA is of a sequence indicated by nucleotide numbers -319 to 1348 in Figure 17.

Accordingly the present invention provides a polypeptide in substantially isolated form comprising a contiguous sequence from a Hepatitis C virus (HCV) polyprotein of at least 10 amino acids wherein said contiguous sequence is from amino acids 1 to 449 of an HCV polyprotein, wherein HCV is characterised by:
a. positive stranded RNA genome;
   said genome comprising an open reading frame (ORF) encoding a polyprotein; and
   the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

Preferably, the contiguous sequence is:
a. HCV amino acids 1 to 84;
b. HCV amino acids 9 to 177;
c. an immunologically reactive fragment of (a) or (b) above.

The immunologically reactive fragment may be from amino acids 1 to 50, 35 to 45, 50 to 100, 40 to 90, 65 to 75, 80 to 90, 99 to 120, 95 to 110 or 100 to 150 of the HCV polyprotein.

Yet another aspect of the invention relates to immunogenic polypeptides produced by a cell transformed with a recombinant expression vector comprising an ORF of DNA derived from HCV cDNA, wherein the HCV cDNA is comprised of a sequence derived from the HCV cDNA sequence in clone CA279a, or clone CA74a, or clone 13i, or clone CA290a, or clone 33C or clone 40b, or clone 33b, or clone 25c, or clone 14c, or clone 8f, or clone 33f, or clone 33g, or clone 39c, or clone 15e, and wherein the ORF is operably linked to a control sequence compatible with a desired host.

Another aspect of the invention is a polypeptide wherein the contiguous sequence has the formula

AAₓ-AA_{y}

wherein x and y designate amino acid numbers shown in Figure 17, and wherein the peptide is selected from the group consisting of AA1-AA35, AA1-AA50, AA1-AA84, AA9-AA177, AA1-AA10, AA5-AA20, AA20-AA25, AA35-AA45, AA50-AA100, AA40-AA90, AA45-AA65, AA65-AA75, AA80-AA90, AA99-AA120, AA95-AA110, AA105-AA120, AA100-AA150, AA150-AA200, AA155-AA170, AA190-AA210, AA200-AA250, AA220-AA240, AA245-AA265, AA250-AA300, AA290-AA330, AA290-305, AA300-AA350, AA310-AA330, AA350-AA400, AA380-AA395, AA405-AA495, AA400-AA450, AA405-AA415, AA415-AA425, AA425-AA435, AA437-AA582, AA440-AA460.

The invention further relates to polynucleotide probes for HCV, in particular probes comprised of an HCV sequence derived from an HCV cDNA sequence indicated by nucleotide numbers -319 to 1348 in Figure 17, or from the complement of the HCV cDNA sequence. Thus the invention provides a recombinant or purified viral polynucleotide which comprises an HCV genomic sequence or the complement thereof which is selectively hybridizable to nucleotides -319 to 1348 of the HCV genome or the complement thereof.

The invention also provides a kit for analysing samples for the presence of polynucleotides from HCV which kits comprise a polynucleotide probe of the invention in a suitable container.

Another aspect of the invention is a kit for analysing samples for the presence of an HCV antigen comprising an antibody which reacts immunologically with an HCV antigen of the invention, such as an antigen containing an epitope encoded within HCV cDNA which is of a sequence indicated by nucleotide numbers -319 to 1348 in Figure 17, or wherein the HCV cDNA is in clone 13i, or clone 26j, or clone 59a, or clone 84a, or clone CA156e, or clone 167b, or clone pi14a, or clone CA216a, or clone CA290a, or clone ag30a, or clone 205a, or clone 18g, or clone 16jh.

Yet another aspect of the invention is a kit for analysing samples for the presence of an HCV antibody comprising an antigenic polypeptide containing an HCV epitope of the invention such as an epitope encoded within HCV cDNA which is of a sequence indicated by nucleotide numbers -319 to 1348 in Figure 17, or is in clone 13i, or clone 26j, or clone 59a, or clone 84a, or clone CA156e, or clone 167b, or clone pi14a, or clone CA 216a, or clone CA 290a, or clone ag30a, or clone 205a, or clone 18g, or clone 16jh.

Another aspect of the invention is a kit for analysing samples for the presence of an HCV antibody comprising an antigenic polypeptide of the invention such as polypeptide expressed from HCV cDNA in clone CA279a, or clone CA74a, or clone 13i, or clone CA290a, or clone 33C or clone 40b, or clone 33b, or clone 25c, or clone 14c, or clone 8f, or clone 33f, or clone 33g, or clone 39c, or clone 15e, wherein the antigenic polypeptide is present in a suitable container.

Still another aspect of the invention is a method for detecting HCV nucleic acids in a sample comprising:
(a) reacting nucleic acids of the sample with a polynucleotide probe for HCV, wherein the probe is comprised of an HCV sequence of the invention, wherein the reacting is under conditions which allow the formation of a polynucleotide duplex between the probe and the HCV nucleic acid from the sample; and (b) detecting a polynucleotide duplex which contains the probe, formed in step (a).

Yet another aspect of the invention is an immunoassay for detecting an HCV antigen comprising:
(a) incubating a sample suspected of containing an HCV antigen with an antibody directed against an HCV epitope encoded in HCV cDNA, wherein the HCV cDNA is of a sequence indicated by nucleotide numbers -319 to 1348 or 8659 to 8866 in Fig. 17, or is the sequence present in clone 13i, or clone 26j, or clone 59a, or clone 84a, or clone CA156e, or clone 167b, or clone pi14a, or clone CA216a, or clone CA290a, or clone ag30a, or clone 205a, or clone 18g, or clone 16jh, and wherein the incubating is under conditions which allow formation of an antigen-antibody complex; and (b) detecting an antibody-antigen complex formed in step (a) which contains the antibody.

Still another aspect of the invention is an immunoassay for detecting antibodies directed against an HCV antigen comprising:
(a) incubating a sample suspected of containing anti-HCV antibodies with an antigen polypeptide containing an epitope encoded in HCV cDNA, wherein the HCV cDNA is of a sequence indicated by nucleotide numbers -319 to 1348 or 8659 to 8866 in Fig. 17, or is the sequence present in clone 13i, or clone 26j, or clone 59a, or clone 84a, or clone CA156e, or clone 167b, or clone pi14a, or clone CA216a, or clone CA290a, or clone ag30a, or clone 205a, or clone 18g, or clone 16jh, and wherein the incubating is under conditions which allow formation of an antigen-antibody complex; and detecting an antibody-antigen complex formed in step (a) which contains the antigen polypeptide.

### Brief Description of the Drawings

Figure 1 shows the sequence ofHCV cDNA in clone 12f, and the amino acids encoded therein.
Figure 2 shows the HCV cDNA sequence in clone k9-1, and the amino acids encoded therein.
Figure 3 shows the sequence of clone 15e, and the amino acids encoded therein.
Figure 4 shows the nucleotide sequence of HCV cDNA in clone 13i, the amino acids encoded therein, and the sequences which overlap with clone 12f.
Figure 5 shows the nucleotide sequence of HCV cDNA in clone 26j, the amino acids encoded therein, and the sequences which overlap clone 13i.
Figure 6 shows the nucleotide sequence of HCV cDNA in clone CA59a, the amino acids encoded therein, and the sequences which overlap with clones 26j and K9-1.
Figure 7 shows the nucleotide sequence of HCV cDNA in clone CA84a, the amino acids encoded therein, and the sequences which overlap with clone CA59a.
Figure 8. shows the nucleotide sequence of HCV cDNA in clone CA156e, the amino acids encoded therein, and the sequences which overlap with CA84a.
Fig. 9 shows the nucleotide sequence of HCV cDNA in clone CA167b, the amino acids encoded therein, and the sequences which overlap CA156e.
Fig. 10 shows the nucleotide sequence of HCV cDNA in clone CA216a, the amino acids encoded therein, and the overlap with clone CA167b.
Fig. 11 shows the nucleotide sequence of HCV cDNA in clone CA290a, the amino acids encoded therein, and the overlap with clone CA216a.
Fig. 12 shows the nucleotide sequence of HCV cDNA in clone ag30a and the overlap with clone CA290a.
Fig. 13 shows the nucleotide sequence of HCV cDNA in clone CA205a, and the overlap with the HCV cDNA sequence in clone CA290a.
Fig. 14 shows the nucleotide sequence of HCV cDNA in clone 18g, and the overlap with the HCV cDNA sequence in clone ag30a.
Fig. 15 shows the nucleotide sequence of HCV cDNA in clone 16jh, the amino acids encoded therein, and the overlap of nucleotides with the HCV cDNA sequence in clone 15e.
Fig. 16 shows the ORF of HCV cDNA derived from clones pi14a, CA167b, CA156e, CA84a, CA59a, K9-1, 12f, 14i, 11b, 7f, 7e, 8h, 33c, 40b, 37b, 35, 36, 81, 32, 33b, 25c, 14c, 8f, 33f, 33g, 39c, 35f, 19g, 26g, and 15e.
Fig. 17 shows the sense strand of the compiled HCV cDNA sequence derived from the above-described clones and the compiled HCV cDNA sequence published in EPO Pub. No. 318,216. The clones from which the sequence was derived are b114a, 18g, ag30a, CA205a, CA290a, CA216a, p114a, CA167b, CA156e, CA84a, CA59a, K9-1 (also called k9-1), 26j, 13i, 12f, 14i, 11b, 7f, 7e, 8h, 33c, 40b, 37b, 35, 36, 81, 32, 33b, 25c, 14c, 8f, 33f, 33g, 39c, 35f, 19g, 26g, 15e, b5a, and 16jh. In the figure the three horizontal dashes above the sequence indicate the position of the putative initiator methionine codon; the two vertical dashes indicate the first and last nucleotides of the published sequence. Also shown in the figure is the amino acid sequence of the putative polyprotein encoded in the HCV cDNA.
Fig. 18 is a diagram of the immunological colony screening method used in antigenic mapping studies.
Fig. 19 shows the hydrophobicity profiles of polyproteins encoded in HCV and in West Nile virus.
Fig. 20 is a tracing of the hydrophilicity/ hydrophobicity profile and of the antigenic index of the putative HCV polyprotein.
Fig. 21 shows the conserved co-linear peptides in HCV and Flaviviruses.

### Modes for Carrying Out the Invention

### I. Definitions

The term "hepatitis C virus" has been reserved by workers in the field for an heretofore unknown etiologic agent of NANBH. Accordingly, as used herein, "hepatitis C virus" (HCV) refers to an agent causitive of NANBH, which was formerly referred to as NANBV and/or BB-NANBV. The terms HCV, NANBV, and BB-NANBV are used interchangeably herein. As an extension of this terminology, the disease caused by HCV, formerly called NANB hepatitis (NANBH), is called hepatitis C. The terms NANBH and hepatitis C may be used interchangeably herein.

The term "HCV", as used herein, denotes a viral species of which pathogenic strains cause NANBH, and attenuated strains or defective interfering particles derived therefrom. As shown infra., the HCV genome is comprised of RNA. It is known that RNA containing viruses have relatively high rates of spontaneous mutation, i.e., reportedly on the order of 10⁻³ to 10⁻⁴ per incorporated nucleotide (Fields & Knipe (1986)). Therefore, there are multiple strains, which may be virulent or avirulent, within the HCV species described infra. The compositions and methods described herein, enable the propagation, identification, detection, and isolation of the various HCV strains or isolates. Moreover, the disclosure herein allows the preparation of diagnostics and vaccines for the various strains, as well as compositions and methods that have utility in screening procedures for anti-viral agents for pharmacologic use, such as agents that inhibit replication of HCV.

The information provided herein, although derived from the prototype strain or isolate of HCV, hereinafter referred to as CDC/HCV1 (also called HCV1), is sufficient to allow a viral taxonomist to identify other strains which fall within the species. The information provided herein allows the belief that HCV is a Flavi-like virus. The morphology and composition of Flavivirus particles are known, and are discussed in Brinton (1986). Generally, with respect to morphology, Flaviviruses contain a central nucleocapsid surrounded by a lipid bilayer. Virions are spherical and have a diameter of about 40-50 nm. Their cores are about 25-30 nm in diameter. Along the outer surface of the virion envelope are projections that are about 5-10 nm long with terminal knobs about 2 nm in diameter.

Different strains or isolates of HCV are expected to contain variations at the amino acid and nucleic acids compared with the prototype isolate, HCV1. Many isolates are expected to show much (i.e. more than about 40%) homology in the total amino acid sequence compared with HCV1. However, it may also be found that other less homologous HCV isolates. These would be defined as HCV strains according to various criteria such as an ORF of approximately 9,000 nucleotides to approximately 12,000 nucleotides, encoding a polyprotein similar in size to that of HCV1, an encoded polyprotein of similar hydrophobic and antigenic character to that of HCV1, and the presence of co-linear peptide sequences that are conserved with HCV1. In addition, the genome would be a positive-stranded RNA.

HCV encodes at least one epitope which is immunologically identifiable with an epitope in the HCV genome from which the cDNAs described herein are derived; preferably the epitope is contained an amino acid sequence described herein. The epitope is unique to HCV when compared to other known Flaviviruses. The uniqueness of the epitope may be determined by its immunological reactivity with anti-HCV antibodies and lack of immunological reactivity with antibodies to other Flavivirus species. Methods for determining immunological reactivity are known in the art, for example, by radioimmunoassay, by Elisa assay, by hemagglutination, and several examples of suitable techniques for assays are provided herein.

In addition to the above, the following parameters of nucleic acid homology and amino acid homology are applicable, either alone or in combination, in identifying a strain or isolate as HCV. Since HCV strains and isolates are evolutionarily related, it is expected that the overall homology of the genomes at the nucleotide level probably will be about 40% or greater, probably about 60% or greater, and even more probably about 80% or greater; and in addition that there will be corresponding contiguous sequences of at least about 13 nucleotides. The correspondence between the putative HCV strain genomic sequence and the CDC/HCV1 cDNA sequence can be determined by techniques known in the art. For example, they can be determined by a direct comparison of the sequence information of the polynucleotide from the putative HCV, and the HCV cDNA sequence(s) described herein. For example, also, they can be determined by hybridization of the polynucleotides under conditions which form stable duplexes between homologous regions (for example, those which would be used prior to S₁ digestion), followed by digestion with single stranded specific nuclease(s), followed by size determination of the digested fragments.

Because of the evolutionary relationship of the strains or isolates of HCV, putative HCV strains or isolates are identifiable by their homology at the polypeptide level. Generally, HCV strains or isolates are expected to be more than 40% homologous, probably more than about 70% homologous, and even more probably more than about 80% homologous, and some may even be more than about 90% homologous at the polypeptide level. The techniques for determining amino acid sequence homology are known in the art. For example, the amino acid sequence may be determined directly and compared to the sequences provided herein. Alternatively the nucleotide sequence of the genomic material of the putative HCV may be determined (usually via a cDNA intermediate), the amino acid sequence encoded therein can be determined, and the corresponding regions compared.

As used herein, a polynucleotide "derived from" a designated sequence refers to a polynucleotide sequence which is comprised of a sequence of at least 10-12 nucleotides, and preferably at least about 15-20 nucleotides corresponding to a region of the designated nucleotide sequence. "Corresponding" means homologous to or complementary to the designated sequence. Preferably, the sequence of the region from which the polynucleotide is derived is homologous to or complementary to a sequence which is unique to an HCV genome. Whether or not a sequence is unique to the HCV genome can be determined by techniques known to those of skill in the art. For example, the sequence can be compared to sequences in databanks, e.g., Genebank, to determine whether it is present in the uninfected host or other organisms. The sequence can also be compared to the known sequences of other viral agents, including those which are known to induce hepatitis, e.g., HAV, HBV, and HDV, and to other members of the Flaviviridae. The correspondence or non-correspondence of the derived sequence to other sequences can also be determined by hybridization under the appropriate stringency conditions. Hybridization techniques for determining the complementarity of nucleic acid sequences are known in the art, and are discussed infra. See also, for example, Maniatis et al. (1982). In addition, mismatches of duplex polynucleotides formed by hybridization can be determined by known techniques, including for example, digestion with a nuclease such as S1 that specifically digests single-stranded areas in duplex polynucleotides. Regions from which typical DNA sequences may be "derived" include but are not limited to, for example, regions encoding specific epitopes, as well as non-transcribed and/or non-translated regions.

The derived polynucleotide is not necessarily physically derived from the nucleotide sequence shown, but may be generated in any manner, including for example, chemical synthesis or DNA replication or reverse transcription or transcription. In addition, combinations of regions corresponding to that of the designated sequence may be modified in ways known in the art to be consistent with an intended use.

Similarly, a polypeptide or amino acid sequence "derived from" a designated nucleic acid sequence refers to a polypeptide having an amino acid sequence identical to that of a polypeptide encoded in the sequence, or a portion thereof wherein the portion consists of at least 10 amino acids, and even more preferably at least 11-15 amino acids, or which is immunologically identifiable with a polypeptide encoded in the sequence.

A recombinant or derived polypeptide is not necessarily translated from a designated nucleic acid sequence, for example, the HCV cDNA sequences described herein, or from an HCV genome; it may be generated in any manner, including for example, chemical synthesis, or expression of a recombinant expression system, or isolation from mutated HCV. A recombinant or derived polypeptide may include one or more analogs of amino acids or unnatural amino acids in its sequence. Methods of inserting analogs of amino acids into a sequence are known in the art. It also may include one or more labels, which are known to those of skill in the art.

The term "recombinant polynucleotide" as used herein intends a polynucleotide of genomic, cDNA, semisynthetic, or synthetic origin which, by virtue of its origin or manipulation which: (1) is not associated with all or a portion of a polynucleotide with which it is associated in nature, (2) is linked to a polynucleotide other than that to which it is linked in nature, or (3) does not occur in nature.

The term "polynucleotide" as used herein refers to a polymeric form of nucleotides of any length, either ribonucleotides or deoxyribonucleotides. This term refers only to the primary structure of the molecule. Thus, this term includes double- and single-stranded DNA, as well as double- and single stranded RNA. It also includes known types of modifications, for example, labels which are known in the art, methylation, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example proteins (including for e.g., nucleases, toxins, antibodies, signal peptides, poly-L-lysine, etc.), those with intercalators (e.g., acridine, psoralen, etc.), those containing chelators (e.g., metals, radioactive metals, boron, oxida- tive metals, etc.), those containing alkylators, those with modified linkages (e.g., alpha anomeric nucleic acids, etc.), as well as unmodified forms of the polynucleotide.

"Recombinant host cells", "host cells", "cells", "cell lines", "cell cultures", and other such terms denoting microorganisms or higher eukaryotic cell lines cultured as unicellular entities refer to cells which can be, or have been, used as recipients for recombinant vector or other transfer DNA, and include the progeny of the original cell which has been transfected. It is understood that the progeny of a single parental cell may hot necessarily be completely identical in morphology or in genomic or total DNA complement as the original parent, due to natural, accidental, or deliberate mutation.

A "replicon" is any genetic element, e.g., a plasmid, a chromosome, a virus, a cosmid, etc. that behaves as an autonomous unit of polynucleotide replication within a cell; i.e., capable of replication under its own control.

A "vector" is a replicon in which another polynucleotide segment is attached, so as to bring about the replication and/or expression of the attached segment.

"Control sequence" refers to polynucleotide sequences which are necessary to effect the expression of coding sequences to which they are ligated. The nature of such control sequences differs depending upon the host organism; in prokaryotes, such control sequences generally include promoter, ribosomal binding site, and terminators; in eukarybtes, generally, such control sequences include promoters, terminators and, in some instances, enhancers. The term "control sequences" is intended to include, at a minimum, all components whose presence is necessary for expression, and may also include additional components whose presence is advantageous, for example, leader sequences.

"Operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

An "open reading frame" (ORF) is a region of a polynucleotide sequence which encodes a polypeptide; this region may represent a portion of a coding sequence or a total coding sequence.

A "coding sequence" is a polynucleotide sequence which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, and recombinant polynucleotide sequences.

"Immunologically identifiable with/as" refers to the presence of epitope(s) and polypeptides(s) which are also present in the designated polypeptide(s), usually HCV proteins. Immunological identity may be determined by antibody binding and/or competition in binding; these techniques are known to those of average skill in the art, and are also illustrated infra.

As used herein, "epitope" refers to an antigenic determinant of a polypeptide; an epitope could comprise 3 amino acids in a spatial conformation which is unique to the epitope, generally an epitope consists of at least 5 such amino acids, and more usually, consists of at least 8-10 such amino acids. Methods of determining the spatial conformation of amino acids are known in the art, and include, for example, x-ray crystallography and 2-dimensional nuclear magnetic resonance.

A polypeptide is "immunologically reactive" with an antibody when it binds to an antibody due to antibody recognition of a specific epitope contained within the polypeptide. Immunological reactivity may be determined by antibody binding, more particularly by the kinetics of antibody binding, and/or by competition in binding using as competitor(s) a known polypeptide(s) containing an epitope against which the antibody is directed. The techniques for determining whether a polypeptide is immunologically reactive with an antibody are known in the art.

As used herein, the term "immunogenic polypeptide" is a polypeptide that elicits a cellular and/ or humoral response, whether alone or linked to a carrier in the presence or absence of an adjuvant.

The term "polypeptide" refers to a polymer of amino acids and does not refer to a specific length of the product; thus, peptides, oligopeptides, and proteins are included within the definition of polypeptide. This term also does not refer to or exclude post-expression modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

"Transformation", as used herein, refers to the insertion of an exogenous polynucleotide into a host cell, irrespective of the method used for the insertion, for example, direct uptake, transduction, f-mating or electroporation. The exogenous polynucleotide may be maintained as a non-integrated vector, for example, a plasmid, or alternatively, may be integrated into the host genome.

"Treatment" as used herein refers to prophylaxis and/or therapy.

An "individual", as used herein, refers to vertebrates, particularly members of the mammalian species, and includes but is not limited to domestic animals, sports animals, and primates, including humans.

As used herein, the "sense strand" of a nucleic acid contains the sequence that has sequence homology to that of mRNA. The "anti-sense strand" contains a sequence which is complementary to that of the "sense strand".

As used herein, a "positive stranded genome" of a virus is one in which the genome, whether RNA or DNA, is single-stranded and which encodes a viral polypeptide(s). Examples of positive stranded RNA viruses include Togaviridae, Coronaviridae, Retroviridae, Picornaviridae, and Caliciviridae. Included also, are the Flaviviridae, which were formerly classified as Togaviradae. See Fields & Knipe (1986).

As used herein, "antibody-containing body component" refers to a component of an individual's body which is a source of the antibodies of interest. Antibody containing body components are known in the art, and include but are not limited to, for example, plasma, serum, spinal fluid, lymph fluid, the external sections of the respiratory, intestinal, and genitourinary tracts, tears, saliva, milk, white blood cells, and myelomas.

As used herein, "purified HCV" refers to a preparation of HCV which has been isolated from the cellular constituents with which the virus is normally associated, and from other types of viruses which may be present in the infected tissue. The techniques for isolating viruses are known to those of skill in the art, and include, for example, centrifugation and affinity chromatography; a method of preparing purified HCV is discussed infra.

The term "HCV particles" as used herein include entire virion as well as particles which are intermediates in virion formation. HCV particles generally have one or more HCV proteins associated with the HCV nucleic acid.

As used herein, the term "probe" refers to a polynucleotide which forms a hybrid structure with a sequence in a target region, due to complementarity of at least one sequence in the probe with a sequence in the target region. The probe, however, does not contain a sequence complementary to sequence(s) used to prime the polymerase chain reaction.

As used herein, the term "target region" refers to a region of the nucleic acid which is to be amplified and/or detected.

As used herein, the term "viral RNA", which includes HCV RNA, refers to RNA from the viral genome, fragments thereof, transcripts thereof, and mutant sequences derived therefrom.

As used herein, a "biological sample" refers to a sample of tissue or fluid isolated from an individual, including but not limited to, for example, plasma, serum, spinal fluid, lymph fluid, the external sections of the skin, respiratory, intestinal, and genitourinary tracts, tears, saliva, milk, blood cells, tumors, organs, and also samples of in vitro cell culture constituents (including but not limited to conditioned medium resulting from the growth of cells in cell culture medium, putatively virally infected cells, recombinant cells, and cell components).

### II. Description of the Invention

The practice of the present invention will employ, unless otherwise indicated, conventional techniques of molecular biology, microbiology, recombinant DNA, and immunology, which are within the skill of the art. Such techniques are explained fully in the literature. See e.g., Maniatis, Fitsch & Sambrook, MOLECULAR CLONING; A LABORATORY MANUAL (1982); DNA CLONING, VOLUMES I AND II (D.N Glover ed. 1985); OLIGONUCLEOTIDE SYNTHESIS (M.J. Gait ed, 1984); NUCLEIC ACID HYBRIDIZATION (B.D. Hames & S.J. Higgins eds. 1984); TRANSCRIPTION AND TRANSLATION (B.D. Hames & S.J. Higgins eds. 1984); ANIMAL CELL CULTURE (R.I. Freshney ed. 1986); IMMOBILIZED CELLS AND ENZYMES (IRL Press, 1986); B. Perbal, A PRACTICAL GUIDE TO MOLECULAR CLONING (1984); the series, METHODS IN ENZYMOLOGY (Academic Press, Inc.); GENE TRANSFER VECTORS FOR MAMMALIAN CELLS (J.H. Miller and M.P. Calos eds. 1987, Cold Spring Harbor Laboratory), Methods in Enzymology Vol. 154 and Vol. 155 (Wu and Grossman, and Wu, eds., respectively), Mayer and Walker, eds. (1987), IMMUNOCHEMICAL METHODS IN CELL AND MOLECULAR BIOLOGY (Academic Press, London), Scopes, (1987), PROTEIN PURIFICATION: PRINCIPLES AND PRACTICE, Second Edition (Springer-Verlag, N.Y.), and HANDBOOK OF EXPERIMENTAL IMMUNOLOGY, VOLUMES I-IV (D.M. Weir and C. C. Blackwell eds 1986). All patents, patent applications, and publications mentioned herein, both supra and infra, are hereby incorporated herein by reference.

The useful materials and processes of the present invention are made possible by the provision of a family of nucleotide sequences isolated from cDNA libraries which contain HCV cDNA sequences. These cDNA libraries were derived from nucleic acid sequences present in the plasma of an HCV-infected chimpanzee. The construction of one of these libraries, the "c" library (ATCC No. 40394), was reported in EPO Pub. No. 318,216. Several of the clones containing HCV cDNA reported herein were obtained from the "c" library. Although other clones reported herein were obtained from other HCV cDNA libraries, the presence of clones containing the sequences in the "c" library was confirmed. As discussed in EPO Pub. No. 318,216, the family of HCV cDNA sequences isolated from the "c" library are not of human or chimpanzee origin, and show no significant homology to sequences contained within the HBV genome.

The availability of the HCV cDNAs described herein permits the construction of polynucleotide probes which are reagents useful for detecting viral polynucleotides in biological samples, including donated blood. For example, from the sequences it is possible to synthesize DNA oligomers of about 8-10 nucleotides, or larger, which are useful as hybridization probes to detect the presence of HCV RNA in, for example, donated blood, sera of subjects suspected of harboring the virus, or cell culture systems in which the virus is replicating. In addition, the cDNA sequences also allow the design and production of HCV specific polypeptides which are useful as diagnostic reagents for the presence of antibodies raised during HCV infection. Antibodies to purified polypeptides derived from the cDNAs may also be used to detect viral antigens in biological samples, including, for example, donated blood samples, sera from patients with NANBH, and in tissue culture systems being used for HCV replication. Moreover, the immunogenic polypeptides disclosed herein, which are encoded in portions of the ORF of HCV cDNA shown in Fig. 17, are also useful for HCV screening, diagnosis, and treatment, and for raising antibodies which are also useful for these purposes.

In addition, the novel cDNA sequences described herein enable further characterization of the HCV genome. Polynucleotide probes and primers derived from these sequences may be used to amplify sequences present in cDNA libraries, and/or to screen cDNA libraries for additional overlapping cDNA sequences, which, in turn, may be used to obtain more overlapping sequences. As indicated infra. and in EPO Pub. No. 318,216, the genome of HCV appears to be RNA comprised primarily of a large open reading frame (ORF) which encodes a large polyprotein.

The HCV cDNA sequences provided herein, the polypeptides derived from these sequences, and the immunogenic polypeptides described herein, as well as antibodies directed against these polypeptides are also useful in the isolation and identification of the blood-borne NABV (BB-NANBV) agent(s). For example, antibodies directed against HCV epitopes contained in polypeptides derived from the cDNAs may be used in processes based upon affinity chromatography to isolate the virus. Alternatively, the antibodies may be used to identify viral particles isolated by other techniques. The viral antigens and the genomic material within the isolated viral particles may then be further characterized.

In addition to the above, the information provided infra allows the identification of additional HCV strains or isolates. The isolation and characterization of the additional HCV strains or isolates may be accomplished by isolating the nucleic acids from body components which contain viral particles and/or viral RNA, creating cDNA libraries using polynucleotide probes based on the HCV cDNA probes described infra., screening the libraries for clones containing HCV cDNA sequences described infra., and comparing the HCV cDNAs from the new isolates with the cDNAs described infra. The polypeptides encoded therein, or in the viral genome, may be monitored for immunological cross-reactivity utilizing the polypeptides and antibodies described supra. Strains or isolates which fit within the parameters of HCV, as described in the Definitions section, supra., are readily identifiable. Other methods for identifying HCV strains will be obvious to those of skill in the art, based upon the information provided herein.

### Isolation of the HCV cDNA Sequences

The novel HCV cDNA sequences described infra. extend the sequence of the cDNA to the HCV genome reported in EPO Pub. No. 318,216. The sequences which are present in clones b114a, 18g, ag30a, CA205a, CA290a, CA216a, pi14a, CA167b, CA156e, CA84a, and CA59a lie upstream of the reported sequence, and when compiled, yield nucleotides nos. -319 to 1348 of the composite HCV cDNA sequence. (The negative number on a nucleotide indicates its distance upstream of the nucleotide which starts the putative initiator MET codon.) The composite HCV cDNA sequence which includes the sequences in the aforementioned clones is shown in Figure 17.

The novel HCV cDNAs described herein were isolated from a number of HCV cDNA libraries, including the "c" library present in lambda gt11 (ATCC No. 40394). The HCV cDNA libraries were constructed using pooled serum from a chimpanzee with a chronic HCV infection and containing a high titre of the virus, i.e. at least 10⁶ chimp infectious doses/ml (CID/ml). The pooled serum was used to isolate viral particles; nucleic acids isolate from these particles was used as the template in the construction of cDNA libraries to the viral genome. The procedures for isolation of putative HCV particles and for constructing the "c" HCV cDNA library is described in EPO Pub. No. 318,216. Other methods for constructing HCV cDNA libraries are known in the art, and some of these methods are described infra., in the Examples. Isolation of the sequences was by screening the libraries using synthetic polynucleotide probes, the sequences of which were derived from the 5'-region and the 3'-region of the known HCV cDNA sequence. The description of the method to retrive the cDNa sequences is mostly of historical interest. The resultant sequences (and their complements) are provided herein, and the sequences, or any portion thereof, could be prepared using synthetic methods, or by a combination of synthetic methods with retrieval of partial sequences using methods similar to those described herein.

### Preparation of Viral Polypeptides and Fragments

The availability of HCV cDNA sequences, or nucleotide sequences derived therefrom (including segments and modifications of the sequence), permits the construction of expression vectors encoding antigenically active regions of the polypeptide encoded in either strand. These antigenically active regions may be derived from coat or envelope antigens or from core antigens, or from antigens which are non-structural including, for example, polynucleotide binding proteins, polynucleotide polymerase(s), and other viral proteins required for the replication and/or assembly of the virus particle. Fragments encoding the desired polypeptides are derived from the cDNA clones using conventional restriction digestion or by synthetic methods, and are ligated into vectors which may, for example, contain portions of fusion sequences such as beta-galactosidase or superoxide dismutase (SOD), preferably SOD. Methods and vectors which are useful for the production of polypeptides which contain fusion sequences of SOD are described in European Patent Office Publication number 0196056, published October 1, 1986. vectors for the expression of fusion polypeptides of SOD and HCV polypeptides encoded in a number of HCV clones are described infra., in the Examples. Any desired portion of the HCV cDNA containing an open reading frame, in either sense strand, can be obtained as a recombinant polypeptide, such as a mature or fusion protein; alternatively, a polypeptide encoded in the cDNA can be provided by chemical synthesis.

The DNA encoding the desired polypeptide, whether in fused or mature form, and whether or not containing a signal sequence to permit secretion, may be ligated into expression vectors suitable for any convenient host. Both eukaryotic and prokaryotic host systems are presently used in forming recombinant polypeptides, and a summary of some of the more common control systems and host cell lines is given infra. The polypeptide is then isolated from lysed cells or from the culture medium and purified to the extent needed for its intended use. Purification may be by techniques known in the art, for example, differential extraction, salt fractionation, chromatography on ion exchange resins, affinity chromatography, centrifugation, and the like. See, for example, Methods in Enzymology for a variety of methods for purifying proteins. Such polypeptides can be used as diagnostics, or those which give rise to neutralizing antibodies may be formulated into vaccines. Antibodies raised against these polypeptides can also be used as diagnostics, or for passive immunotherapy. In addition, as discussed infra., antibodies to these polypeptides are useful for isolating and identifying HCV particles.

### Preparation of Antigenic Polypeptides and Conjugation with Carrier

An antigenic region of a polypeptide is generally relatively small--typically 8 to 10 amino acids or less in length. Fragments of as few as 5 amino acids may characterize an antigenic region. These segments may correspond to regions of HCV antigen. Accordingly, using the cDNAs of HCV as a basis, DNAs encoding short segments of HCV polypeptides can be expressed recombinantly either as fusion proteins, or as isolated polypeptides. In addition, short amino acid sequences can be conveniently obtained by chemical synthesis. In instances wherein the synthesized polypeptide is correctly configured so as to provide the correct epitope, but is too small to be immunogenic, the polypeptide may be linked to a suitable carrier.

A number of techniques for obtaining such linkage are known in the art, including the formation of disulfide linkages using N-succinimidyl-3-(2-pyridylthio)propionate (SPDP) and succinimidyl 4-(N-maleimidomethyl)cyclohexane-1-carboxylate (SMCC) obtained from Pierce Company, Rockford, Illinois, (if the peptide lacks a sulfhydryl group, this can be provided by addition of a cysteine residue.) These reagents create a disulfide linkage between themselves and peptide cysteine residues on one protein and an amide linkage through the epsilon-amino on a lysine, or other free amino group in the other. A variety of such disulfide/amide-forming agents are known. See, for example, Immun. Rev. (1982) 62:185. Other bifunctional coupling agents form a thioether rather than a disulfide linkage. Many of these thio-ether-forming agents are commercially available and include reactive esters of 6-maleimidocaproic acid, 2-bromoacetic acid, 2-iodoacetic acid, 4-(N-maleimidomethyl)cyclohexane-1-carboxylic acid, and the like. The carboxyl groups can be activated by combining them with succinimide or 1-hydroxyl-2-nitro-4-sulfonic acid, sodium salt. Additional methods of coupling antigens employs the rotavirus/"binding peptide" system described in EPO Pub. No. 259,149, the disclosure of which is incorporated herein by reference. The foregoing list is not meant to be exhaustive, and modifications of the named compounds can clearly be used.

Any carrier may be used which does not itself induce the production of antibodies harmful to the host. Suitable carriers are typically large, slowly metabolized macromolecules such as proteins; polysaccharides, such as latex functionalized sepharose, agarose, cellulose, cellulose beads and the like; polymeric amino acids, such as polyglutamic acid, polylysine, and the like; amino acid copolymers; and inactive virus particles. Especially useful protein substrates are serum albumins, keyhole limpet hemocyanin, immunoglobulin molecules, thyroglobulin, ovalbumin, tetanus toxoid, and other proteins well known to those skilled in the art.

In addition to full-length viral proteins, polypeptides comprising truncated HCV amino acid sequences encoding at least one viral epitope are useful immunological reagents. For example, polypeptides comprising such truncated sequences can be used as reagents in an immunoassay. These polypeptides also are candidate subunit antigens in compositions for antiserum production or vaccines. While these truncated sequences can be produced by various known treatments of native viral protein, it is generally preferred to make synthetic or recombinant polypeptides comprising an HCV sequence. Polypeptides comprising these truncated HCV sequences can be made up entirely of HCV sequences (one or more epitopes, either contiguous or noncontiguous), or HCV sequences and heterologous sequences in a fusion protein. Useful heterologous sequences include sequences that provide for secretion from a recombinant host, enhance the immunological reactivity of the HCV epitope(s), or facilitate the coupling of the polypeptide to an immunoassay support or a vaccine carrier. See, e.g., EPO Pub. No. 116,201; U.S. Pat. No. 4,722,840; EPO Pub. No. 259,149; U.S. Pat. No. 4,629,783, the disclosures of which are incorporated herein by reference.

The size of polypeptides comprising the truncated HCV sequences can vary widely, the minimum size being a sequence of sufficient size to provide an HCV epitope, while the maximum size is not critical. For convenience, the maximum size usually is not substantially greater than that required to provide the desired HCV epitopes and function(s) of the heterologous sequence, if any. Typically, the truncated HCV amino acid sequence will range from about 5 to about 100 amino acids in length. More typically, however, the HCV sequence will be a maximum of about 50 amino acids in length, preferably a maximum of about 30 amino acids. It is usually desirable to select HCV sequences of at least about 10, 12 or 15 amino acids, up to a maximum of about 20 or 25 amino acids.

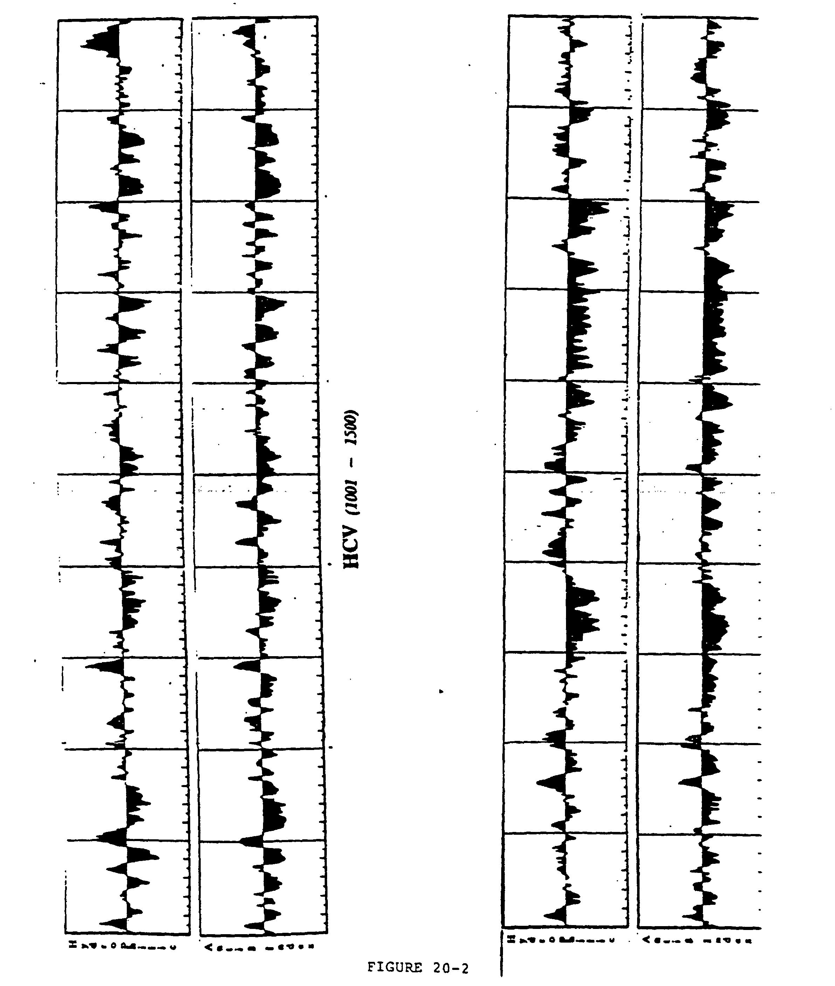

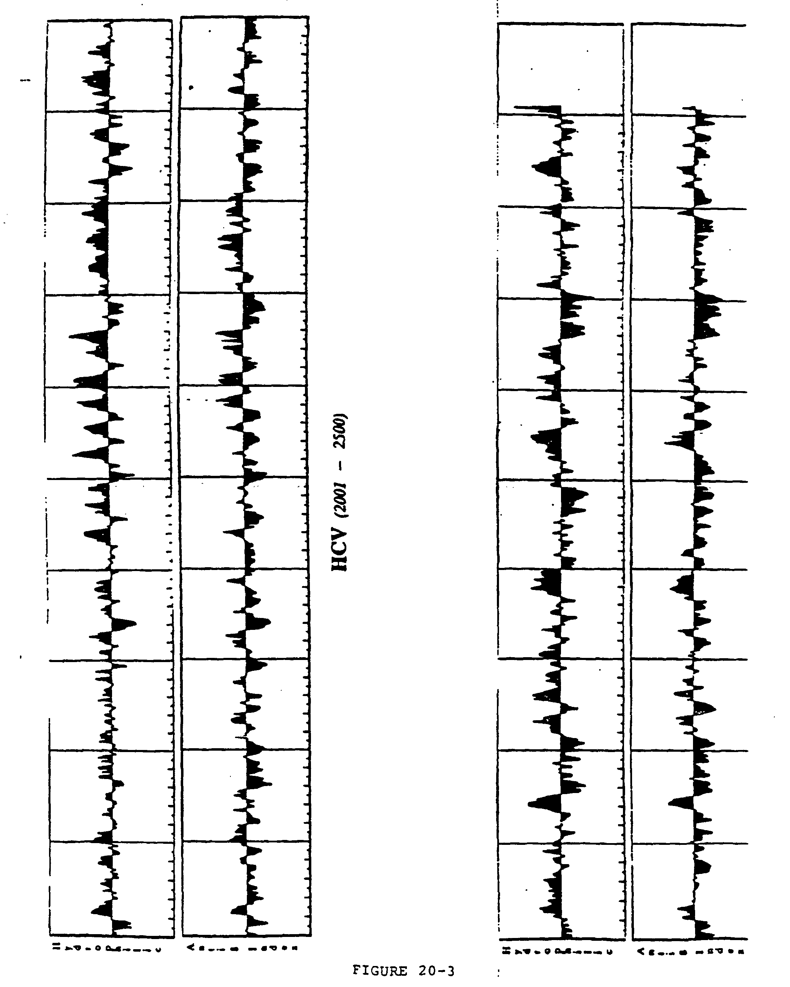

Truncated HCV amino acid sequences comprising epitopes can be identified in a number of ways. For example, the entire viral protein sequence can be screened by preparing a series of short peptides that together span the entire protein sequence. An example of antigenic screening of the regions of the HCV polyprotein is shown infra. In addition, by starting with, for example, 100mer polypeptides, it would be routine to test each polypeptide for the presence of epitope(s) showing a desired reactivity, and then testing progressively smaller and overlapping fragments from an identified 100mer to map the epitope of interest. Screening such peptides in an immunoassay is within the skill of the art. It is also known to carry out a computer analysis of a protein sequence to identify potential epitopes, and then prepare oligopeptides comprising the identified regions for screening. Such a computer analysis of the HCV amino acid sequence is shown in Fig. 20, where the hydrophilic/ hydrophobic character is displayed above the antigen index. The amino acids are numbered from the starting MET (position 1) as shown in Fig. 17. It is appreciated by those of skill in the art that such computer analysis of antigenicity does not always identify an epitope that actually exists, and can also incorrectly identify a region of the protein as containing an epitope.

Examples of HCV amino acid sequences that may be useful, which are expressed from expression vectors comprised of clones 5-1-1, 81, CA74a, 35f, 279a, C36, C33b, CA290a, CBf, C12f, 14c, 15e, C25c, C33c, C33f, 33g, C39c, C40b, CA167b are described infra. Other examples of HCV amino acid sequences that may be useful as described herein are set forth below. It is to be understood that these peptides do not necessarily precisely map one epitope, and may also contain HCV sequence that is not immunogenic. These non-immunogenic portions of the sequence can be defined as described above using conventional techniques and deleted from the described sequences. Further, additional truncated HCV amino acid sequences that comprise an epitope or are immunogenic can be identified as described above. The following sequences are given by amino acid number (i.e., "AAn") where n is the amino acid number as shown in Fig. 17: AA1-AA25; AA1-AA50; AA1-AA84; AA9-AA177; AA1-AA10; AA5-AA20; AA35-AA45; AA50-A.A100; AA40-AA90; AA45-AA65; AA65-AA75; AA80-90; AA99-AA120; AA95-AA110; AA105-AA120; AA100-AA150; AA150-AA200; AA155-AA170; AA190-AA210; AA200-AA250; AA220-AA240 AA245-AA265; AA250-AA300; AA290-AA330; AA290-305; AA300-AA350; AA310-AA330; AA350-AA400; AA380-AA395; AA405-AA495; AA400-AA450; AA405-AA415; AA415-AA425; AA425-AA435; AA437-AA582; AA440-AA460; The above HCV amino acid sequences can be discrete peptides or incorporated into a larger polypeptide, and may find use as described herein. Additional polypeptides comprising truncated HCV sequences are described in the examples.

The observed relationship of the putative polyproteins of HCV and the Flaviviruses allows some prediction of the putative domains of the HCV "non-structural" (NS) proteins. The locations of the individual NS proteins in the putative Flavivirus precursor polyprotein are fairly well-known. Moreover, these also coincide with observed gross fluctuations in the hydrophobicity profile of the polyprotein. It is established that NS5 of Flaviviruses encodes the virion polymerase, and that NS1 corresponds with a complement fixation antigen which has been shown to be an effective vaccine in animals. Recently, it has been shown that a flaviviral protease function resides in NS3. Due to the observed similarities betwen HCV and the Flaviviruses, described infra., deductions concerning the approximate locations of the corresponding protein domains and functions in the HCV polyprotein:are possible. The expression of polypeptides containing these domains in a variety of recombinant host cells, including, for example, bacteria, yeast, insect, and vertebrate cells, should give rise to important immunological reagents which can be used for diagnosis, detection, and vaccines.

Although the non-structural protein regions of the putative polyproteins of the HCV isolate described herein and of Flaviviruses appear to have some similarity, there is less similarity between the putative structural regions which are towards the N-terminus. In this region, there is a greater divergence in sequence, and in addition, the hydrophobic profile of the two regions show less similarity. This "divergence" begins in the N-terminal region of the putative NS1 domain in HCV, and extends to the presumed N-terminus. Nevertheless, it may still be possible to predict the approximate locations of the putative nucleocapsid (N-terminal basic domain) and E (generally hydrophobic) domains within the HCV polyprotein. In the Examples the predictions are based on the changes observed in the hydrophobic profile of the HCV polyprotein, and on a knowledge of the location and character of the flaviviral proteins. From these predictions it may be possible to identify approximate regions of the HCV polyprotein that could correspond with useful immunological reagents. For example, the E and NS1 proteins of Flaviviruses are known to have efficacy as protective vaccines. These regions, as well as some which are shown to be antigenic in the HCV isolate described herein, for example those within putative NS3, C, and NS5, etc., should also provide diagnostic reagents. Moreover, the location and expression of viral-encoded enzymes may also allow the evaluation of anti-viral enzyme inhibitors, i.e., for example, inhibitors which prevent enzyme activity by virtue of an interaction with the enzyme itself, or substances which may prevent expression of the enzyme, (for example, anti-sense RNA, or other drugs which interfere with expression).

### Preparation of Hybrid Particle Immunogens Containing HCV Epitopes

The immunogenicity of the epitopes of HCV may also be enhanced by preparing them in mammalian or yeast systems fused with or assembled with particle-forming proteins such as, for example, that associated with hepatitis B surface antigen. Constructs wherein the NANBV epitope is linked directly to the particle-forming protein coding sequences produce hybrids which are immunogenic with respect to the HCV epitope. In addition, all of the vectors prepared include epitopes specific to HBV, having various degrees of immunogenicity, such as, for example, the pre-S peptide. Thus, particles constructed from particle forming protein which include HCV sequences are immunogenic with respect to HCV and HBV.

Hepatitis surface antigen (HBSAg) has been shown to be formed and assembled into particles in S. cerevisiae (Valenzuela et al. (1982)), as well as in, for example, mammalian cells (Valenzuela, P., et al. (1984)). The formation of such particles has been shown to enhance the immunogenicity of the monomer subunit. The constructs may also include the immunodominant epitope of HBSAg, comprising the 55 amino acids of the presurface (pre-S) region. Neurath et al. (1984). Constructs of the pre-S-HBSAg particle expressible in yeast are disclosed in EPO 174,444, published March 19, 1986; hybrids including heterologous viral sequences for yeast expression are disclosed in EPO 175,261, published March 26, 1966. These constructs may also be expressed in mammalian cells such as Chinese hamster ovary (CHO) cells using an SV40-dihydrofolate reductase vector (Michelle et al. (1984)).

In addition, portions of the particle-forming protein coding sequence may be replaced with codons encoding an HCV epitope. In this replacement, regions which are not required to mediate the aggregation of the units to form immunogenic particles in yeast or mammals can be deleted, thus eliminating additional HBV antigenic sites from competition with the HCV epitope.

### Preparation of Vaccines

Vaccines may be prepared from one or more immunogenic polypeptides derived from HCV cDNA, including the cDNA sequences described in the Examples. The observed homology between HCV and Flaviviruses provides information concerning the polypeptides which may be most effective as vaccines, as well as the regions of the genome in which they are encoded. The general structure of the Flavivirus genome is discussed in Rice et al (1986). The flavivirus genomic RNA is believed to be the only virus-specific mRNA species, and it is translated into the three viral structural proteins, i.e., C, M, and E, as well as two large nonstructural proteins, NS4 and NS5, and a complex set of smaller nonstructural proteins. It is known that major neutralizing epitopes for Flaviviruses reside in the E (envelope) protein (Roehrig (1986)). Thus, vaccines may be comprised of recombinant polypeptides containing epitopes of HCV E. These polypeptides may be expressed in bacteria, yeast, or mammalian cells, or alternatively may be isolated from viral preparations. It is also anticipated that the other structural proteins may also contain epitopes which give rise to protective anti-HCV antibodies. Thus, polypeptides containing the epitopes of E, C, and M may also be used, whether singly or in combination, in HCV vaccines.

In addition to the above, it has been shown that immunization with NS1 (nonstructural protein 1), results in protection against yellow fever (Schlesinger et al (1986)). This is true even though the immunization does not give rise to neutralizing antibodies. Thus, particularly since this protein appears to be highly conserved among Flaviviruses, it is likely that HCV NS1 will also be protective against HCV infection. Moreover, it also shows that nonstructural proteins may provide protection against viral pathogenicity, even if they do not cause the production of neutralizing antibodies.

The information provided in the Examples concerning the immunogenicity of the polypeptides expressed from cloned HCV cDNAs which span the various regions of the HCV ORF also allows predictions concerning their use in vaccines.

In view of the above, multivalent vaccines against HCV may be comprised of one or more epitopes from one or more structural proteins, and/or one or more epitopes from one or more nonstructural proteins. These vaccines may be comprised of, for example, recombinant HCV polypeptides and/or polypeptides isolated from the virions. In particular, vaccines are contemplated comprising one or more of the following HCV proteins, or subunit antigens derived therefrom: E, NS1, C, NS2, NS3, NS4 and NS5. Particularly preferred are vaccines comprising E and/or NS1, or subunits thereof.

The preparation of vaccines which contain an immunogenic polypeptide(s) as active ingredients, is known to one skilled in the art. Typically, such vaccines are prepared as injectables, either as liquid solutions or suspensions; solid forms suitable for solution in, or suspension in, liquid prior to injection may also be prepared. The preparation may also be emulsified, or the protein encapsulated in liposomes. The active immunogenic ingredients are often mixed with excipients which are pharmaceutically acceptable and compatible with the active ingredient. Suitable excipients are, for example, water, saline, dextrose, glycerol, ethanol, or the like and combinations thereof. In addition, if desired, the vaccine may contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. The effectiveness of an adjuvant may be determined by measuring the amount of antibodies directed against an immunogenic polypeptide containing an HCV antigenic sequence resulting from administration of this polypeptide in vaccines which are also comprised of the various adjuvants.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1%-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10%-95% of active ingredient, preferably 25%-70%.

The proteins may be formulated into the vaccine as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric, maleic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

### Dosage and Administration of Vaccines

The vaccines are administered in a manner compatible with the dosage formulation, and in such amount as will be prophylactically and/or therapeutically effective. The quantity to be administered, which is generally in the range of 5 micrograms to 250 micrograms of antigen per dose, depends on the subject to be treated, capacity of the subject's immune system to synthesize antibodies, and the degree of protection desired. Precise amounts of active ingredient required to be administered may depend on the judgment of the practitioner and may be peculiar to each subject.

The vaccine may be given in a single dose schedule, or preferably in a multiple dose schedule. A multiple dose schedule is one in which a primary course of vaccination may be with 1-10 separate doses, followed by other doses given at subsequent time intervals required to maintain and or reenforce the immune response, for example, at 1-4 months for a second dose, and if needed, a subsequent dose(s) after several months. The dosage regimen will also, at least in part, be determined by the need of the individual and be dependent upon the judgment of the practitioner.

In addition, the vaccine containing the immunogenic HCV antigen(s) may be administered in conjunction with other immunoregulatory agents, for example, immune globulins.

### Preparation of Antibodies Against HCV Epitopes

The immunogenic polypeptides prepared as described above are used to produce antibodies, both polyclonal and monoclonal. If polyclonal antibodies are desired, a selected mammal (e.g., mouse, rabbit, goat, horse, etc.) is immunized with an immunogenic polypeptide bearing an HCV epitope(s). Serum from the immunized animal is collected and treated according to known procedures. If serum containing polyclonal antibodies to an HCV epitope contains antibodies to other antigens, the polyclonal antibodies can be purified by immunoaffinity chromatography. Techniques for producing and processing polyclonal antisera are known in the art, see for example, Mayer and Walker (1987).

Alternatively, polyclonal antibodies may be isolated from a mammal which has been previously infected with HCV. An example of a method for purifying antibodies to HCV epitopes from serum from an infected individual, based upon affinity chromatography and utilizing a fusion polypeptide of SOD and a polypeptide encoded within cDNA clone 5-1-1, is presented in EPO Pub. No. 318,216.

Monoclonal antibodies directed against HCV epitopes can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. See, e.g., M. Schreier et al. (1980); Hammerling et al. (1981); Kennett et al. (1980); see also, U.S. Patent Nos. 4,341,761; 4,399,121; 4,427,783; 4,444,887; 4,466,917; 4,472,500; 4,491,632; and 4,493,890. Panels of monoclonal antibodies produced against HCV epitopes can be screened for various properties; i.e., for isotype, epitope affinity, etc.

Antibodies, both monoclonal and polyclonal, which are directed against HCV epitopes are particularly useful in diagnosis, and those which are neutralizing are useful in passive immunotherapy. Monoclonal antibodies, in particular, may be used to raise anti-idiotype antibodies.

Anti-idiotype antibodies are immunoglobulins which carry an "internal image" of the antigen of the infectious agent against which protection is desired. See, for example, Nisonoff, A., et al. (1981) and Dreesman et al. (1985).

Techniques for raising anti-idiotype antibodies are known in the art. See, for example, Grzych (1985), MacNamara et al. (1984), and Uytdehaag et al. (1985). These anti-idiotype antibodies may also be useful for treatment and/or diagnosis of NANBH, as well as for an elucidation of the immunogenic regions of HCV antigens.

It would also be recognized by one of ordinary skill in the art that a variety of types of antibodies directed against HCV epitopes may be produced. As used herein, the term "antibody" refers to a polypeptide or group of polypeptides which are comprised of at least one antibody combining site. An "antibody combining site" or "binding domain" is formed from the folding of variable domains of an antibody molecule(s) to form three-dimensional binding spaces with an internal surface shape and charge distribution complementary to the features of an epitope of an antigen, which allows an immunological reaction with the antigen. An antibody combining site may be formed from a heavy and/or a light chain domain (VH and VL, respectively), which form hypervariable loops which contribute to antigen binding. The term "antibody" includes, for example, vertebrate antibodies, hybrid antibodies, chimeric antibodies, altered antibodies, univalent antibodies, the Fab proteins, and single domain antibodies.

A "single domain antibody" (dAb) is an antibody which is comprised of an VH domain, which reacts immunologically with a designated antigen. A dAB does not contain a VL domain, but may contain other antigen binding domains known to exist in antibodies, for example, the kappa and lambda domains. Methods for preparing dABs are known in the art. See, for example, Ward et al. (1989).

Antibodies may also be comprised of VH and VL domains, as well as other known antigen binding domains. Examples of these types of antibodies and methods for their preparation are known in the art (see, e.g., U.S. Patent No. 4,816,467, which is incorporated herein by reference), and include the following. For example, "vertebrate antibodies" refers to antibodies which are tetramers or aggregates thereof, comprising light and heavy chains which are usually aggregated in a "Y" configuration and which may or may not have covalent linkages between the chains. In vertebrate antibodies, the amino acid sequences of all the chains of a particular antibody are homologous with the chains found in one antibody produced by the lymphocyte which produces that antibody in situ, or in vitro (for example, in hybridomas). Vertebrate antibodies typicallly include native antibodies, for example, purified polyclonal antibodies and monoclonal antibodies. Examples of the methods for the preparation of these antibodies are described infra.

"Hybrid antibodies" are antibodies wherein one pair of heavy and light chains is homologous to those in a first antibody, while the other pair of heavy and light chains is homologous to those in a different second antibody. Typically, each of these two pairs will bind different epitopes, particularly on different antigens. This results in the property of "divalence", i.e., the ability to bind two antigens simultaneously. Such hybrids may also be formed using chimeric chains, as set forth below.

"Chimeric antibodies", are antibodies in which the heavy and/or light chains are fusion proteins. Typically the constant domain of the chains is from one particular species and/or class, and the variable domains are from a different species and/or class. Also included is any antibody in which either or both of the heavy or light chains are composed of combinations of sequences mimicking the sequences in antibodies of different sources, whether these sources be differing classes, or different species of origin, and whether or not the fusion point is at the variable/constant boundary. Thus, it is possible to produce antibodies in which neither the constant nor the variable region mimic known antibody sequences. It then becomes possible, for example, to construct antibodies whose variable region has a higher specific affinity for a particular antigen, or whose constant region can elicit enhanced complement fixation, or to make other improvements in properties possessed by a particular constant region.

Another example is "altered antibodies", which refers to antibodies in which the naturally occurring amino acid sequence in a vertebrate antibody has been varied. Utilizing recombinant DNA techniques, antibodies can be redesigned to obtain desired characteristics. The possible variations are many, and range from the changing of one or more amino acids to the complete redesign of a region, for example, the constant region. Changes in the constant region, in general, to attain desired cellular process characteristics, e.g., changes in complement fixation, interaction with membranes, and other effector functions. Changes in the variable region may be made to alter antigen binding characeristics. The antibody may also be engineered to aid the specific delivery of a molecule or substance to a specific cell or tissue site. The desired alterations may be made by known techniques in molecular biology, e.g., recombinant techniques, site directed mutagenesis, etc.

Yet another example are "univalent antibodies", which are aggregates comprised of a heavy chain/light chain dimer bound to the Fc (i.e., constant) region of a second heavy chain. This type of antibody escapes antigenic modulation. See, e.g., Glennie et al. (1982).

Included also within the definition of antibodies are "Fab" fragments of antibodies. The "Fab" region refers to those portions of the heavy and light chains which are roughly equivalent, or analogous, to the sequences which comprise the branch portion of the heavy and light chains, and which have been shown to exhibit immunological binding to a specified antigen, but which lack the effector Fc portion. "Fab" includes aggregates of one heavy and one light chain (commonly known as Fab'), as well as tetramers containing the 2H and 2L chains (referred to as F(ab)₂), which are capable of selectively reacting with a designated antigen or antigen family. "Fab" antibodies may be divided into subsets analogous to those described above, i.e, "vertebrate Fab", "hybrid Fab", "chimeric Fab", and "altered Fab". Methods of producing "Fab" fragments of antibodies are known within the art and include, for example, proteolysis, and synthesis by recombinant techniques.

### II.H. Diagnostic Oligonucleotide Probes and Kits

Using the disclosed portions of the isolated HCV cDNAs as a basis, oligomers of approximately 8 nucleotides or more can be prepared, either by excision or synthetically, which hybridize with the HCV genome and are useful in identification of the viral agent(s), further characterization of the viral genome(s), as well as in detection of the virus(es) in diseased individuals. The probes for HCV polynucleotides (natural or derived) are a length which allows the detection of unique viral sequences by hybridization. While 6-8 nucleotides may be a workable length, sequences of 10-12 nucleotides are preferred, and about 20 nucleotides appears optimal. Preferably, these sequences will derive from regions which lack heterogeneity. These probes can be prepared using routine methods, including automated oligonucleotide synthetic methods. Among useful probes, for example, are those derived from the newly isolated clones disclosed herein, as well as the various oligomers useful in probing cDNA libraries, set forth below. A complement to any unique portion of the HCV genome will be satisfactory. For use as probes, complete complementarity is desirable, though it may be unnecessary as the length of the fragment is increased.

For use of such probes as diagnostics, the biological sample to be analyzed, such as blood or serum, may be treated, if desired, to extract the nucleic acids contained therein. The resulting nucleic acid from the sample may be subjected to gel electrophoresis or other size separation techniques; alternatively, the nucleic acid sample may be dot blotted without size separation. The probes are then labeled. Suitable labels, and methods for labeling probes are known in the art, and include, for example, radioactive labels incorporated by nick translation or kinasing, biotin, fluorescent probes, and chemiluminescent probes. The nucleic acids extracted from the sample are then treated with the labeled probe under hybridization conditions of suitable stringencies, and polynucleotide duplexes containing the probe are detected.

The probes can be made completely complementary to the HCV genome. Therefore, usually high stringency conditions are desirable in order to prevent false positives. However, conditions of high stringency should only be used if the probes are complementary to regions of the viral genome which lack heterogeneity. The stringency of hybridization is determined by a number of factors during hybridization and during the washing procedure, including temperature, ionic strength, length of time, and concentration of formamide. These factors are outlined in, for example, Maniatis, T. (1982).

Generally, it is expected that the HCV genome sequences will be present in serum of infected individuals at relatively low levels, i.e., at approximately 10²-10³ chimp infectious doses (CID) per ml. This level may require that amplification techniques be used in hybridization assays. Such techniques are known in the art. For example, the Enzo Biochemical Corporation "Bio-Bridge" system uses terminal deoxynucleotide transferase to add unmodified 3'-poly-dT-tails to a DNA probe. The poly dT-tailed probe is hybridized to the target nucleotide sequence, and then to a biotin-modified poly-A. PCT application 84/03520 and EPA124221 describe a DNA hybridization assay in which: (1) analyte is annealed to a single-stranded DNA probe that is complementary to an enzyme-labeled oligonucleotide; and (2) the resulting tailed duplex is hybridized to an enzyme-labeled oligonucleotide. EPA 204510 describes a DNA hybridization assay in which analyte DNA is contacted with a probe that has a tail, such as a poly-dT tail, an amplifier strand that has a sequence that hybridizes to the tail of the probe, such as a poly-A sequence, and which is capable of binding a plurality of labeled strands. A particularly desirable technique may first involve amplification of the target HCV sequences in sera approximately 10,000 fold, i.e., to approximately 10⁶ sequences/ml. This may be accomplished, for example, by the polymerase chain reactions (PCR) technique described which is by Saiki et al. (1986), by Mullis, U.S. Patent No. 4,683,195, and by Mullis et al. U.S. Patent No. 4,683,202. The amplified sequence(s) may then be detected using a hybridization assay which is described in EP 317,077, published May 24, 1989. These hybridization assays, which should detect sequences at the level of 10⁶/ml, utilize nucleic acid multimers which bind to single-stranded analyte nucleic acid, and which also bind to a multiplicity of single-stranded labeled oligonucleotides. A suitable solution phase sandwich assay which may be used with labeled polynucleotide probes, and the methods for the preparation of probes is described in EPO 225,807, published June 16, 1987.

The probes can be packaged into diagnostic kits. Diagnostic kits include the probe DNA, which may be labeled; alternatively, the probe DNA may be unlabeled and the ingredients for labeling may be included in the kit in separate containers. The kit may also contain other suitably packaged reagents and materials needed for the particular hybridization protocol, for example, standards, as well as instructions for conducting the test.

### Immunoassay and Diagnostic Kits

Both the polypeptides which react immunologically with serum containing HCV antibodies, for example, those detected by the antigenic screening method described infra in the Examples, as well those derived from or encoded within the isolated clones described in the Examples, and composites thereof, and the antibodies raised against the HCV specific epitopes in these polypeptides, are useful in immunoassays to detect presence of HCV antibodies, or the presence of the virus and/ or viral antigens, in biological samples. Design of the immunoassays is subject to a great deal of variation, and a variety of these are known in the art. For example, the immunoassay may utilize one viral epitope; alternatively, the immunoassay may use a combination of viral epitopes derived from these sources; these epitopes may be derived from the same or from different viral polypeptides, and may be in separate recombinant or natural polypeptides, or together in the same recombinant polypeptides. It may use, for example, a monoclonal antibody directed towards a viral epitope(s), a combination of monoclonal antibodies directed towards epitopes of one viral antigen, monoclonal antibodies directed towards epitopes of different viral antigens, polyclonal antibodies directed towards the same viral antigen, or polyclonal antibodies directed towards different viral antigens. Protocols may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

Some of the antigenic regions of the putative polyprotein have been mapped and identified by screening the antigenicitiy of bacterial expression products of HCV cDNAs which encode portions of the polyprotein. See the Examples. Other antigenic regions of HCV may be detected by expressing the portions of the HCV cDNAs in other expression systems, including yeast systems and cellular systems derived from insects and vertebrates. In addition, studies giving rise to an antigenicity index and hydrophobicity/hydrophilicity profile give rise to information concerning the probability of a region's antigenicity.

The studies on antigenic mapping by expression of HCV cDNAs showed that a number of clones containing these cDNAs expressed polypeptides which were immunologically reactive with serum from individuals with NANBH. No single polypeptide was immunologically reactive with all sera. Five of these polypeptides were very immunogenic in that antibodies to the HCV epitopes in these polypeptides were detected in many different patient sera, although the overlap in detection was not complete. Thus, the results on the immunogenicity of the polypeptides encoded in the various clones suggest that effecient detection systems may include the use of panels of epitopes. The epitopes in the panel may be constructed into one or multiple polypeptides.

Kits suitable for immunodiagnosis and containing the appropriate labeled reagents are constructed by packaging the appropriate materials, including the polypeptides of the invention containing HCV epitopes or antibodies directed against HCV epitopes in suitable containers, along with the remaining reagents and materials required for the conduct of the assay, as well as a suitable set of assay instructions.

### Further Characterization of the HCV Genome, Virions, and Viral Antigens Using Probes Derived From cDNA to the Viral Genome

The HCV cDNA sequence information in the newly isolated clones described in the Examples may be used to gain further information on the sequence of the HCV genome, and for identification and isolation of the HCV agent, and thus will aid in its characterization including the nature of the genome, the structure of the viral particle, and the nature of the antigens of which it is composed. This information, in turn, can lead to additional polynucleotide probes, polypeptides derived from the HCV genome, and antibodies directed against HCV epitopes which would be useful for the diagnosis and/or treatment of HCV caused NANBH.

The cDNA sequence information in the above-mentioned clones is useful for the design of probes for the isolation of additional cDNA sequences which are derived from as yet undefined regions of the HCV genome(s) from which the cDNAs in clones described herein and in EP 0,316,218 are derived. For example, labeled probes containing a sequence of approximately 8 or more nucleotides, and preferably 20 or more nucleotides, which are derived from regions close to the 5'-termini or 3'-termini of the composite HCV cDNA sequence shown in Fig. 17 may be used to isolate overlapping cDNA sequences from HCV cDNA libraries. Alternatively, characterization of the genomic segments could be from the viral genome(s) isolated from purified HCV particles. Methods for purifying HCV particles and for detecting them during the purification procedure are described herein, infra. Procedures for isolating polynucleotide genomes from viral particles are known in the art, and one procedure which may be used is that described in EP 0,218,316. The isolated genomic segments could then be cloned and sequenced. An example of this technique, which utilizes amplification of the sequences to be cloned, is provided infra., and yielded clone 16jh.

Methods for constructing cDNA libraries are known in the art, and are discussed supra and infra; a method for the construction of HCV cDNA libraries in lambda-gt11 is discussed in EPO Pub. No. 318,216. However, cDNA libraries which are useful for screening with nucleic acid probes may also be constructed in other vectors known in the art, for example, lambda-gt10 (Huynh et al. (1985)).

### Screening for Anti-Viral Agents for HCV

The availability of cell culture and animal model systems for HCV makes it possible to screen for anti-viral agents which inhibit HCV replication, and particularly for those agents which preferentially allow cell growth and multiplication while inhibiting viral replication. These screening methods are known by those of skill in the art. Generally, the anti-viral agents are tested at a variety of concentrations, for their effect on preventing viral replication in cell culture systems which support viral replication, and then for an inhibition of infectivity or of viral pathogenicity (and a low level of toxicity) in an animal model system.

The methods and compositions provided herein for detecting HCV antigens and HCV polynucleotides are useful for screening of anti-viral agents in that they provide an alternative, and perhaps more sensitive means, for detecting the agent's effect on viral replication than the cell plaque assay or ID₅₀ assay. For example, the HCV-polynucleotide probes described herein may be used to quantitate the amount of viral nucleic acid produced in a cell culture. This could be accomplished, for example, by hybridization or competition hybridization of the infected cell nucleic acids with a labeled HCV-polynucleotide probe. For example, also, anti-HCV antibodies may be used to identify and quantitate HCV antigen(s) in the cell culture utilizing the immunoassays described herein. In addition, since it may be desirable to quantitate HCV antigens in the infected cell culture by a competition assay, the polypeptides encoded within the HCV cDNAs described herein are useful in these competition assays. Generally, a recombinant HCV polypeptide derived from the HCV cDNA would be labeled, and the inhibition of binding of this labeled polypeptide to an HCV polypeptide due to the antigen produced in the cell culture system would be monitored. Moreover, these techniques are particularly useful in cases where the HCV may be able to replicate in a cell line without causing cell death.

The anti-viral agents which may be tested for efficacy by these methods are known in the art, and include, for example, those which interact with virion components and/or cellular components which are necessary for the binding and/or replication of the virus. Typical anti-viral agents may include, for example, inhibitors of virion polymerase and/or protease(s) necessary for cleavage of the precursor polypeptides. Other anti-viral agents may include those which act with nucleic acids to prevent viral replication, for example, anti-sense polynucleotides, etc.

Antisense polynucleotides molecules are comprised of a complementary nucleotide sequence which allows them to hybridize specifically to designated regions of genomes or RNAs. Antisense polynucleotides may include, for example, molecules that will block protein translation by binding to mRNA, or may be molecules which prevent replication of viral RNA by transcriptase. They may also include molecules which carry agents (non-covalently attached or covalently bound) which cause the viral RNA to be inactive by causing, for example, scissions in the viral RNA. They may also bind to cellular polynucleotides which enhance and/or are required for viral infectivity, replicative ability, or chronicity. Antisense molecules which are to hybridize to HCV derived RNAs may be designed based upon the sequence information of the HCV cDNAs provided herein. The antiviral agents based upon anti-sense polynucleotides for HCV may be designed to bind with high specificity, to be of increased solubility, to be stable, and to have low toxicity. Hence, they may be delivered in specialized systems, for example, liposomes, or by gene therapy. In addition, they may include analogs, attached proteins, substituted or altered bonding between bases, etc.

Other types of drugs may be based upon polynucleotides which "mimic" important control regions of the HCV genome, and which may be therapeutic due to their interactions with key components of the system responsible for viral infectivity or replication.

### General Methods

The general techniques used in extracting the genome from a virus, preparing and probing a cDNA library, sequencing clones, constructing expression vectors, transforming cells, performing immunological assays such as radioimmunoassays and ELISA assays, for growing cells in culture, and the like are known in the art and laboratory manuals are available describing these techniques. However, as a general guide, the following sets forth some sources currently available for such procedures, and for materials useful in carrying them out.

Both prokaryotic and eukaryotic host cells may be used for expression of desired coding sequences when appropriate control sequences which are compatible with the designated host are used. Among prokaryotic hosts, E. coli is most frequently used. Expression control sequences for prokaryotes include promoters, optionally containing operator portions, and ribosome binding sites. Transfer vectors compatible with prokaryotic hosts are commonly derived from, for example, pBR322, a plasmid containing operons conferring ampicillin and tetracycline resistance, and the various pUC vectors, which also contain sequences conferring antibiotic resistance markers. These markers may be used to obtain successful transformants by selection. Commonly used prokaryotic control sequences include the Beta-lactamase (penicillinase) and lactose promoter systems (Chang et al. (1977)), the tryptophan (trp) promoter system (Goeddel et al. (1980)) and the lambda-derived P_{L} promoter and N gene ribosome binding site (Shimatake et al. (1981)) and the hybrid tac promoter (De Boer et al. (1983)) derived from sequences of the trp and lac UV5 promoters. The foregoing systems are particularly compatible with E. coli; if desired, other prokaryotic hosts such as strains of Bacillus or Pseudomonas may be used, with corresponding control sequences.

Eukaryotic hosts include yeast and mammalian cells in culture systems. Saccharomyces cerevisiae and Saccharomyces carlsbergensis are the most commonly used yeast hosts, and are convenient fungal hosts. Yeast compatible vectors carry markers which permit selection of successful transformants by conferring prototrophy to auxotrophic mutants or resistance to heavy metals on wild-type strains. Yeast compatible vectors may employ the 2 micron origin of replication (Broach et al. (1983)), the combination of CEN3 and ARS1 or other means for assuring replication, such as sequences which will result in incorporation of an appropriate fragment into the host cell genome. Control sequences for yeast vectors are known in the art and include promoters for the synthesis of glycolytic enzymes (Hess et al. (1968); Holland et al. (1978)), including the promoter for 3 phosphoglycerate kinase (Hitzeman (1980)). Terminators may also be included, such as those derived from the enolase gene (Holland (1981)). Particularly useful control systems are those which comprise the glyceraldehyde-3 phosphate dehydrogenase (GAPDH) promoter or alcohol dehydrogenase (ADH) regulatable promoter, terminators also derived from GAPDH, and if secretion is desired, leader sequence from yeast alpha factor. In addition, the transcriptional regulatory region and the transcriptional initiation region which are operably linked may be such that they are not naturally associated in the wild-type organism. These systems are described in detail in EPO 120,551, published October 3, 1984; EPO 116, 201, published August 22, 1984; and EPO 164,556, published December 18, 1985, all of which are assigned to the herein assignee, and are hereby incorporated herein by reference.

Mammalian cell lines available as hosts for expression are known in the art and include many immortalized cell lines available from the American Type Culture Collection (ATCC), including HeLa cells, Chinese hamster ovary (CHO) cells, baby hamster kidney (BHK) cells, and a number of other cell lines. Suitable promoters for mammalian cells are also known in the art and include viral promoters such as that from Simian Virus 40 (SV40) (Fiers (1978)), Rous sarcoma virus (RSV), adenovirus (ADV), and bovine papilloma virus (BPV). Mammalian cells may also require terminator sequences and poly A addition sequences; enhancer sequences which increase expression may also be included, and sequences which cause amplification of the gene may also be desirable. These sequences are known in the art. Vectors suitable for replication in mammalian cells may include viral replicons, or sequences which insure integration of the appropriate sequences encoding NANBV epitopes into the host genome.

Transformation may be by any known method for introducing polynucleotides into a host cell, including, for example packaging the polynucleotide in a virus and transducing a host cell with the virus, and by direct uptake of the polynucleotide. The transformation procedure used depends upon the host to be transformed. For example, transformation of the E. coli host cells with lambda-gt11 containing BB-NANBV sequences is discussed in the Example section, infra. Bacterial transformation by direct uptake generally employs treatment with calcium or rubidium chloride (Cohen (1972); Maniatis (1982)). Yeast transformation by direct uptake may be carried out using the method of Hinnen et al. (1978). Mammalian transformations by direct uptake may be conducted using the calcium phosphate precipitation method of Graham and Van der Eb (1978), or the various known modifications thereof.

Vector construction employs techniques which are known in the art. Site-specific DNA cleavage is performed by treating with suitable restriction enzymes under conditions which generally are specified by the manufacturer of these commercially available enzymes. In general, about 1 microgram of plasmid or DNA sequence is cleaved by 1 unit of enzyme in about 20 microliters buffer solution by incubation of 1-2 hr at 37° C. After incubation with the restriction enzyme, protein is removed by phenol/ chloroform extraction and the DNA recovered by precipitation with ethanol. The cleaved fragments may be separated using polyacrylamide or agarose gel electrophoresis techniques, according to the general procedures found in Methods in Enzymology (1980) 65:499-560.

Sticky ended cleavage fragments may be blunt ended using E. coli DNA polymerase I (Klenow) in the presence of the appropriate deoxynucleotide triphosphates (dNTPs) present in the mixture. Treatment with S1 nuclease may also be used, resulting in the hydrolysis of any single stranded DNA portions.

Ligations are carried out using standard buffer and temperature conditions using T4 DNA ligase and ATP; sticky end ligations require less ATP and less ligase than blunt end ligations. When vector fragments are used as part of a ligation mixture, the vector fragment is often treated with bacterial alkaline phosphatase (BAP) or calf intestinal alkaline phosphatase to remove the 5'-phosphate and thus prevent religation of the vector; alternatively, restriction enzyme digestion of unwanted fragments can be used to prevent ligation.

Ligation mixtures are transformed into suitable cloning hosts, such as E. coli, and successful transformants selected by, for example, antibiotic resistance, and screened for the correct construction.

Synthetic oligonucleotides may be prepared using an automated oligonucleotide synthesizer as described by Warner (1984). If desired the synthetic strands may be labeled with ³²P by treatment with polynucleotide kinase in the presence of ³²P-ATP, using standard conditions for the reaction.

DNA sequences, including those isolated from cDNA libraries, may be modified by known techniques, including, for example site directed mutagenesis, as described by Zoller (1982). Briefly, the DNA to be modified is packaged into phage as a single stranded sequence, and converted to a double stranded DNA with DNA polymerase using, as a primer, a synthetic oligonucleotide complementary to the portion of the DNA to be modified, and having the desired modification included in its own sequence. The resulting double stranded DNA is transformed into a phage supporting host bacterium. Cultures of the transformed bacteria, which contain replications of each strand of the phage, are plated in agar to obtain plaques. Theoretically, 50% of the new plaques contain phage having the mutated sequence, and the remaining 50% have the original sequence. Replicates of the plaques are hybridized to labeled synthetic probe at temperatures and conditions which permit hybridization with the correct strand, but not with the unmodified sequence. The sequences which have been identified by hybridization are recovered and cloned.

DNA libraries may be probed using the procedure of Grunstein and Hogness (1975). Briefly, in this procedure, the DNA to be probed is immobilized on nitrocellulose filters, denatured, and prehybridized with a buffer containing 0-50% formamide, 0.75 M NaCl, 75 mM Na citrate, 0.02% (wt/v) each of bovine serum albumin, polyvinyl pyrollidone, and Ficoll, 50 mM Na Phosphate (pH 6.5), 0.1% SDS, and 100 micrograms/ml carrier denatured DNA. The percentage of formamide in the buffer, as well as the time and temperature conditions of the prehybridization and subsequent hybridization steps depends on the stringency required. Oligomeric probes which require lower stringency conditions are generally used with low percentages of formamide, lower temperatures, and longer hybridization times. Probes containing more than 30 or 40 nucleotides such as those derived from cDNA or genomic sequences generally employ higher temperatures, e.g., about 40-42°C, and a high percentage, e.g., 50%, formamide. Following prehybridization, 5'- ³²P-labeled oligonucleotide probe is added to the buffer, and the filters are incubated in this mixture under hybridization conditions. After washing, the treated filters are subjected to autoradiography to show the location of the hybridized probe; DNA in corresponding locations on the original agar plates is used as the source of the desired DNA.

For routine vector constructions, ligation mixtures are transformed into E. coli strain HB101 or other suitable host, and successful transformants selected by antibiotic resistance or other markers. Plasmids from the transformants are then prepared according to the method of Clewell et al. (1969), usually following chloramphenicol amplification (Clewell (1972)). The DNA is isolated and analyzed, usually by restriction enzyme analysis and/or sequencing. Sequencing may be by the dideoxy method of Sanger et al. (1977) as further described by Messing et al. (1981), or by the method of Maxam et al. (1980). Problems with band compression, which are sometimes observed in GC rich regions, were overcome by use of T-deazoguanosine according to Barr et al. (1986).

The enzyme-linked immunosorbent assay (ELISA) can be used to measure either antigen or antibody concentrations. This method depends upon conjugation of an enzyme to either an antigen or an antibody, and uses the bound enzyme activity as a quantitative label. To measure antibody, the known antigen is fixed to a solid phase (e.g., a microplate or plastic cup), incubated with test serum dilutions, washed, incubated with anti-immunoglobulin labeled with an enzyme, and washed again. Enzymes suitable for labeling are known in the art, and include, for example, horseradish peroxidase. Enzyme activity bound to the solid phase is measured by adding the specific substrate, and determining product formation or substrate utilization colorimetrically. The enzyme activity bound is a direct function of the amount of antibody bound.

To measure antigen, a known specific antibody is fixed to the solid phase, the test material containing antigen is added, after an incubation the solid phase is washed, and a second enzyme-labeled antibody is added. After washing, substrate is added, and enzyme activity is estimated colorimetrically, and related to antigen concentration.

### Examples

Described below are examples of the present invention which are provided only for illustrative purposes, and not to limit the scope of the present invention. In light of the present disclosure, numerous embodiments within the scope of the claims will be apparent to those of ordinary skill in the art.

### Isolation and Sequence of Overlapping HCV cDNA Clones 13i, 26j, CA59a, CA84a, CA156e and CA167b

The clones 13i, 26j, CA59a, CA84a, CA156e and CA167b were isolated from the lambda-gt11 library which contains HCV cDNA (ATCC No. 40394), the preparation of which is described in EPO Pub. No. 318,216 (published 31 May 1989), and WO 89/04669 (published 1 June 1989). Screening of the library was with the probes described infra., using the method described in Huynh (1985). The frequencies with which positive clones appeared with the respective probes was about 1 in 50,000.

The isolation of clone 13i was accomplished using a synthetic probe derived from the sequence of clone 12f. The sequence of the probe was:

5′ GAA CGT TGC GAT CTG GAA GAC AGG GAC AGG 3′.

The isolation of clone 26j was accomplished using a probe derived from the 5'-region of clone K9-1. The sequence of the probe was:

5′ TAT CAG TTA TGC CAA CGG AAG CGG CCC CGA 3′.

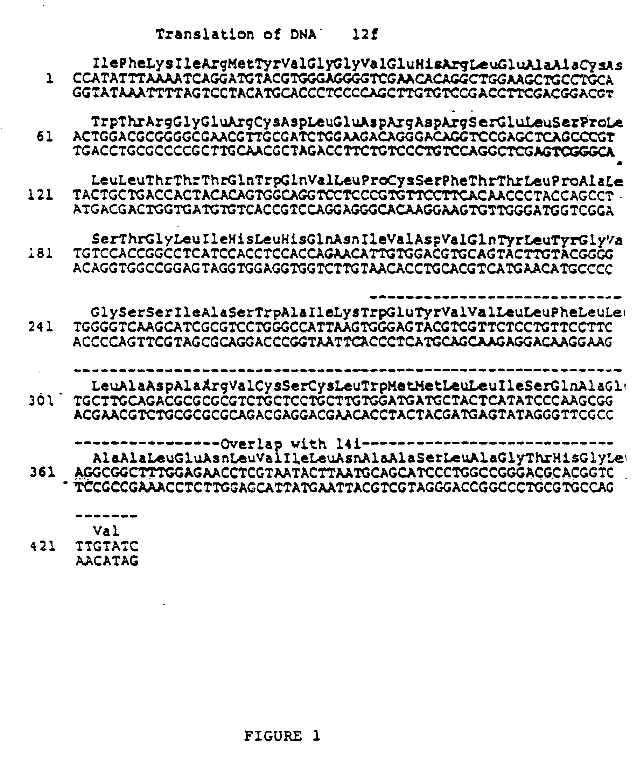

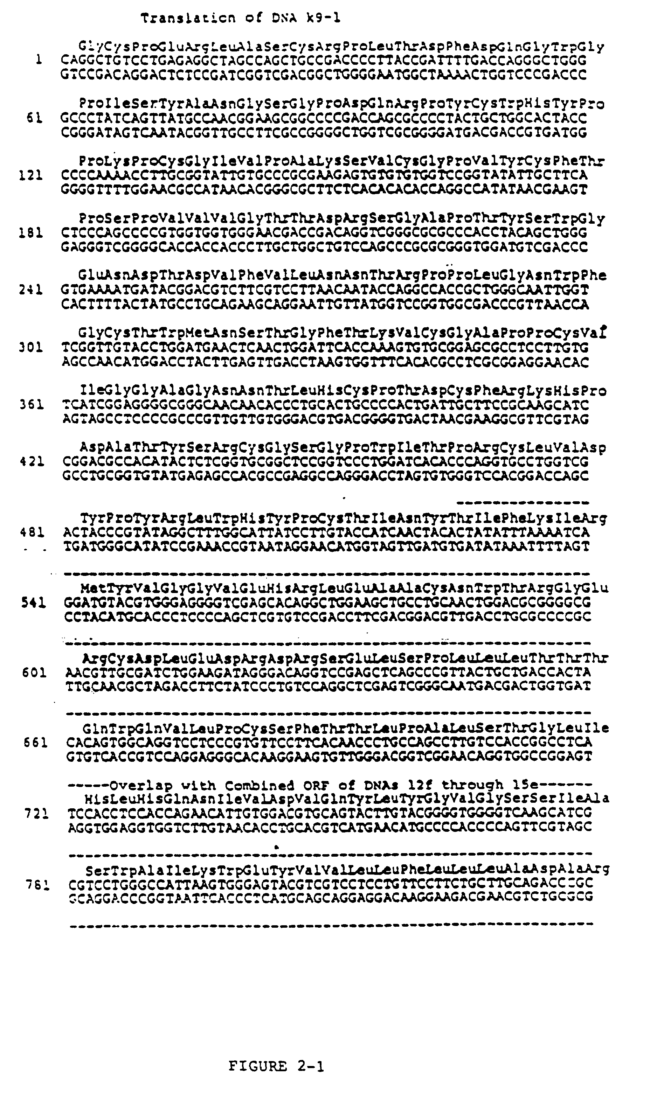

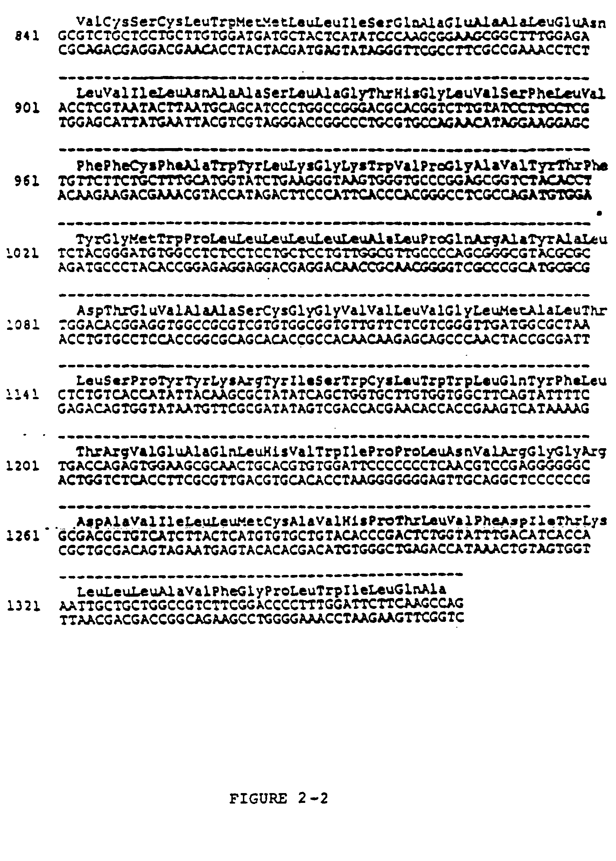

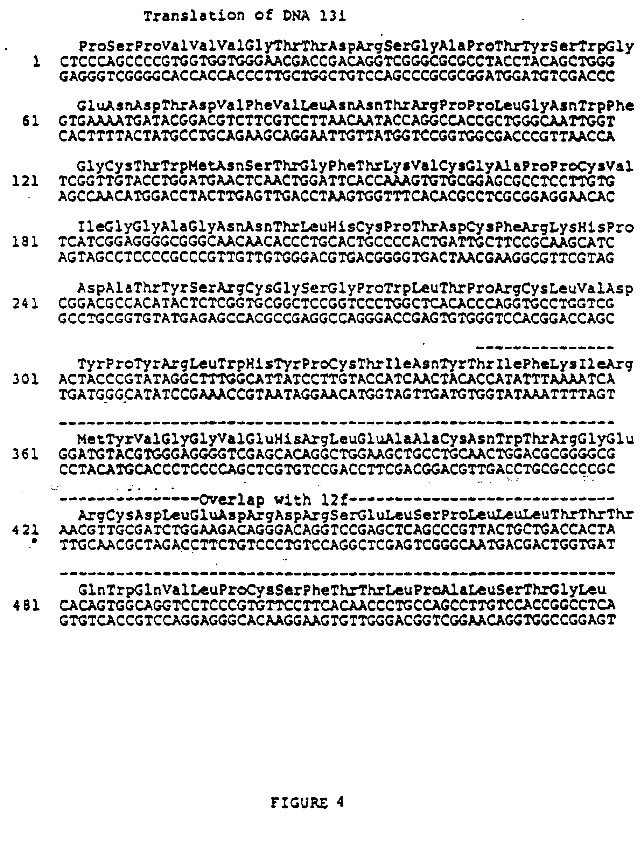

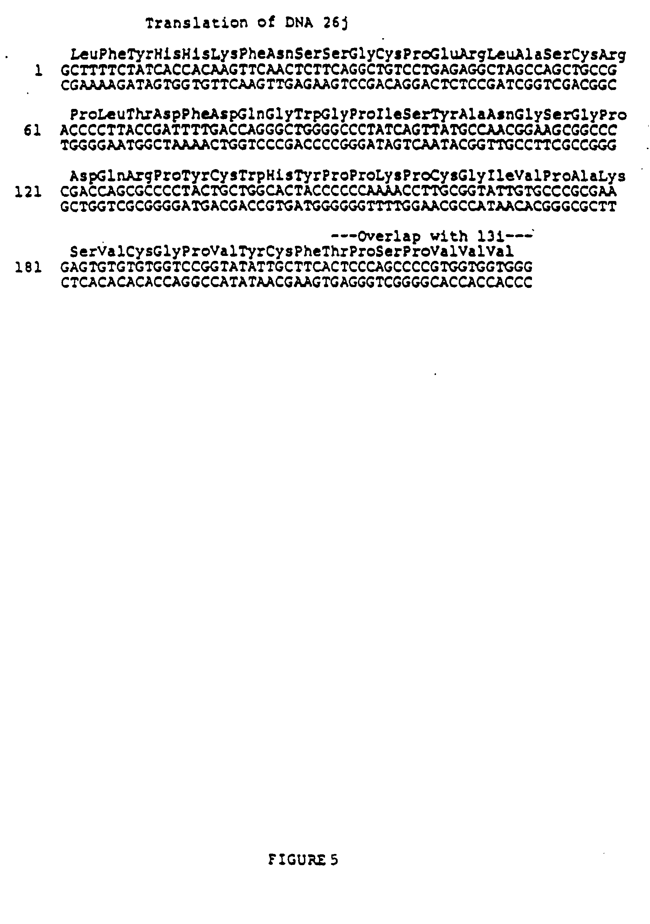

The isolation procedures for clone 12f and for clone k9-1 (also called K9-1) are described in EPO Pub. No. 318,216, and their sequences are shown in Figs. 1 and 2, respectively. The HCV cDNA sequences of clones 13i and 26j, are shown in Figs. 4 and 5, respectively. Also shown are the amino acids encoded therein, as well as the overlap of clone 13i with clone 12f, and the overlap of clone 26j with clone 13i. The sequences for these clones confirmed the sequence of clone K9-1. Clone K9-1 had been isolated from a different HCV cDNA library (See EP 0,218,316).

Clone CA59a was isolated utilizing a probe based upon the sequence of the 5'-region of clone 26j. The sequence of this probe was:

5′ CTG GTT AGC AGG GCT TTT CTA TCA CCA CAA 3′.

A probe derived from the sequence of clone CA59a was used to isolate clone CA84a. The sequence of the probe used for this isolation was:

5′ AAG GTC CTG GTA GTG CTG CTG CTA TTT GCC 3′.

Clone CA156e was isolated using a probe derived from the sequence of clone CA84a. The sequence of the probe was:

5′ ACT GGA CGA CGC AAG GTT GCA ATT GCT CTA 3′.

Clone CA167b was isolated using a probe derived from the sequence of clone CA 156e. The sequence of the probe was:

5′ TTC GAC GTC ACA TCG ATC TGC TTG TCG GGA 3′.

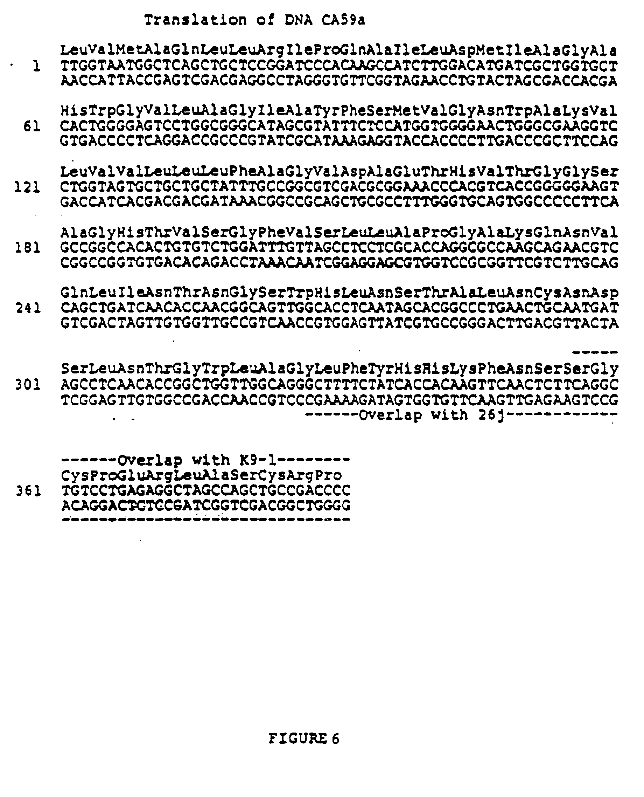

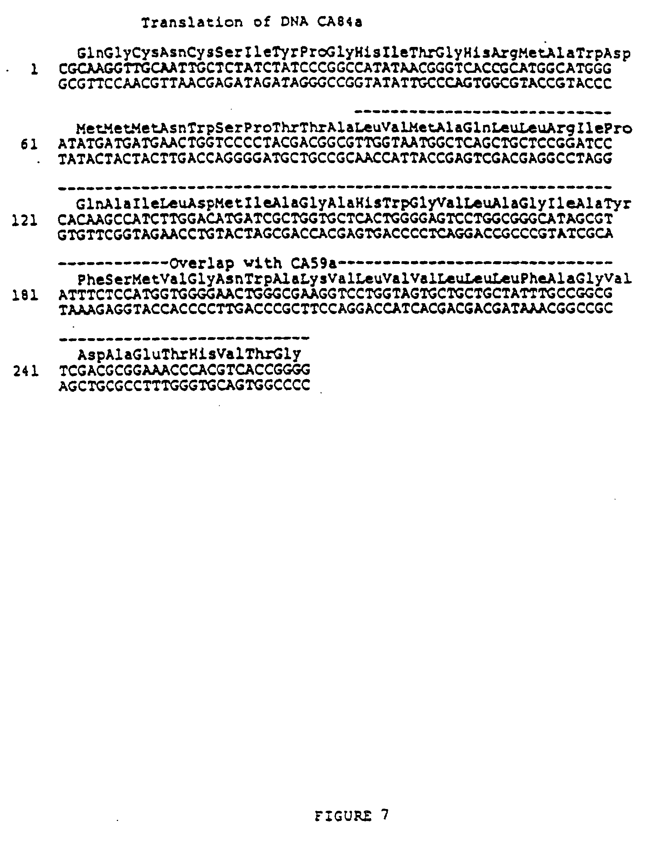

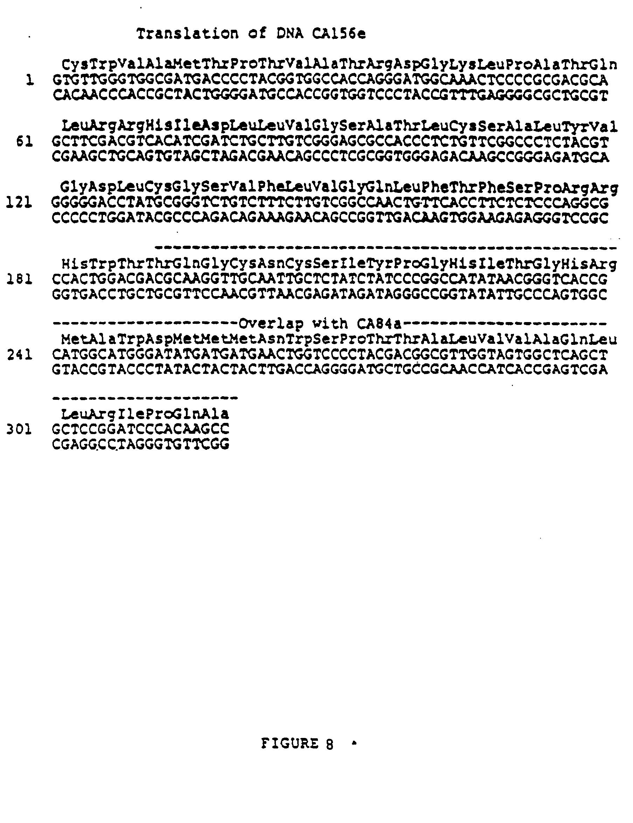

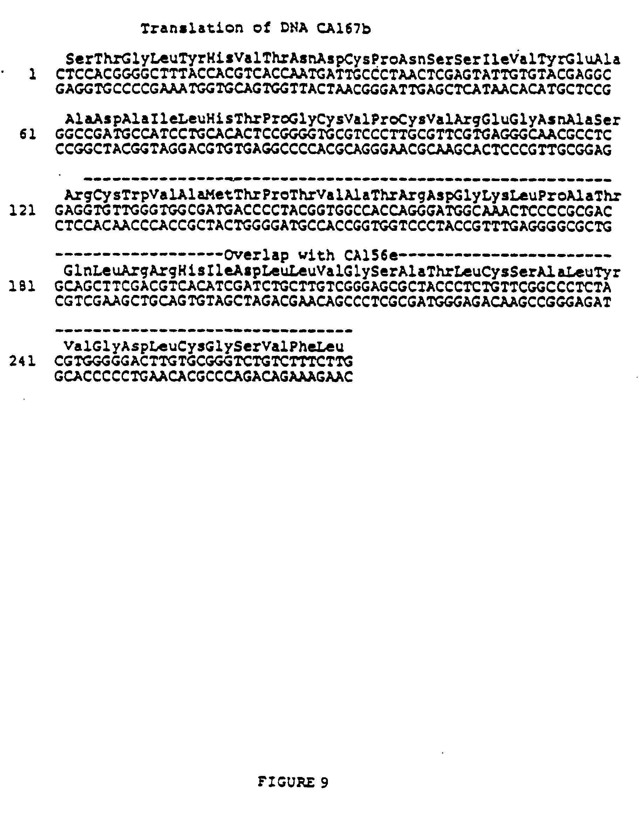

The nucleotide sequences of the HCV cDNAs in clones CA59a, CA84a, CA156e, and CA167b, are shown Figs. 6, 7, 8, and 9, respectively. The amino acids encoded therein, as well as the overlap with the sequences of relevant clones, are also shown in the Figs.

### Creation of "pi" HCV cDNA Library

A library of HCV cDNA, the "pi" library, was constructed from the same batch of infectious chimpanzee plasma used to construct the lambda-gt11 HCV cDNA library (ATCC No. 40394) described in EPO Pub. No. 318,216, and utilizing essentially the same techniques. However, construction of the pi library utilized a primer-extension method, in which the primer for reverse transcriptase was based on the sequence of clone CA59A. The sequence of the primer was:

5′ GGT GAC GTG GGT TTC 3′.

### Isolation and Sequence of Clone pi14a

Screening of the "pi" HCV cDNA library described supra., with the probe used to isolate clone CA167b (See supra.) yielded clone pil4a. The clone contains about 800 base pairs of cDNA which overlaps clones CA167b, CA156e, CA84a and CA59a, which were isolated from the lambda gt-11 HCV cDNA library (ATCC No. 40394). In addition, pi14a also contains about 250 base pairs of DNA which are upstream of the HCV cDNA in clone CA167b.

### Isolation and Sequence of Clones CA216a, CA290a and ag30a

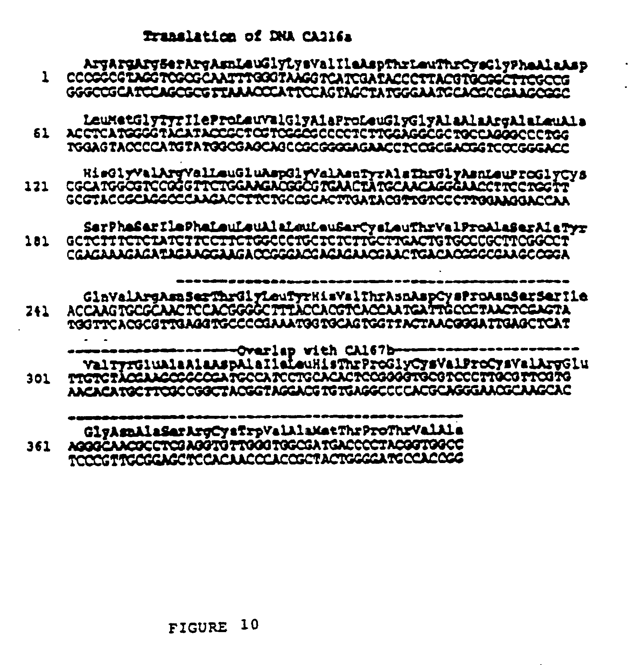

Based on the sequence of clone CA167b a synthetic probe was made having the following sequence: 5′ GGC TTT ACC ACG TCA CCA ATG ATT GCC CTA 3′ The above probe was used to screen the lambda-gt11 library (ATCC No. 40394), which yielded clone CA216a, whose HCV sequences are shown in Fig. 10.

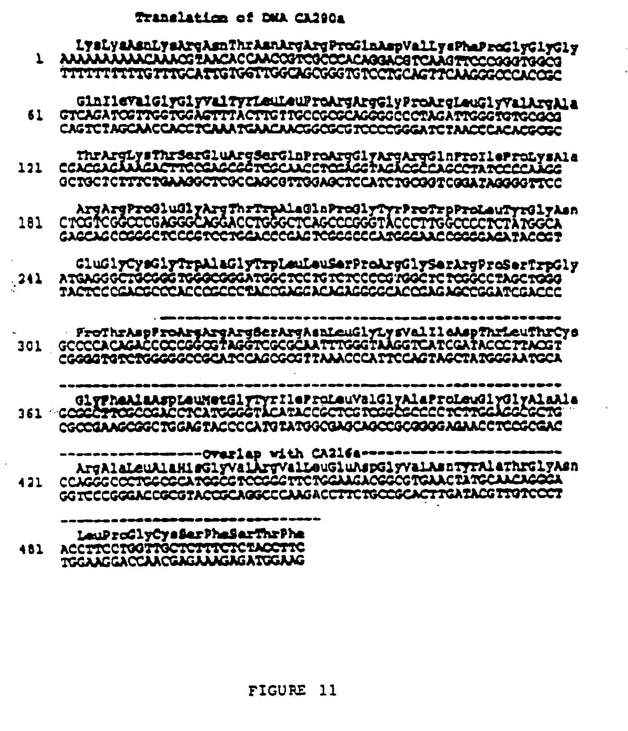

Another probe was made based on the sequence of clone CA216a having the following sequence: 5' TTT GGG TAA GGT CAT CGA TAC CCT TAC GTG 3' Screening the 'lambda-gt11 library (ATCC No. 40394)' with this probe yielded clone CA290a, the HCV sequences therein being shown in Fig. 11.

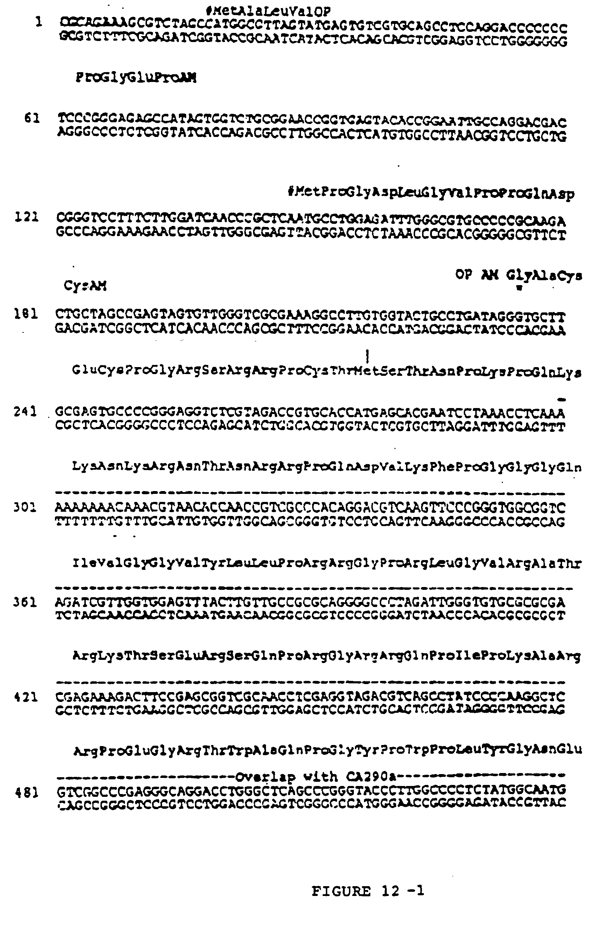

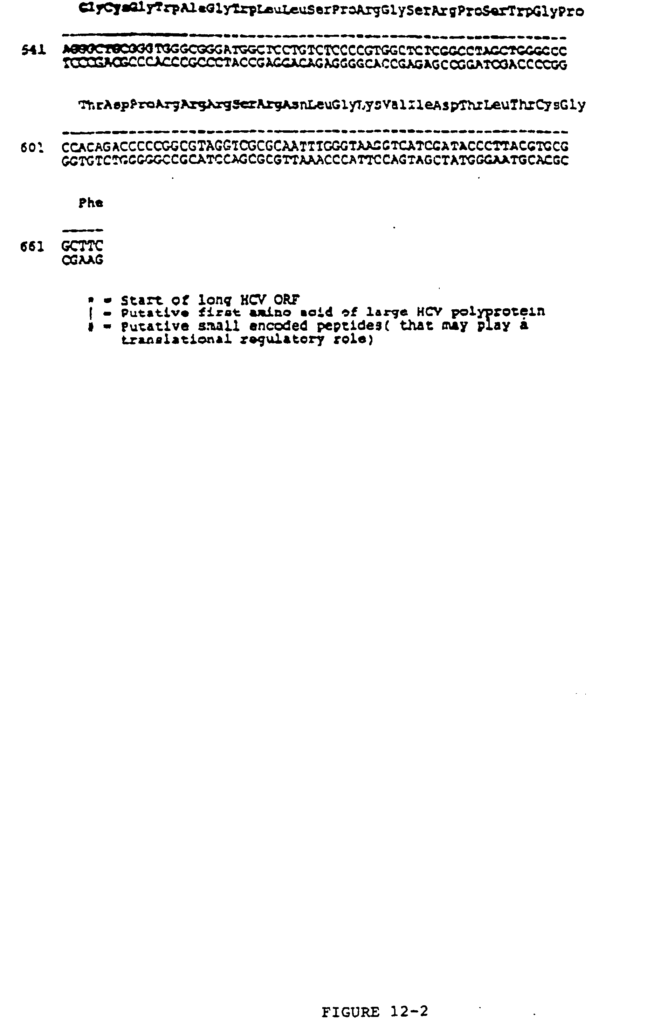

. In a parallel approach, a primer-extension cDNA library was made using nucleic acid extracted from the same infectious plasma used in the original lambda-gtll cDNA library described above. The primer used was based on the sequence of clones CA216a and CA290a: 5' GAA GCC GCA CGT AAG 3' The cDNA library was made using methods similar to those described previously for libraries used in the isolation of clones pi14a and k9-1. The probe used to screen this library was based on the sequence of clone CA290a: 5′ CCG GCG TAG GTC GCG CAA TTT GGG TAA 3′ Clone ag30a was isolated from the new library with the above probe, and contained about 670 basepairs of HCV sequence. See Fig. 12. Part of this sequence overlaps the HCV sequence of clones CA216a and CA290a. About 300 , base-pairs of the ag30a sequence, however, is upstream of the sequence from clone CA290a. The non-overlapping sequence shows a start codon (*) and stop codons that may indicate the start of the HCV ORF. Also indicated in Fig. 12 are putative small encoded peptides (#) which may play a role in regulating translation, as well as the putative first amino acid of the putative polypeptide (/), and downstream amino acids encoded therein.

### Isolation and Sequence of Clone CA205a

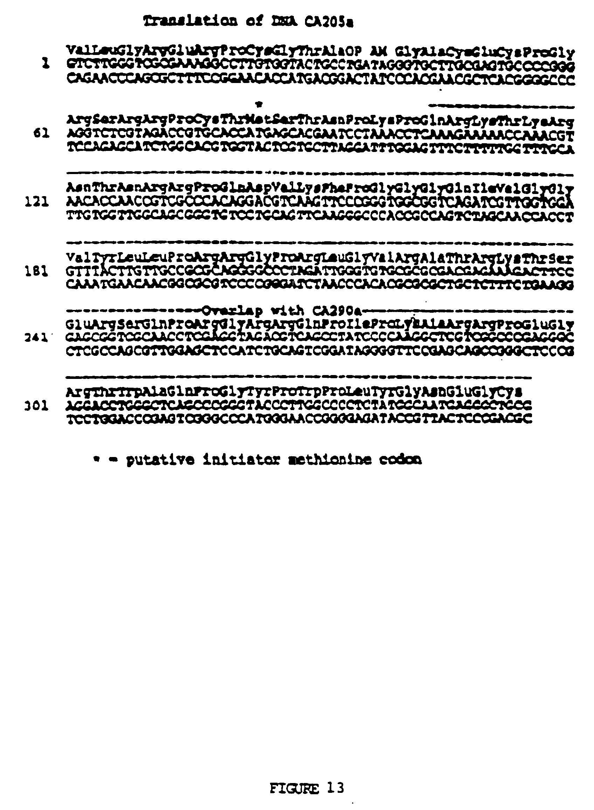

Clone CA205a was isolated from the original lambda gt-11 library (ATCC No. 40394), using a synthetic probe derived from the HCV sequence in clone CA290a (Fig. 11). The sequence of the probe was: 5′ TCA GAT CGT TGG TGG AGT TTA CTT GTT GCC 3′. The sequence of the HCV cDNA in CA205a, shown in Fig. 13, overlaps with the cDNA sequences in both clones ag30a and CA290a. The overlap of the sequence with that of CA290a is shown by the dotted line above the sequence (the figure also shows the putative amino acids encoded in this fragment).

As observed from the HCV cDNA sequences in clones CA205a and ag30a, the putative HCV polyprotein appears to begin at the ATG start codon; the HCV sequences in both clones contain an in-frame, contiguous double stop codon (TGATAG) forty two nucleotides upstream from this ATG. The HCV ORF appears to begin after these stop codons, and to extend for at least 8907 nucleotides (See the composite HCV cDNA shown in Fig. 17).

### Isolation and Sequence of Clone 18g

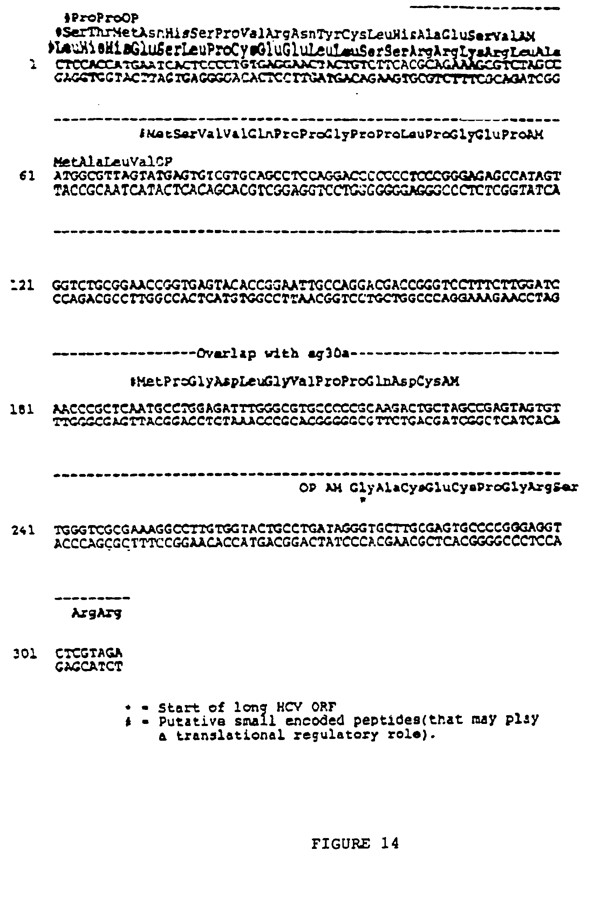

Based on the sequence of clone ag30a (See Fig. 12) and of an overlapping clone from the original lambda gt-11 library (ATCC No. 40394), CA230a, a synthetic probe was made having the following sequence: 5′ CCA TAG TGG TCT GCG GAA CCG GTG AGT ACA 3′. Screening of the original lambda-gt11 HCV cDNA library with the probe yielded clone 18g, the HCV cDNA sequence of which is shown in Fig. 14. Also shown in the figure are the overlap with clone ag30a, and putative polypeptides encoded within the HCV cDNA.

The cDNA in clone 18g (C18g or 18g) overlaps that in clones ag30a and CA205a, described supra. The sequence of C18g also contains the double stop codon region observed in clone ag30a. The polynucleotide region upstream of these stop codons presumably represents part of the 5'-region of the HCV genome, which may contain short ORFs, and which can be confirmed by direct sequencing of the purified HCV genome. These putative small encoded peptides may play a regulatory role in translation. The region of the HCV genome upstream of that represented by C18g can be isolated for sequence analysis using essentially the technique described in EPO Pub. No. 318,216 for isolating cDNA sequences upstream of the HCV cDNA sequence in clone 12f. Essentially, small synthetic oligonucleotide primers of reverse transcriptase, which are based upon the sequence of C18g, are synthesized and used to bind to the corresponding sequence in HCV genomic RNA. The primer sequences are proximal to the known 5'-terminal of C18g, but sufficiently downstream to allow the design of probe sequences upstream of the primer sequences. Known standard methods of priming and cloning ar eused. The resulting cDNA libraries are screened with sequences upstream of the priming sites (as deduced from the elucidated sequence of C18g). The HCV genomic RNA is obtained from either plasma or liver samples from individuals with NANBH. Since HCV appears to be a Flavi-like virus, the 5'-terminus of the genome may be modified with a "cap" structure. It is known that Flavivirus genomes contain 5'-terminal "cap" structures. (Yellow Fever virus, Rice et al. (1988); Dengue virus, Hahn et al (1988); Japanese Encephalitis Virus (1987)).

### Isolation and Sequence of Clones from the beta-HCV cDNA library

Clones containing cDNA representative of the 3'-terminal region of the HCV genome were isolated from a cDNA library constructed from the original infectious chimpanzee plasma pool which was used for the creation of the HCV cDNA lambda-gt11 library (ATCC No. 40394), described in EPO Pub. No. 318,216. In order to create the DNA library, RNA extracted from the plasma was "tailed" with poly rA using poly (rA) polymerase, and cDNA was synthesized using oligo(dT)₁₂₋₁₈ as a primer for reverse transcriptase. The resulting RNA:cDNA hybrid was digested with RNAase H, and converted to double stranded HCV cDNA. The resulting HCV cDNA was cloned into lambda-gt10, using essentially the technique described in Huynh (1985), yielding the beta (or b) HCV cDNA library. The procedures used were as follows.

An aliquot (12ml) of the plasma was treated with proteinase K, and extracted with an equal volume of phenol saturated with 0.05M Tris-Cl, pH 7.5, 0.05% (v/v) beta-mercaptoethanol, 0.1% (w/v) hydroxyquinolone, 1 mM EDTA. The resulting aqueous phase was re-extracted with the phenol mixture, followed by 3 extractions with a 1:1 mixture containing phenol and chloroform:isoamyl alcohol (24:1), followed by 2 extractions with a mixture of chloroform and isoamyl alcohol (1:1). Subsequent to adjustment of the aqueous phase to 200 mM with respect to NaCl, nucleic acids in the aqueous phase were precipitated overnight at -20°C, with 2.5 volumes of cold absolute ethanol. The precipitates were collected by centrifugation at 10,000 RPM for 40 min., washed with 70% ethanol containing 20 mM NaCl, and with 100% cold ethanol, dried for 5 min. in a dessicator, and dissolved in water.

The isolated nucleic acids from the infectious chimpanzee plasma pool were tailed with poly rA utilizing poly-A polymerase in the presence of human placenta ribonuclease inhibitor (HPRI) (purchased from Amersham Corp.), utilizing MS2 RNA as carrier. Isolated nucleic acids equivalent to that in 2 ml of plasma were incubated in a solution containing TMN (50 mM Tris HCl, pH 7.9, 10 mM MgCl₂, 250 mM NaCl, 2.5 mM MnCl₂, 2 mM dithiothreitol (DTT)), 40 micromolar alpha-[³²P] ATP, 20 units HPRI (Amersham Corp.), and about 9 to 10 units of RNase free poly-A polymerase (BRL). Incubation was for 10 min. at 37°C, and the reactions were stopped with EDTA (final concentration about 250 mM). The solution was extracted with an equal volume of phenol-chloroform, and with an equal volume of chloroform, and nucleic acids were precipitated overnight at -20°C with 2.5 volumes of ethanol in the presence of 200 mM NaCl.

### Isolation of Clone b5a

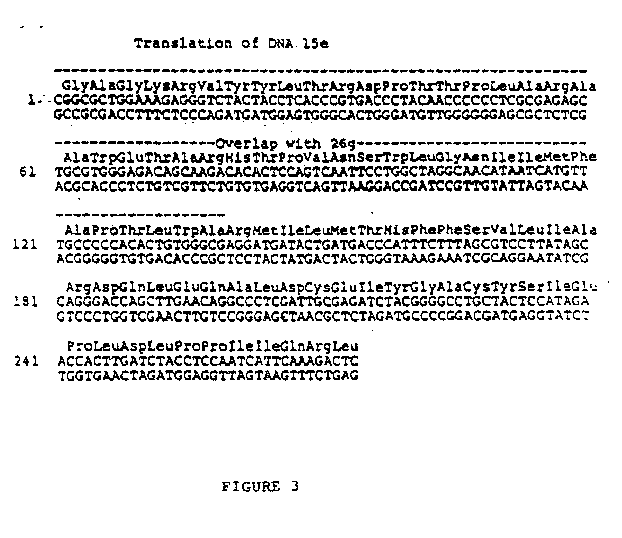

The beta HCV cDNA library was screened by hybridization using a synthetic probe, which had a sequence based upon the HCV cDNA sequence in clone 15e. The isolation of clone 15e is described in EPO Pub. No. 318,216, and its sequence is shown in Fig. 3. The sequence of the synthetic probe was: 5′ ATT GCG AGA TCT ACG GGG CCT GCT ACT CCA 3′. Screening of the library yielded clone beta-5a (b5a), which contains an HCV cDNA region of approximately 1000 base pairs. The 5'-region of this cDNA overlaps clones 35f, 19g, 26g, and 15e (these clones are described supra). The region between the 3'-terminal poly-A sequence and the 3'-sequence which overlaps clone 15e, contains approximately 200 base pairs. This clone allows the identification of a region of the 3'-terminal sequence the HCV genome.

The sequence of b5a is contained within the sequence of the HCV cDNA in clone 16jh (described infra). Moreover, the sequence is also present in CC34a, isolated from the original lambda-gt11 library (ATCC No. 40394). (The original lambda-gt11 library is referred to herein as the "C" library).

### Isolation and Sequence of Clones Generated by PCR Amplification of the 3'-Region of the HCV Genome

Multiple cDNA clones have been generated which contain nucleotide sequences derived from the 3'-region of the HCV genome. This was accomplished by amplifying a targeted region of the genome by a polymerase chain reaction technique described in Saiki et al. (1986), and in Saiki et al. (1988), which was modified as described below. The HCV RNA which was amplified was obtained from the original infectious chimpanzee plasma pool which was used for the creation of the HCV cDNA lambda-gt11 library (ATCC No. 40394) described in EPO Pub. No. 318,216. Isolation of the HCV RNA was as described supra. The isolated RNA was tailed at the 3'-end with ATP by E. coli poly-A polymerase as described in Sippel (1973), except that the nucleic acids isolated from chimp serum were substituted for the nucleic acid substrate. The tailed RNA was then reverse transcribed into cDNA by reverse transcriptase, using an oligo dT-primer adapter, essentially as described by Han (1987), except that the components and sequence of the primer-adapter were:

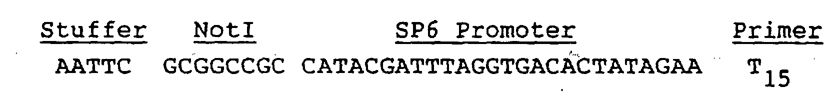

The resultant cDNA was subjected to amplification by PCR using two primers:

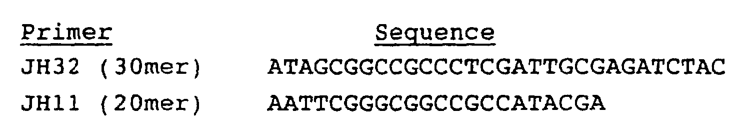

The JH32 primer contained 20 nucleotide sequences hybridizable to the 5'-end of the target region in the cDNA, with an estimated Tₘ of 66°C. The JH11 was derived from a portion of the oligo dT-primer adapter; thus, it is specific to the 3'-end of the cDNA with a Tₘ of 64°C. Both primers were designed to have a recognition site for the restriction enzyme, NotI, at the 5'-end, for use in subsequent cloning of the amplified HCV cDNA.

The PCR reaction was carried out by suspending the cDNA and the primers in 100 microliters of reaction mixture containing the four deoxynucleoside triphosphates, buffer salts and metal ions, and a thermostable DNA polymerase isolated from Thermus aquaticus (Taq polymerase), which are in a Perkin Elmer Cetus PCR kit (N801-0043 or N801-0055). The PCR reaction was performed for 35 cycles in a Perkin Elmer Cetus DNA thermal cycler. Each cycle consisted of a 1.5 min denaturation step at 94°C, an annealing step at 60°C for 2 min, and a primer extension step at 72°C for 3 min. The PCR products were subjected to Southern blot analysis using a 30 nucleotide probe, JH34, the sequence of which was based upon that of the 3'-terminal region of clone 15e. The sequence of JH34 is : 5′ CTT GAT CTA CCT CCA ATC ATT CAA AGA CTC 3′. The PCR products detected by the HCV cDNA probe ranged in size from about 50 to about 400 base pairs.

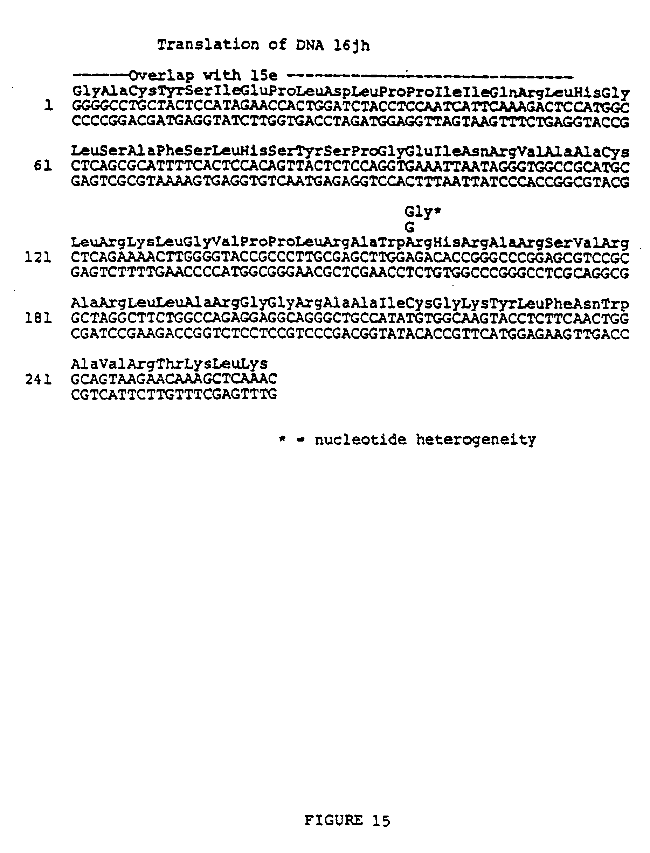

In order to clone the amplified HCV cDNA, the PCR products were cleaved with NotI and size selected by polyacrylamide gel electrophoresis. DNA larger than 300 base pairs was cloned into the NotI site of pUC18S The vector pUC18S is constructed by including a NotI polylinker cloned between the EcoRI and SalI sites of pUC18. The clones were screened for HCV cDNA using the JH34 probe. A number of positive clones were obtained and sequenced. The nucleotide sequence of the HCV cDNA insert in one of these clones, 16jh, and the amino acids encoded therein, are shown in Fig. 15. A nucleotide heterogeneity, detected in the sequence of the HCV cDNA in clone 16jh as compared to another clone of this region, is indicated in the figure.

### Compiled HCV cDNA Sequences

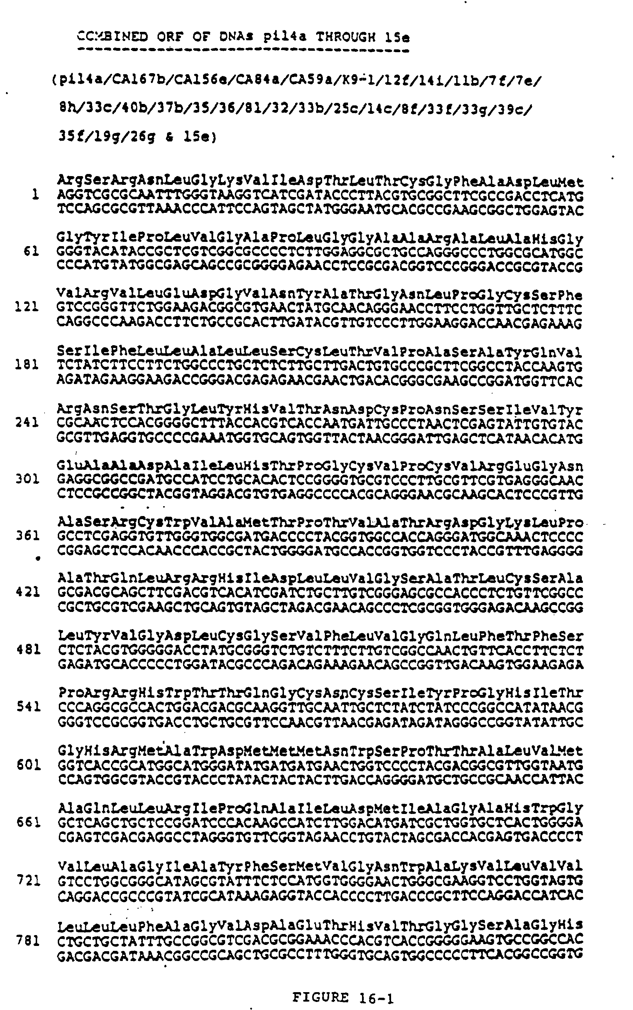

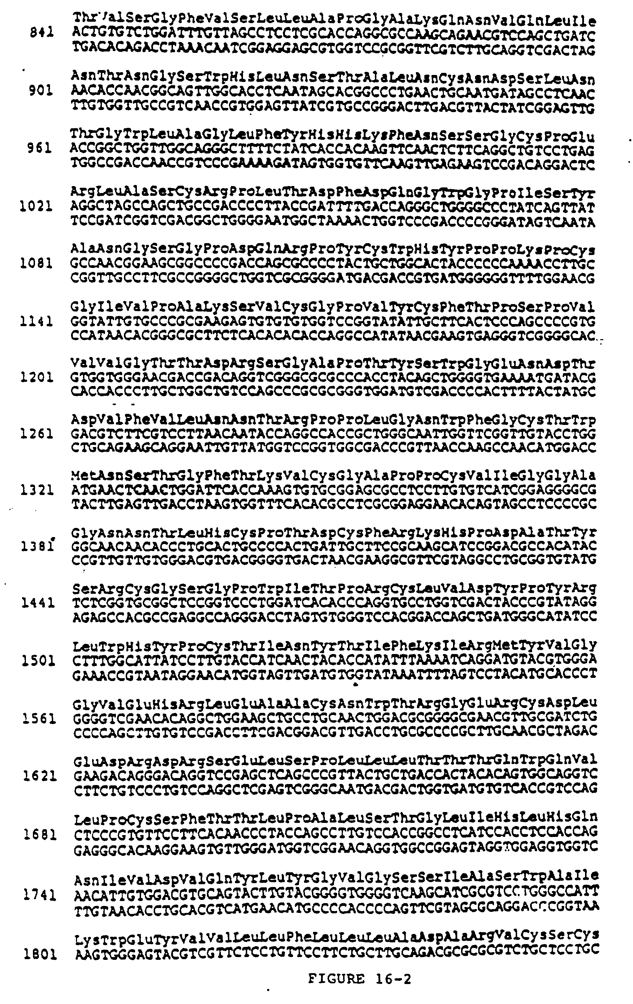

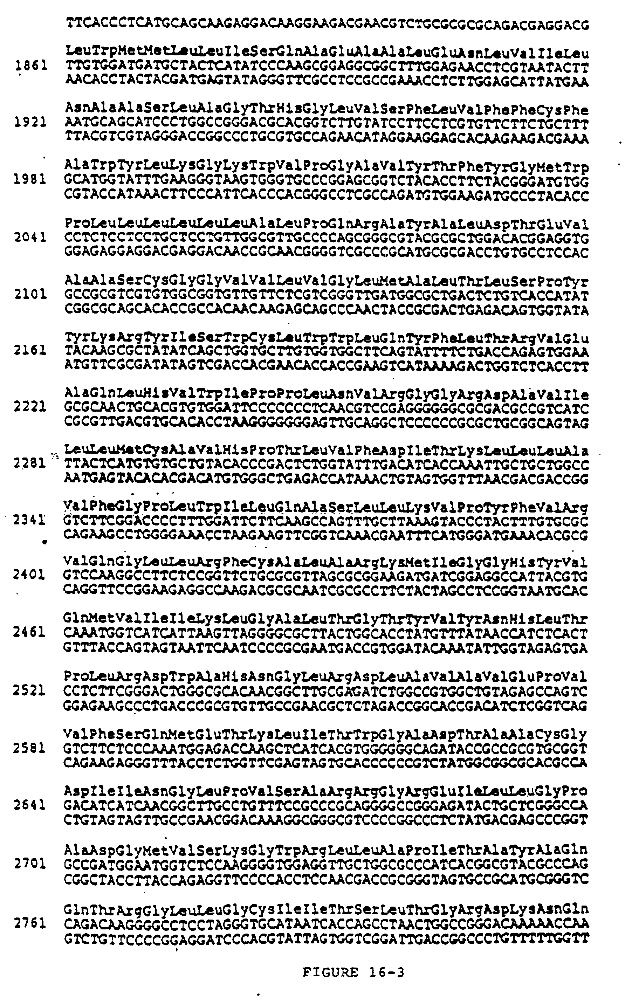

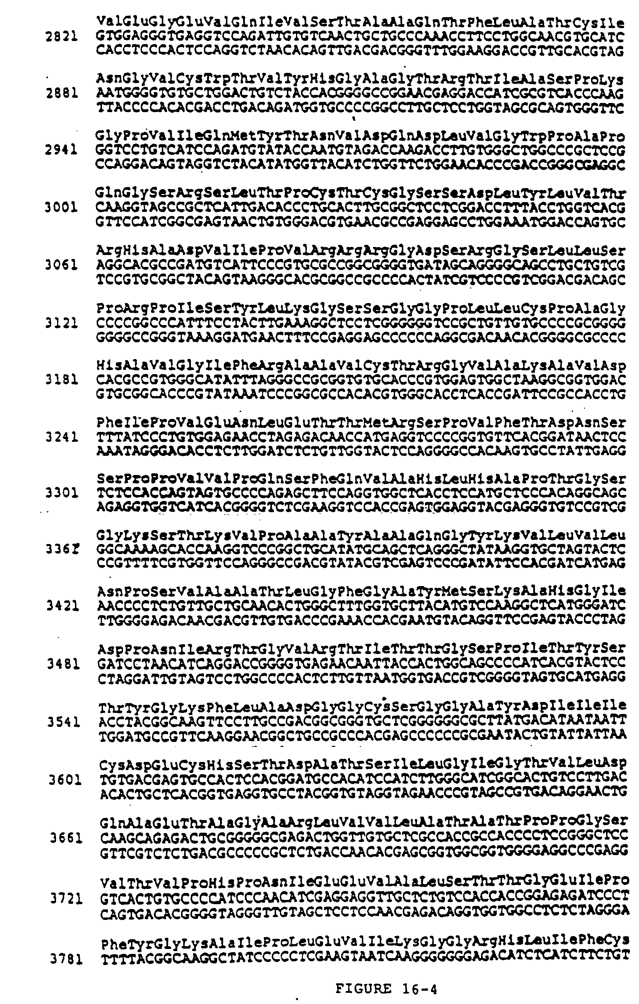

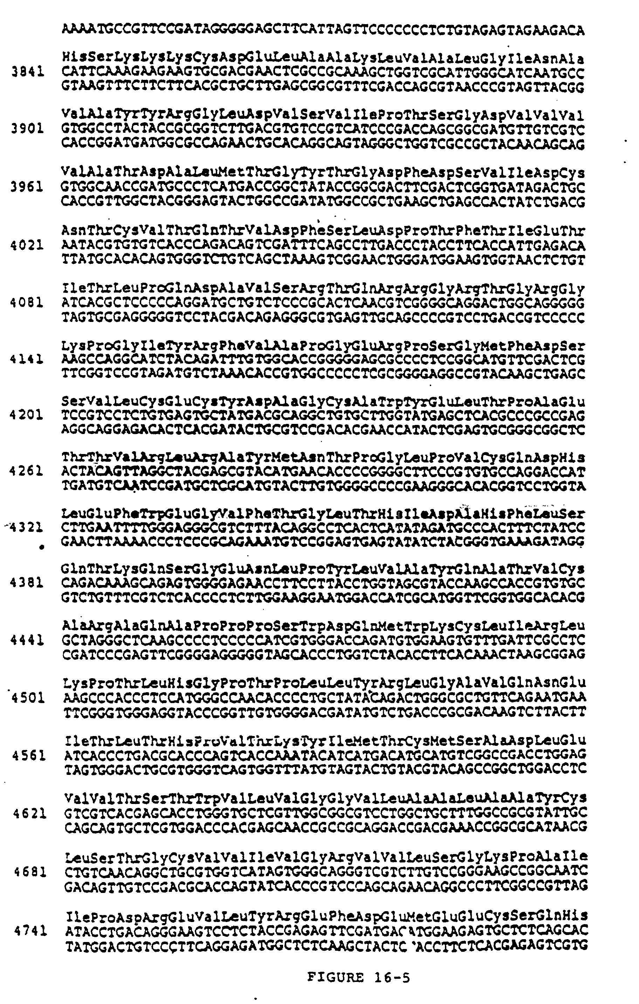

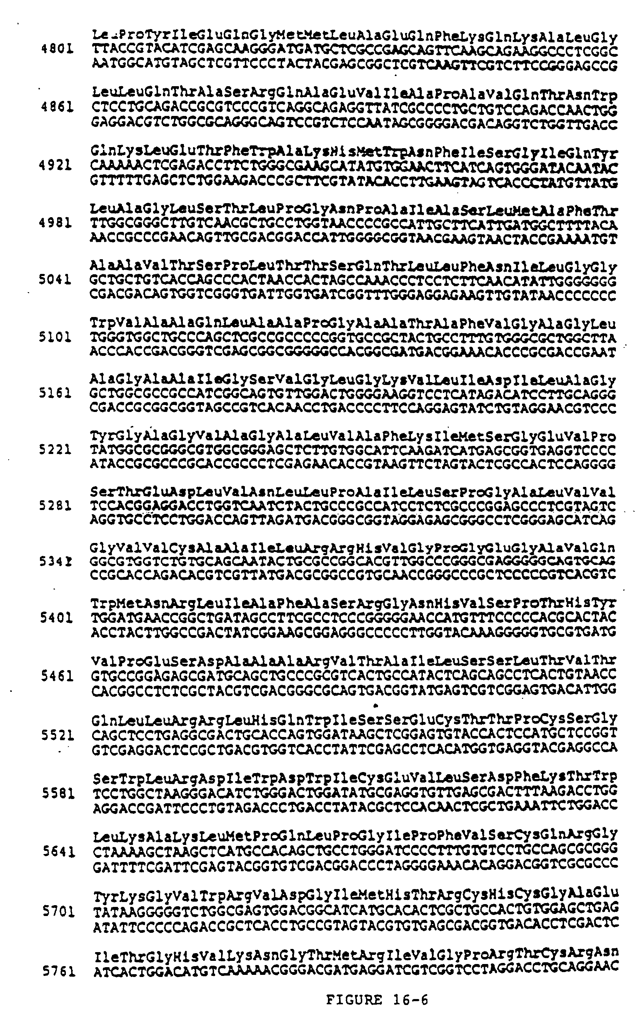

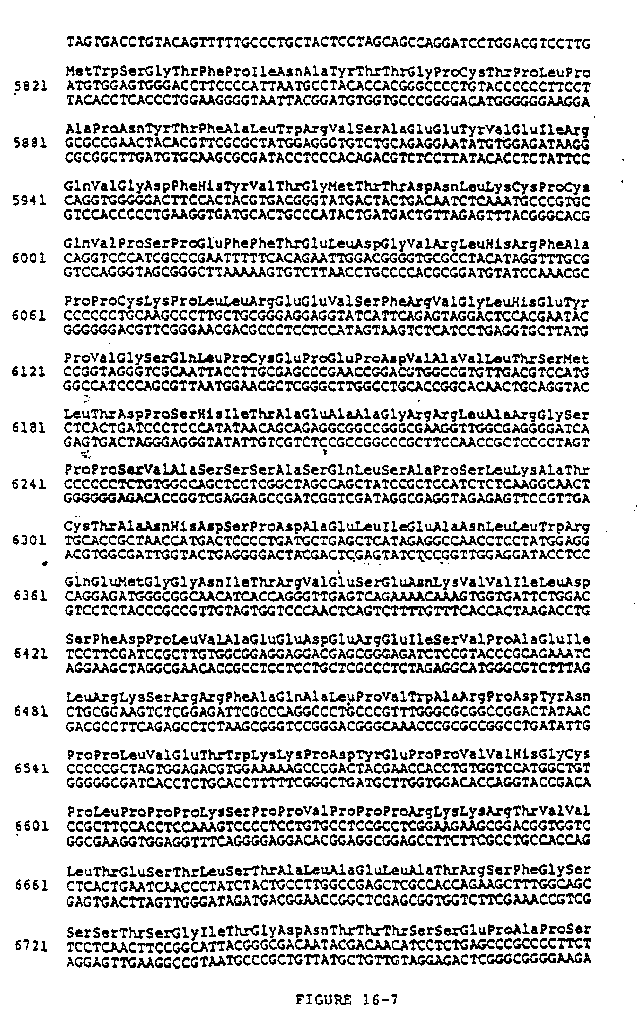

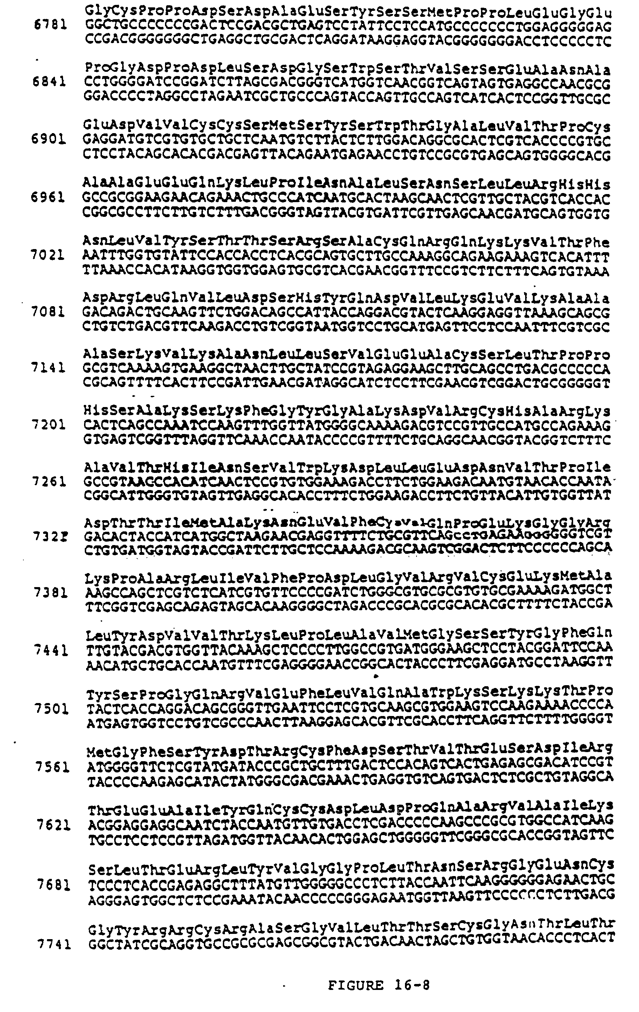

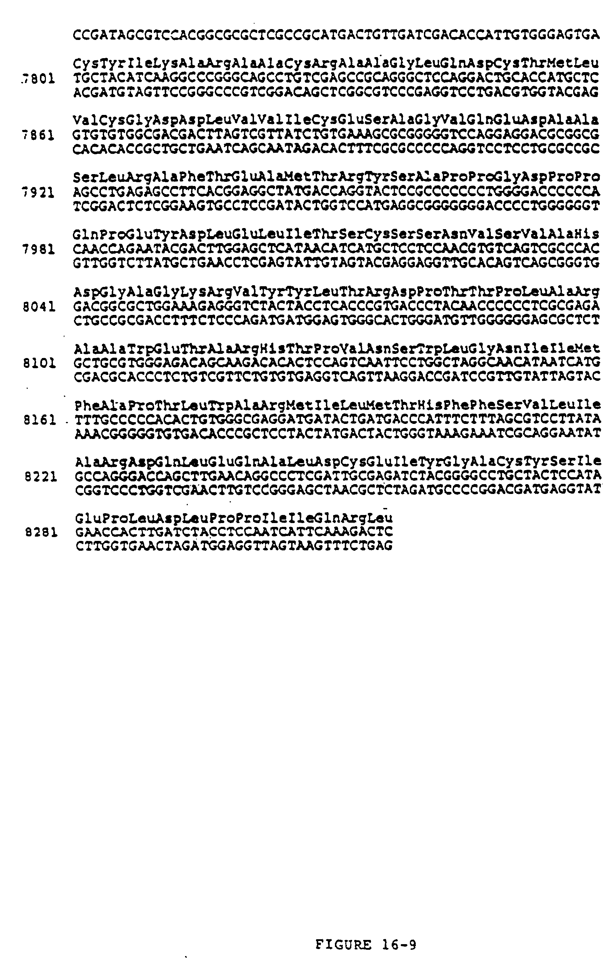

An HCV cDNA sequence has been compiled from a series of overlapping clones derived from the various HCV cDNA libraries described supra.. In this sequence, the compiled HCV cDNA sequence obtained from clones b114a, 18g, ag30a, CA205a, CA290a, CA216a, pi14a, CA167b, CA156e, CA84a, and CA59a is upstream of the compiled HCV cDNA sequence published in EPO Pub. No. 318,216, which is shown in Fig. 16. The compiled HCV cDNA sequence obtained from clones b5a and 16jh downstream of the compiled HCV cDNA sequence published in EPO Pub. No. 318,216.

Fig. 17 shows the compiled HCV cDNA sequence derived from the above-described clones and the compiled HCV cDNA sequence published in EPO Pub. No. 318,216. The clones from which the sequence was derived are b114a, 18g, ag30a, CA205a, CA290a, CA216a, pi14a, CA167b, CA156e, CA84a, CA59a, K9-1 (also called k9-1),26j, 13i, 12f, 14i, 11b, 7f, 7e, 8h, 33c, 40b, 37b, 35, 36, 81, 32, 33b, 25c, 14c, 8f, 33f, 33g, 39c, 35f, 19g, 26g, 15e, b5a, and 16jh. In the figure the three dashes above the sequence indicate the position of the putative initiator methionine codon.

Clone-b114a was obtained using the cloning procedure described for clone b5a, supra., except that the probe was the synthetic probe used to detect clone 18g, supra. Clone b114a overlaps with clones 18g, ag30a, and CA205a, except that clone b114a contains an extra two nucleotides upstream of the sequence in clone 18g (i.e., 5'-CA). These extra two nucleotides have been included in the HCV genomic sequence shown in Fig. 17.

It should be noted that although several of the clones described supra. have been obtained from libraries other than the original HCV cDNA lambda-gi11 C library (ATCC No. 40394), these clones contain HCV cDNA sequences which overlap HCV cDNA sequences in the original library. Thus, essentially all of the HCV sequence is derivable from the original lambda-gt11 C library (ATCC No. 40394) which was used to isolate the first HCV cDNA clone (5-1-1). The isolation of clone 5-1-1 is described in EPO Pub. No. 318,216.

### Purification of Fusion Polypeptide C100-3 (Alternate method)

The fusion polypeptide, C100-3 (also called HCV c100-3 and alternatively, c100-3), is comprised of superoxide dismutase (SOD) at the N-terminus an in-frame C100 HCV polypeptide at the C-terminus. A method for preparing the polypeptide by expression in yeast, and differential extraction of the insoluble fraction of the extracted host yeast cells, is described in EPO Pub. No. 318,216. An alternative method for the preparation of this fusion polypeptide is described below. In this method the antigen is precipitated from the crude cell lysate with acetone; the acetone precipitated antigen is then subjected to ion-exchange chromatography, and further purified by gel filtration.

The fusion polypeptide, C100-3 (HCV c100-3), is expressed in yeast strain JSC 308 (ATCC No. 20879) transformed with pAB24C100-3 (ATCC No. 67976); the transformed yeast are grown under conditions which allow expression (i.e., by growth in YEP containing 1% glucose). (See EPO Pub. No. 318,216). A cell lysate is prepared by suspending the cells in Buffer A (20 mM Tris HCl, pH 8.0, 1 mM EDTA, 1 mM PMSF. The cells are broken by grinding with glass beads in a Dynomill type homogenizer or its equivalent. The extent of cell breakage is monitored by counting cells under a microscope with phase optics. Broken cells appear dark, while viable cells are light-colored. The percentage of broken cells is determined.

When the percentage of broken cells is approximately 90% or greater, the broken cell debris is separated from the glass beads by centrifugation, and the glass beads are washed with Buffer A. After combining the washes and homogenate, the insoluble material in the lysate is obtained by centrifugation. The material in the pellet is washed to remove soluble proteins by suspension in Buffer B (50 mM glycine, pH 12.0, 1 mM DTT, 500 mM NaCl), followed by Buffer C (50 mM glycine, pH 10.0, 1 mM DTT). The insoluble material is recovered by centrifugation, and solubilized by suspension in Buffer C containing SDS. The extract solution may be heated in the presence of beta-mercaptoethanol and concentrated by ultrafiltration. The HCV c100-3 in the extract is precipitated with cold acetone. If desired, the precipitate may be stored at temperatures at about or below -15°C.

Prior to ion exchange chromatography, the acetone precipitated material is recovered by centrifugation, and may be dried under nitrogen. The precipitate is suspended in Buffer D (50 mM glycine, pH 10.0, 1 mM DTT, 7 M urea), and centrifuged to pellet insoluble material. The supernatant material is applied to an anion exchange column previously equilibrated with Buffer D. Fractions are collected and analyzed by ultraviolet absorbance or gel electrophoresis on SDS polyacrylamide gels. Those fractions containing the HCV c100-3 polypeptide are pooled.

In order to purify the HCV c100-3 polypeptide by gel filtration, the pooled fractions from the ion-exchange column are heated in the presence of beta-mercaptoethanol and SDS, and the eluate is concentrated by ultrafiltration. The concentrate is applied to a gel filtration column previously equilibrated with Buffer E (20 mM Tris HCl, pH 7.0, 1 mM DTT, 0.1% SDS). The presence of HCV c100-3 in the eluted fractions, as well as the presence of impurities, are determined by gel electrophoresis on polyacrylamide gels in the presence of SDS and visualization of the polypeptides. Those fractions containing purified HCV c100-3 are pooled. Fractions high in HCV c100-3 may be further purified by repeating the gel filtration process. If the removal of particulate material is desired, the HCV c100-3 containing material may be filtered through a 0.22 micron filter.

### Expression and Antigenicity of Polypeptides Encoded in HCV cDNA

### Polypeptides Expressed in E. coli

The polypeptides encoded in a number of HCV cDNAs which span the HCV genomic ORF were expressed in E. coli, and tested for their antigenicity using serum obtained from a variety of individuals with NANBH. The expression vectors containing the cloned HCV cDNAs were constructed from pSODcf1 (Steimer et al. (1986). In order to be certain that a correct reading frame would be achieved, three separate expression vectors, pcflAB, pcf1CD, and pcf1EF were created by ligating either of three linkers, AB, CD, and EF to a BamHI-EcoRI fragment derived by digesting to completion the vector pSODcf1 with EcoRI and BamHI, followed by treatment with alkaline phosphatase. The linkers were created from six oligomers, A, B, C, D, E, and F. Each oligomer was phosphorylated by treatment with kinase in the presence of ATP prior to annealing to its complementary oligomer. The sequences of the synthetic linkers were the following.

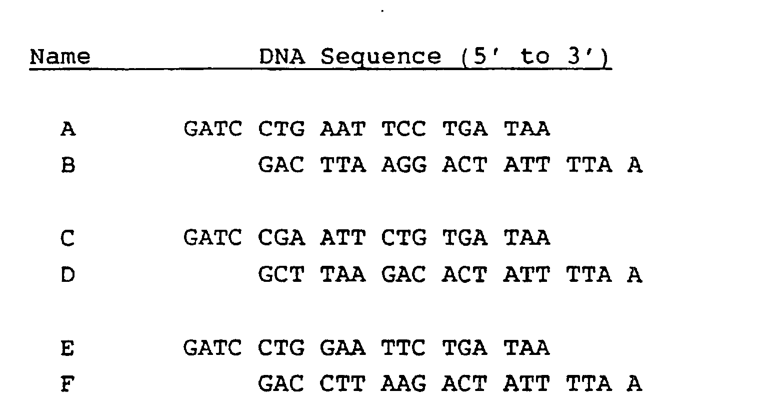

Each of the three linkers destroys the original EcoRI site, and creates a new EcoRI site within the linker, but within a different reading frame. Hence, the HCV cDNA EcoRI fragments isolated from the clones when inserted into the expression vector, were in three different reading frames.

The HCV cDNA fragments in the designated lambda-gt11 clones were excised by digestion with EcoRI; each fragment was inserted into pcflAB, pcf1CD, and pcf1EF. These expression constructs were then transformed into D1210 E. coli cells, the transformants were cloned, and recombinant bacteria from each clone were induced to express the fusion polypeptides by growing the bacteria in the presence of IPTG.

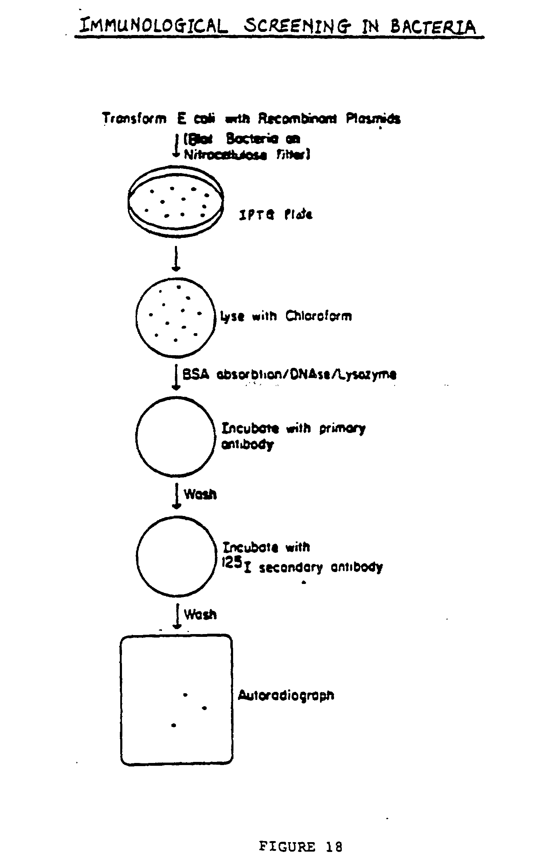

Expression products of the indicated HCV cDNAs were tested for antigenicity by direct immunological screening of the colonies, using a modification of the method described in Helfman et al. (1983). Briefly, as shown in Fig. 18, the bacteria were plated onto nitrocellulose filters overlaid on ampicillin plates to give approximately 1,000 colonies per filter. Colonies were replica plated onto nitrocellulose filters, and the replicas were regrown overnight in the presence of 2 mM IPTG and ampicillin. The bacterial colonies were lysed by suspending the nitrocellulose filters for about 15 to 20 min in an atmosphere saturated with CHCl₃ vapor. Each filter then was placed in an individual 100 mm Petri dish containing 10 ml of 50 mM Tris HCl, pH 7.5, 150 mM NaCl, 5 mM MgCl₂, 3% (w/v) BSA, 40 micrograms/ml lysozyme, and 0.1 microgram/ml DNase. The plates were agitated gently for at least 8 hours at room temperature. The filters were rinsed in TBST (50 mM Tris HCl, pH8.0, 150 mM NaCl, 0.005% Tween 20). After incubation, the cell residues were rinsed and incubated in TBS (TBST without Tween) containing 10% sheep serum; incubation was for 1 hour. The filters were then incubated with pretreated sera in TBS from individuals with NANBH, which included: 3 chimpanzees; 8 patients with chronic NANBH whose sera were positive with respect to antibodies to HCV C100-3 polypeptide (described in EPO Pub. No. 318,216, and supra.) (also called C100); 8 patients with chronic NANBH whose sera were negative for anti-C100 antibodies; a convalescent patient whose serum was negative for anti-C100 antibodies; and 6 patients with community acquired NANBH, including one whose sera was strongly positive with respect to anti-C100 antibodies, and one whose sera was marginally positive with respect to anti-C100 antibodies. The sera, diluted in TBS, was pretreated by preabsorption with hSOD. Incubation of the filters with the sera was for at least two hours. After incubation, the filters were washed two times for 30 min with TBST. Labeling of expressed proteins to which antibodies in the sera bound was accomplished by incubation for 2 hours with ¹²⁵I- labeled sheep anti-human antibody. After washing, the filters were washed twice for 30 min with TBST, dried, and autoradiographed.

A number of clones (see infra.) expressed polypeptides containing HCV epitopes which were immunologically reactive with serum from individuals with NANBH. Five of these polypeptides were very immunogenic in that antibodies to HCV epitopes in these polypeptides were detected in many different patient sera. The clones encoding these polypeptides, and the location of the polypeptide in the putative HCV polyprotein (wherein the amino acid numbers begin with the putative initiator codon) are the following: clone 5-1-1, amino acids 1694-1735; clone C100, amino acids 1569-1931; clone 33c, amino acids 1192-1457; clone CA279a, amino acids 1-84; and clone CA290a amino acids 9-177. The location of the immunogenic polypeptides within the putative HCV polyprotein are shown immediately below.

### Clones encoding polypeptides of proven reactivity with sera from NANBH patients.

| Clone | Location within the HCV polyprotein (amino acid no. beginning with putative initiator methionine) |
|---|---|
| CA279a | 1-84 |
| | |
| CA290a | 9-177 |

The results on the immunogenicity of the polypeptides encoded in the various clones examined suggest efficient detection and immunization systems may include panels of HCV polypeptides/epitopes.

### Expression of HCV Epitopes in Yeast

Three different yeast expression vectors which allow the insertion of HCV cDNA into three different reading frames are constructed. The construction of one of the vectors, pAB24C100-3 is described in EPO Pub. No. 318,216. In the studies below, the HCV cDNA from the clones listed in supra. in the antigenicity mapping study using the E. coli expressed products are substituted for the C100 HCV cDNA. The construction of the other vectors replaces the adaptor described in the above E. coli studies with one of the following adaptors: 
Adaptor 1

ATT TTG AAT TCC TAA TGA G
  AC TTA AGG ATT ACT CAG CT
 
Adaptor 2

AAT TTG GAA TTC TAA TGA G
    AC CTT AAG ATT ACT CAG CT.
 The inserted HCV cDNA is expressed in yeast transformed with the vectors, using the expression conditions described supra. for the expression of the fusion polypeptide, C100-3. The resulting polypeptides are screened using the sera from individuals with NANBH, described supra. for the screening of immunogenic polypeptides encoded in HCV cDNAs expressed in E. coli.

### Comparison of the Hydrophobic Profiles of HCV Polyproteins with West Nile Virus Polyprotein and with Dengue Virus NS1

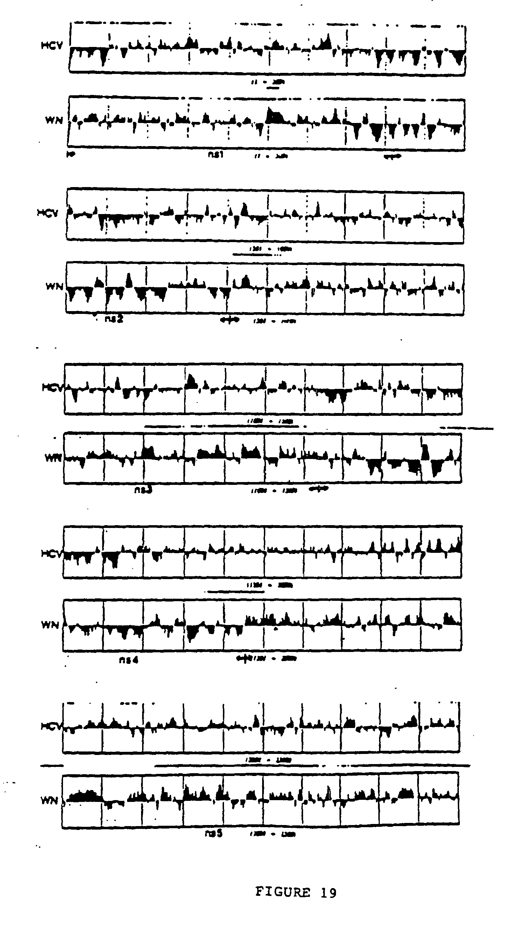

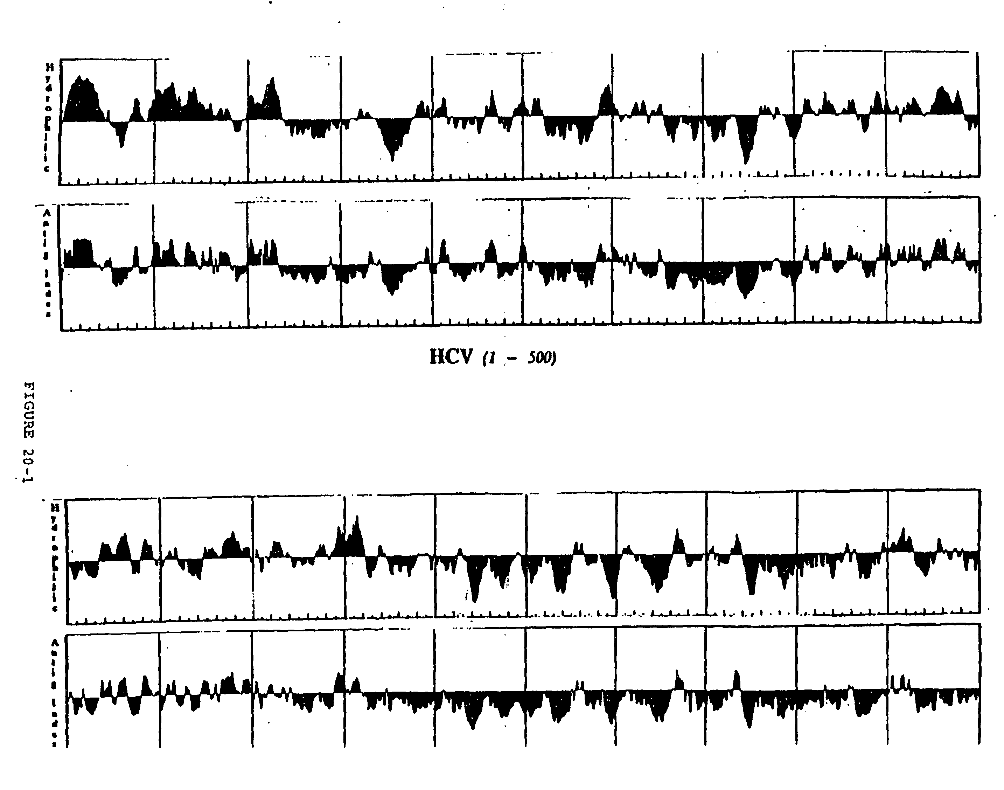

The hydrophobicity profile of an HCV polyprotein segment was compared with that of a typical Flavivirus, West Nile virus. The polypeptide sequence of the West Nile virus polyprotein was deduced from the known polynucleotide sequences encoding the non-structural proteins of that virus. The HCV polyprotein sequence was deduced from the sequence of overlapping cDNA clones. The profiles were determined using an antigen program which uses a window of 7 amino acid width (the amino acid in question, and 3 residues on each side) to report the average hydrophobicity about a given amino acid residue. The parameters giving the reactive hydrophobicity for each amino acid residue are from Kyte and Doolittle (1982). Fig. 19 shows the hydrophobic profiles of the two polyproteins; the areas corresponding to the non-structural proteins of West Nile virus, ns1 through ns5, are indicated in the figure. As seen in the figure, there is a general similarity in the profiles of the HCV polyprotein and the West Nile virus polyprotein.

The sequence of the amino acids encoded in the 5'-region of HCV cDNA shown in Fig. 16 has been compared with the corresponding region of one of the strains of Dengue virus, described supra., with respect to the profile of regions of hydrophobicity and hydrophilicity (data not shown). This comparison indicated that the polypeptides from HCV and Dengue encoded in this region, which corresponds to the region encoding NS1 (or a portion thereof), have a similar hydrophobic/hydrophilic profile.

The similarity in hydrophobicity profiles, in combination with the previously identified homologies in the amino acid sequences of HCV and Dengue Flavivirus in EP 0,218,316 suggests that HCV is related to these members of the Flavivirus family.

### Characterization of the Putative Polypeptides Encoded Within the HCV ORF

The sequence of the HCV cDNA sense strand, shown in Fig. 17, was deduced from the overlapping HCV cDNAs in the various clones described in EPO Pub. No. 318,216 and those described supra. It may be deduced from the sequence that the HCV genome contains primarily one long continuous ORF, which encodes a polyprotein. In the sequence, nucleotide number 1 corresponds to the first nucleotide of the initiator MET codon; minus numbers indicate that the nucleotides are that distance away in the 5'-direction (upstream), while positive numbers indicate that the nucleotides are that distance away in the 3'-direction (downstream). The composite sequence shows the "sense" strand of the HCV cDNA.

The amino acid sequence of the putative HCV polyprotein deduced from the HCV cDNA sense strand sequence is also shown in Fig. 17, where position 1 begins with the putative initiator methionine.

Possible protein domains of the encoded HCV polyprotein, as well as the approximate boundaries, are the following (the polypeptides identified within the parentheses are those which are encoded in the Flavivirus domain):

| Putative Domain | Approximate Boundary (amino acid nos.) |
|---|---|
| "C" (nucleocapsid protein) | 1-120 |
| "E" (Virion envelope protein(s) and possibly matrix (M) proteins | 120-400 |
| "NS1" (complement fixation antigen?) | 400-660 |
| "NS2" (unknown function) | 660-1050 |
| "NS3" (protease?) | 1050-1640 |
| "NS4" (unknown function) | 1640-2000 |
| "NS5" (polymerase) | 2000-? end |

It should be noted, however, that hydrophobicity profiles (described infra), indicate that HCV diverges from the Flavivirus model, particularly with respect to the region upstream of NS2. Moreover, the boundaries indicated are not intended to show firm demarcations between the putative polypeptides.

### The Hydrophilic and Antigenic Profile of the Polypeptide

Profiles of the hydrophilicity/hydrophobicity and the antigenic index of the putative polyprotein encoded in the HCV cDNA sequence shown in Fig. 16 were determined by computer analysis. The program for hydrophilicity/hydrophobicity was as described supra. The antigenic index results from a computer program which relies on the following criteria: 1)surface probability, 2) prediction of alpha-helicity by two different methods; 3) prediction of beta-sheet regions by two different methods; 4) prediction of U-turns by two different methods; 5) hydrophilicity/hydrophobicity; and flexibility. The traces of the profiles generated by the computer analyses are shown in Fig. 20. In the hydrophilicity profile, deflection above the abscissa indicates hydrophilicity, and below the abscissa indicates hydrophobicity. The probability that a polypeptide region is antigenic is usually considered to increase when there is a deflection upward from the abscissa in the hydrophilic and/or antigenic profile. It should be noted, however, that these profiles are not necessarily indicators of the strength of the immunogenicity of a polypeptide.

### Identification of Co-linear Peptides in HCV and Flaviviruses

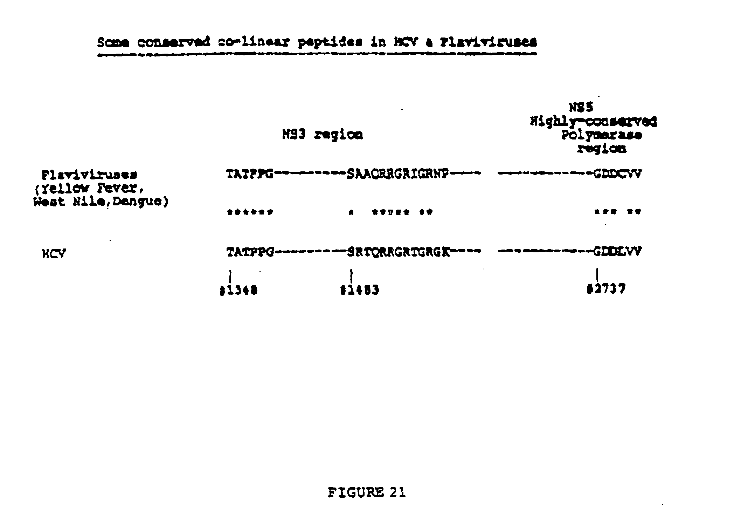

The amino acid sequence of the putative polyprotein encoded in the HCV cDNA sense strand was compared with the known amino acid sequences of several members of Flaviviruses. The comparison shows that homology is slight, but due to the regions in which it is found, it is probably significant. The conserved co-linear regions are shown in Fig. 21. The amino acid numbers listed below the sequences represent the number in the putative HCV polyprotein (See Fig. 17.)

The spacing of these conserved motifs is similar between the Flaviviruses and HCV, and implies that there is some similarity between HCV and these flaviviral agents.

The following listed materials are on deposit under the terms of the Budapest Treaty with the American Type Culture Collection (ATCC), 12301 Parklawn Dr., Rockville, Maryland 20852, and have been assigned the following Accession Numbers.

| lambda-gt11 | ATCC No. | Deposit Date |
|---|---|---|
| HCV cDNA library | 40394 | 1 Dec. 1987 |
| clone 81 | 40388 | 17 Nov. 1987 |
| clone 91 | 40389 | 17 Nov. 1987 |
| clone 1-2 | 40390 | 17 Nov. 1987 |
| clone 5-1-1 | 40391 | 18 Nov. 1987 |
| clone 12f | 40514 | 10 Nov. 1988 |
| clone 35f | 40511 | 10 Nov. 1988 |
| clone 15e | 40513 | 10 Nov. 1988 |
| clone K9-1 | 40512 | 10 Nov. 1988 |
| JSC 308 | 20879 | 5 May 1988 |
| pS356 | 67683 | 29 April 1988 |

In addition, the following deposits were made on 11 May 1989.

| Strain | Linkers | ATCC No. |
|---|---|---|
| D1210 (Cf1/5-1-1) | EF | 67967 |
| D1210 (Cf1/81) | EF | 67968 |
| D1210 (Cf1/CA74a) | EF | 67969 |
| D1210 (Cf1/35f) | AB | 67970 |
| D1210 (Cf1/279a) | EF | 67971 |
| D1210 (Cf1/C36) | CD | 67972 |
| D1210 (Cf1/13i) | AB | 67973 |
| D1210 (Cf1/C33b) | EF | 67974 |
| D1210 (Cf1/CA290a) | AB | 67975 |
| HB101 (AB24/C100 #3R) | | 67976 |

The following derivatives of strain D1210 were deposited on 3 May 1989.

| Strain Derivative | ATCC No. |
|---|---|
| pCF1CS/C8f | 67956 |
| pCF1AB/C12f | 67952 |
| pCF1EF/14c | 67949 |
| pCF1EF/15e | 67954 |
| pCF1AB/C25c | 67958 |
| pCF1EF/C33c | 67953 |
| pCF1EF/C33f | 67050 |
| pCF1CD/33g | 67951 |
| pCF1CD/C39c | 67955 |
| pCF1EF/C40b | 67957 |
| pCF1EF/CA167b | 67959 |

The following strains were deposited on May 12, 1989.

| Strain | ATCC No. |
|---|---|
| Lambda gt11 (C35) | 40603 |
| Lambda gt10 (beta-5a) | 40602 |
| D1210 (C40b) | 67980 |
| D1210 (M16) | 67981 |

The deposited materials mentioned herein are intended for convenience only, and are not required to practice the present invention in view of the descriptions herein, and in addition these materials are incorporated herein by reference.

### Industrial Applicability

The invention, in the various manifestations disclosed herein, has many industrial uses, some of which are the following. The HCV cDNAs may be used for the design of probes for the detection of HCV nucleic acids in samples. The probes derived from the cDNAs may be used to detect HCV nucleic acids in, for example, chemical synthetic reactions. They may also be used in screening programs for anti-viral agents, to determine the effect of the agents in inhibiting viral replication in cell culture systems, and animal model systems. The HCV polynucleotide probes are also useful in detecting viral nucleic acids in humans, and thus, may serve as a basis for diagnosis of HCV infections in humans.

In addition to the above, the cDNAs provided herein provide information and a means for synthesizing polypeptides containing epitopes of HCV. These polypeptides are useful in detecting antibodies to HCV antigens. A series of immunoassays for HCV infection, based on recombinant polypeptides containing HCV epitopes are described herein, and will find commercial use in diagnosing HCV induced NANBH, in screening blood bank donors for HCV-caused infectious hepatitis, and also for detecting contaminated blood from infectious blood donors. The viral antigens will also have utility in monitoring the efficacy of anti-viral agents in animal model systems. In addition, the polypeptides derived from the HCV cDNAs disclosed herein will have utility as vaccines for treatment of HCV infections.

The polypeptides derived from the HCV cDNAs, besides the above stated uses, are also useful for raising anti-HCV antibodies. Thus, they may be used in anti-HCV vaccines. However, the antibodies produced as a result of immunization with the HCV polypeptides are also useful in detecting the presence of viral antigens in samples. Thus, they may be used to assay the production of HCV polypeptides in chemical systems. The anti-HCV antibodies may also be used to monitor the efficacy of anti-viral agents in screening programs where these agents are tested in tissue culture systems. They may also be used for passive immunotherapy, and to diagnose HCV caused NANBH by allowing the detection of viral antigen(s) in both blood donors and recipients. Another important use for anti-HCV antibodies is in affinity chromatography for the purification of virus and viral polypeptides. The purified virus and viral polypeptide preparations may be used in vaccines. However, the purified virus may also be useful for the development of cell culture systems in which HCV replicates.

Antisense polynucleotides may be used as inhibitors of viral replication.

For convenience, the anti-HCV antibodies and HCV polypeptides, whether natural or recombinant, may be packaged into kits.

## Claims (Claims for the following Contracting State(s): AT, BE, CH, DE, DK, FR, GB, IT, LI, LU, NL, SE)

1. A polypeptide in substantially isolated form comprising a contiguous sequence from a Hepatitis C Virus (HCV) polyprotein of at least 10 amino acids, wherein said contiguous sequence is within amino acids 1 to 449 of an HCV polyprotein, wherein HCV is **characterized by**:
a positive stranded RNA genome;
said genome comprising an open reading frame (ORF) encoding a polyprotein; and
the entirety of the said encoded polyprotein having at least 40% homology to the entire polyprotein selected from (i) the polyprotein as shown inFigure 17, or (ii) the polyprotein of a viral isolate from the genome of which was prepared cDNAs deposited in a lambda gt-11 cDNA library with the American Type Culture Collection (ATCC) under Accession No. 40394.

2. A polypeptide according to claim 1 wherein said polyprotein has at least 70% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

3. A polypeptide according to claim 1 wherein said polypeptide has at least 80% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

4. A polypeptide according to claim 1 wherein said polypeptide has at least 90% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

5. A polypeptide according to any one of claims 1 to 4 wherein said contiguous sequence is selected from within amino acids 1-84 or from within amino acids 9-177 of an HCV polyprotein.

6. A polypeptide according to any one of claims 1 to 5 wherein said contiguous sequence is within the sequences selected from the group consisting Figure 4, 11, 12 and 17.

7. A polypeptide according to any one of the preceding claims wherein said contiguous sequence comprises at least 15 amino acids.

8. A polypeptide according to any one of claims 1 to 7 wherein said contiguous sequence has the formula AAx-AAy wherein x and y designate amino acid numbers shown in Figure 17:
AA1-AA25, AA1-AA50; AA1-AA84; AA9-AA177; AA1-AA10; AA5-AA20; AA35-AA45; AA50-AA100; AA40-AA90; AA45-AA65; AA65-AA75; AA80-AA90; AA99-AA120; AA95-AA110; AA105-AA120; AA100-AA150; AA150-AA200; AA155-AA170; AA190-AA210; AA200-AA250; AA220-AA240; AA245-A265; AA250-AA300; AA290-AA330; AA290-305; AA300-AA350; AA310-AA330; AA350-AA400; AA380-AA395; AA405-AA495; AA400-AA450; AA405-AA415; AA415-AA425; AA425-AA435; AA440-AA460; AA437-AA582.

9. A polypeptide according to any one of the preceding claims wherein said polypeptide reacts immunologically with anti-HCV antibodies.

10. A polypeptide as defined in any one of the preceding claims attached to a solid substrate.

11. An immunoassay kit which comprises a polypeptide according to any one of the preceding claims in a suitable container.

12. An immunoassay method for detecting antibodies directed against a hepatitis C virus (HCV) polypeptide comprising:
(a) providing a first polypeptide that is immunologically reactive with anti-HCV antibodies as determined by competition in binding with a second polypeptide comprising an HVC antigenic sequence of at least 10 contiguous amino acids from within amino acid number 1-449 of an HCV polyprotein.
(b) incubating said first polypeptide with a biological sample under conditions that allow for the formatfon of an antibody-polypeptide complex; and
(c) detecting any antibody/polypeptide complexes comprising said polypeptide, wherein HCV is **characterized by**:
a positive stranded RNA genome;
said genome comprissing an open reading frame (ORF) encoding a polyprotein; and
the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

13. An immunoassay according to claim 12 wherein said polyprotein has at least 70% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

14. An immunoassay according to claim 12 wherein said polyprotein has at least 80% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

15. An immunoassay according to claim 12 wherein said polyprotein has at least 90% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

16. An immunoassay according to any one of claims 12 to 15 wherein said amino acids are selected from within amino acids 1-449 of the HCV polyprotein of Figure 17.

17. An immunoassay according to any one of claims 12 to 16 wherein said first polypeptide comprises an HCV antigenic sequence from within amino acid numbers 1-449 of the HCV polyprotein of Figure 17.

18. An immunoassay according to any one of claims 12 to 17 wherein the biological sample is human blood, serum or plasma.

19. A polynucleotide in substantially isolated form which comprises an HCV genomic sequence or the complement thereof which is selectively hybridizable to a polynucleotide found within nucleotides -319 to 1348 of an HCV genome or the complement thereof, wherein HCV is **characterized by**:
a positive stranded RNA genome;
said genome comprising an open reading frame (ORF) encoding a polyprotein; and
the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

20. Apolynucleotide according to claim 19 wherein said polyprotein has at least 70% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

21. A polynucleotide according to claim 19 wherein said polyprotein has at least 80% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

22. A polynucleotide according to claim 19 wherein said polyprotein has at least 90% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

23. A polynucleotide according to any one of claims 19 to 22 wherein said HCV genomic sequence or the complement thereof is found within nucleotides -319 to 1348 or within nucleotides -319 to -1 of an HCV genome.

24. A polynucleotide according to any one of claims 19 to 23 wherein said HCV genomic sequence or the complement thereof is selected from the group of sequences found whithin Figures 11,12 and 17.

25. A polynucleotide according to any one of claims 19 to 24 which is DNA.

26. A polynucleotide according to any one of claim 19 to 25 which is a probe or a primer for DNA synthesis.

27. A polynucleotide according to claim 26 wherein the probe is labelled.

28. A kit for analysing biological samples for the presence of HCV polynucleotides which comprises a polynucleotide according to any one of claims 19 to 27 in a suitable container.

29. A method of detecting HCV nucleic acids in a simple which comprises:
a. providing a polynucleotide as defined in any one of claims 19 to 27;
b. reacting said polynucleotide with said sample under conditions which allow for the selective formation of polynucleotide duplexes between said polynucleotide and any HCV nucleic acid or ECV cDNA in said sample; and
c. detecting any polynucleotide duplexes containing said polynucléotide.

30. A method for amplifying a polynucleotide of HCV in a sample which comprises:
a. amplifying the polynucleotide of HCV using a DNA polymerase and at least one polynucleotide defined in any one of claims 19 to 27; and
b. detecting the amplified polynucleotide.

31. A method according to either of claims 29 or 30 wherein said sample is human blood, serum, or plasma.

32. A recombinant vector comprising a polynucleotide sequence which encodes a polypeptide as defined in any one of claims 1 to 9 wherein said sequence which encodes said polypeptide is operably linked to a control sequence capable of expressing the polypeptide.

33. A recombinant vector according to claim 32 wherein said polynucleotide is DNA

34. A host cell transformed with the vector according to either of claims 32 or 33.

35. A method of making an HCV polypeptide which comprises incubating a host cell according to claim 34 under conditions which permit expression of said sequence which encodes a polypeptide, and recovering the polypeptide.

36. A method of making an HCV polypeptide comprising chemically synthesizing a polypeptide accoroing to any of claims 1 to 9.

37. A pharmaceutical composition comprising:
a. a polynucleotide as defined in any one of claims 19 to 25; or
b. a polypeptide as defined in any one of claims 1 to 9; and
c. a pharmaceutically acceptable carrier.

38. A method for preparing a host cell transformed with a recombinant vector according to claim 32 or 33 comprising:
a. providing a host cell capable of transformation;
b. providing the recombinant vector, and
c. incubating (a) with (b) under conditions which allow transformation - of the host cell with the vector.

39. A method for preparing a recombinant vector according to claim 32 or 33 comprising:
a. providing a host cell tnansformed with a recombinant polynucleotide according to any one of claims 19 to 27; and
b. isolating said polynucleotide from said host cell.

40. A method for producing a polynucleotide probe comprising chemically synthesizing a polynucleotide according to any of claims 19 to 27.

41. A method of preparing a supply of human biological samples prior to use said method comprising:
(a) providing a supply of human biological samples; and
(b) removing from said supply of human biological samples those samples containing:
(i) polynucleotides capable of detecfion in a method according to any of claims 29 or 30;
or (ii) antibodies capable of producing detectable polypeptide-antibody complexes in an immunoassay according to any one of claims 12 to 18.

42. A method according to claim 41 wherein said human biological samples are blood, plasma or sera.

43. Use of a biological sample prepared according to the method of claim 41 or 42 for the preparauon of blood-related products.

## Claims (Claims for the following Contracting State(s): GR, SP)

1. A method of preparing polypeptide in substantially isolated form said polypeptide comprising a contignous sequence from a Hepatitis C Virus (HCV) polyprotein of at least 10 amino acids, wherein said contiguous sequence is within amino acids 1 to 449 of an HCV polyprotein, wherein HCV is **characterized by**:
a positive stranded RNA genome;
said genome comprising an open reading frame (ORF) encoding a polyprotein; and
the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394, **characterised in that** the method is selected from the group of methods comprising chemical synthesis, expression of a recombinant expression system and isolation from mutant HCV.

2. A method according to claim 1 wherein said polyprotein has at least 70% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

3. A method according to claim 1 wherein said polyprotein has at least 80% homology to the entire polyprotein selected from (i) the polyprotein as shown in Figure 17 or (ii) the polyprotein of a viral isolate from the genome of which was prepared cDNAs deposited in a lambda gt-11 cDNA library with the American Type Culture Collection (ATCC) under Accession No. 40394.

4. A method according to claim 1 wherein said polypeptide has at least 90% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 403 94.

5. A method according to any one of claims 1 to 4 wherein said contiguous sequence is selected from within amino acids 1-84 or from within amino acids 9-177 of an HCV polyprotein.

6. A method according to any one of claims 1 to 5 wherein said contiguous sequence is within the sequences selected from the group consisting Figure 4, 11, 12 and 17.

7. A method according to any one of the preceding claims wherein said contiguous sequence comprises at least 15 amino acids.

8. A method according to any one of claims 1 to 7 wherein said contiguous sequence has the formula AAx-AAy wherein x and y designate amino acid numbers shown in Figure 17:
AA1-AA25, AA1-AA50; AA1-AA84; AA9-AA177; AA1-AA10; AA5-AA20; AA35-AA45;AA50-AA100; AA40-AA90; AA45-AA65; AA65-AA75; AA80-AA90; AA99-AA120; AA95-AA110; AA105-AA120, AA100-AA150; AA150-AA200; AA155-AA170; AA190-AA210; AA200-AA250; AA220-AA240; AA245-A265; AA250-AA300; AA290-AA330; AA290-305;AA300-AA350; AA310-AA330; AA350-AA400; AA380-AA395; AA405-AA495; AA400-AA450, AA405-AA415; AA415-AA425; AA425-AA435; AA440-AA460; AA437-AA582.

9. A method according to any one of the preceding claims wherein said polypeptide reacts immunolooicalfy with anti-BCV antibodies.

10. A method as defined in any one of the preceding claims further comprising attaching the polypeptide to a solid substrate.

11. An immunoassay method for detecting antibodies directed against a hepatitis C virus (HCV) polypeptide comprising:
(a) providing a first polypeptide that is immunologically reactive with anti-HCV antibodies as determined by competition in binding with a second polypeptide comprising an HCV antigenic sequence of at least 10 contiguous amino acids from within amino acid number 1-449 of an HCV polyprotein.
(b) incubating said first polypeptide with z biological sample under conditions that allow for the formation of an antibody-polypeptide complex; and
(c) detecting any antibody/polypeptide complexes comprising said polypeptide, wherein HCV is **characterized by**:
a positive stranded RNA genome;
said genome comprising an open reading frame (ORF) encoding a polyprotein; and
the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library witn the American Type Culture Collection (ATCC) under Accession No. 40394.

12. An immunoassay method according to claim 11 wherein said polyprotein has at least 70% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

13. An immunoassay method according to claim 11 wherein said polyprotein has at least 80% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

14. An immunoassay method according to claim 11 wherein said polyprotein has at least 90% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

15. An immunoassay method according to any one of claims 11 to 14 wherein said amino acids are selected from within amino acids 1-449 of the HCV polyprotein of Figure 17.

16. An immunoassay method according to any one of claims 11 to 15 wherein said first polypeptide comprises an HCV antigenic sequence from within amino acid numbers 1-449 of the HCV polyprotein of 17.

17. An immunoassay, method according to any one of claims 11 to 16 wherein the biological sample is human blood, serum or plasma.

18. A method of preparing a polynucleotide in substantially isolated form said polynucleotide comprising an HCV genomic sequence or the complement thereof which is selectively hybridizable to a polynucleotide found within nucleotides -319 to 1348 of an HCV genome or the complement thereof, wherein HCV is **characterized by**:
a positive stranded RNA genome;
said genome comprising an open reading frame (ORF) encoding a polyprotein and
the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394, **characterised in that** the method is selected from the group of methods comprising chemical synthesis, DNA replication, reverse transcription and transcription.

19. A method according to claim 18 wherein said polyprotein has at least 70% homology to the entire polyprotein selected from (i) the polyprotein as shown in Figure 17, or (ii) the polyprotein of a viral isolate the genome of which was prepared cDNAs deposited in a lambda gt-11 cDNA library with the American Type Culture Collection (ATCC) under Accession No. 40394.

20. A method according to claim 18 wherein said polyprotein has at least 80% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

21. A method according to claim 18 wherein said polyprotein has at least 90% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

22. A method according to any one of claims 18 to 21 wherein said HCV genomic sequence or the complement thereof is found within nucleotides -319 to 1348 or within nucleotides -319 to -1 of an HCV genome.

23. A method according to any one of claims 18 to 22 wherein said HCV genomic sequence or the complement thereof is selected from the group of sequences found within Figures 11, 12 and 17.

24. A method according to any one of claims 18 to 23 in which the polynucleotide is DNA.

25. A method according to any one of claim 18 to 24 in which the polynucleotide is a probe or a primer for DNA synthesis.

26. A method according to claim 25 wherein the probe is labelled.

27. A method of detecting HCV nucleic acids in a sample which comprises:
a. providing a polynucleotide as defined in any one of claims 18 to 26;
b. reacting said polynucleotide with said sample under conditions which allow for the selective formation of polynucleotide duplexes between said polynucleotide and any HCV nucleic acid or HCV cDNA in said sample; and
c. detecting any polynucleotide duplexes containing said polynucleotide.

28. A method for amplifying, a polynucleotide of HCV in a sample which comprises:
a. amplifying the polynucleotide of HCV using 2 DNA polymerase and at least one polynucleotide defined in any one of claims 18 to 25; and
b. detecting the amplified polynucleotide.

29. A method according to either of claims 27 or 28 wherein said sample is human blood, serum, or plasma.

30. A method of preparing a recombinant vector comprising a polynucleotide sequence which encodes a polypeptide as defined in any one of claims 1 to 9 wherein said sequence which encodes said polypeptide is operably linked to a control sequence capable of expressing the polypeptide **characterised in that** said method comprises obtaining fragments encoding the desired polypeptide from cDNA clones by restriction digestion or chemically synthesising said polynucleotide sequence and ligating said polynucleotide sequence into a vector.

31. A method according to claim 30 wherein said polynucleotide is DNA.

32. A method for preparing a host cell transformed with a recombinant vector as prepared in claim 30 or 31 comprising:
a providing a host cell capable of transformation;
b. providing the recombinant vector, and
c. incubating (a) with (b) under conditions which allow transformation of the host cell with the vector.

33. A method of making an HCV polypeptide which comprises incubating a host cell prepared according to claim 32 under conditions which permit expression of said sequence which encodes a polypeptide, and recovering the polypeptide.

34. A method for producing a polynucleotide probe comprising chemically synthesizing a polynucleotide as defined in any of claims 18 to 26..

35. A method of preparing a supply of human biological samples prior to use said method comprising:
(a) providing a supply of human biological samples; and
(b) removing from said supply of human biological samples those samples containing:
(i) polynucleotides capable of detection in a method according to any of claims 27 or 28;
or (ii) antibodies capable of producing detectable polypeptide-antibody complexes in an immunoassay method according to any one of claims 11 to 17.

36. A method according to claim 35 wherein said human biological samples are blood, plasma or sera.

37. A polypeptide in substantially isolated form comprising a contiguous sequence from a Hepatitis C Virus (HCV) polyprotein of at least 10 amino acids, wherein said contiguous sequence is within amino acids 1 to 449 of an HCV polyprotein, wherein HCV is **characterized by**:
a positive stranded RNA genome;
said genome comprising an open reading frame (ORF) encoding a polyprotein; and
the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

38. A polypeptide according to claim 37 wherein said polyprotein has at least 70% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

39. A polypeptide according to claim 37 wherein said polyprotein has at least 80% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

40. A polypeptide according to claim 37 wherein said polyprotein has at least 90% homology to the entire polyprotein selected from (i) the polyprotein as-shown in Figure 17, or (ii) the polyprotein of a viral isolate from the genome of which was prepared cDNAs deposited in a lambda gt-11 cDNA library with the American Type Culture Collection (ATCC) under Accession.No. 40394.

41. A polypeptide according to any one of claims 37 to 40 wherein said contiguous sequence is selected from within amino acids 1-84 or from within amino acids 9-177 of an HCV polyprotein.

42. A polypeptide according to any one of claims 37 to 41 wherein said contiguous sequence is within the sequences selected from the group consisting Figure 4, 11, 12 and 17.

43. A polypeptide according to any one of claims 37 to 42 wherein said contiguous sequence comprises at least 15 amino acids.

44. A polypeptide according to any one of claims 37 to 43 wherein said contiguous sequence has the formula AAx-AAy wherein x and y designate amino acid numbers shown in Figure 17:
AA1-AA25;AA1-AA50;AA1-AA84;AA9-AA177;AA1-AA10; AA5-AA20;AA35-AA45;AA50-AA100;AA40-AA90;AA45-AA65; AA65-AA75;AA80-AA90;AA99-AA120;AA95-AA110;AA105-AA120; AA100-AA150;AA150-AA200;AA155-AA170;AA190-AA210; AA200-AA250;AA220-AA240; AA245-A265; AA250-AA300; AA290-AA330;AA290-305;AA300-AA350;AA310-AA330; AA350-AA400;AA380-AA395;AA405-AA495;AA400-AA450; AA405-AA415;AA415-AA425;AA425-AA435;AA440-AA460; AA437-AA582.

45. A polypeptide according to any one of claims 37 to 44 wherein said polypetide reacts immunologically with anti-HCV antibodies.

46. A polypetide as defined in any one of claims 37 to 45 attached to a solid substrate.

47. An immunoassay kit which comprises a polypetide according to any one of claims 37 to 46 in a suitable container.

48. A polynucleotide in substantially isolated form which comprises an HCV genomic sequence or the complement thereof which is selectively hybridizable to a polynucleotide found within nucleotides -319 to 1348 of an HCV genome or the complement thereof, wherein HCV is **characterized by**:
a positive stranded RNA genome;
said genome comprising an open reading frame (ORF) encoding a polyprotein; and
the entirety of the said encoded polyprotein having at least 40% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

49. A polynucleotide according to claim 48 wherein said polyprotein has at least 70% homology to the entire polyprotein selected from (i) the polyprotein as shown in Figure 17, or (ii) the polyprotein of a viral isolate from the genome of which was prepared cDNA deposited in a lambda gt-11 cDNA library with the American Type Culture Collection (ATCC) under Accession No. 40394.

50. A polynucleotide according to claim 48 wherein said polyprotein has at least 80% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

51. A polynucleotide according to claim 48 wherein said polyprotein has at least 90% homology to the entire polyprotein selected from (i) the polyprotein as shown in Figure 17, or (ii) the polyprotein of a viral isolate from the genome of which was prepared cDNAs deposited in a lambda gt-11 cDNA library with the American Type Culture Collection (ATCC) under Accession No. 40394.

52. A polynucleotide according to claims 48 or 51 wherein said HCV genomic sequence or the complement thereof is found within nucleotides -319 to 1348 or within nucleotides -319 to -1 of an HCV genome.

53. A polynucleotide according to any one of claims 48 to 52 wherein said HCV genomic sequence or the complement thereof is selected from the group of sequences found within Figures 11, 12 and 17.

54. A polynucleotide according to any one of claims 48 to 53 which is DNA.

55. A polynucleotide according to any one of claim 48 to 54 which is a probe or a primer for DNA synthesis.

56. A polynucleotide according to claim 55 wherein the probe is labelled.

57. A kit for analysing biological samples for the presence of HCV polynucleotides which comprises a polynucleotide according to any one of claims 48 to 56 in a suitable cantainer.

58. A recombinant vector comprising a polynucleotide sequence which encodes a polypeptide as defined in any one of claims 37 to 45 wherein said sequence which encodes said polypeptide is operably linked to a control sequence capable of expressing the polypeptide.

59. A recombinant vector according to claim 58 wherein said polynucleotide is DNA.

60. A host cell transformed with the vector according to either of claims 58 or 59.

61. A pharmaceutical composition comprising:
a. polynucleotide as defined in any one of claims 48 to 54; or
b. a polypeptide as denned in any one of claims 37 to 45; and
c. a pharmaceutically acceptable carrier.

## Patentansprüche (Patentansprüche für folgende(n) Vertragsstaat(en): AT, DE, CH, De, DK, FR, GB, IT, LI, LU, NL, SE)

1. Polypeptid in im Wesentlichen isolierter Form, das eine zusammenhängende Sequenz eines Hepatitis C Virus (HCV)-Polyproteins von mindestens 10 Aminosäuren umfasst, bei dem die zusammenhängende Sequenz im Bereich der Aminosäuren 1 bis 449 eines HCV-Polyproteins liegt, wobei HCV **gekennzeichnet ist durch**:
ein RNA-Genom mit Positivstrangordnung;
wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

2. Polypeptid nach Anspruch 1, bei dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

3. Polypeptid nach Anspruch 1, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

4. Polypeptid nach Anspruch 1, bei dem das Polyprotein mindestens 90 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

5. Polypeptid nach einem der Ansprüche 1 bis 4, bei dem die zusammenhängende Sequenz ausgewählt ist,aus dem Bereich der Aminosäuren 1 - 84 oder dem Bereich der Aminosäuren 9 - 177 eines HCV-Polyproteins.

6. Polypeptid nach einem der Ansprüche 1 bis 5, bei dem die zusammenhängende Sequenz innerhalb der Sequenzen liegt, die aus der Gruppe ausgewählt sind bestehend aus Figur 4, 11, 12 und 17.

7. Polypeptid nach einem der vorhergehenden Ansprüche, bei dem die zusammenhängende Sequenz mindestens 15 Aminosäuren umfasst.

8. Polypeptid nach einem der Ansprüche 1 bis 7, bei dem die zusammenhängende Sequenz die Formel AAx-AAy hat, bei der x und y die in Figur 17 dargestellten Aminosäurenummern angeben:
AA1-AA25, AA1-AA50, AA1-AA84, AA9-AA177, AA1-AA10, AA5-AA20, AA35-AA45, AA50-AA100, AA40-AA90, AA45-AA65, AA65-AA75, AA80-AA90, AA99-AA120, AA95-AA110, AA105-AA120, AA100-AA150, AA150-AA200, AA155-AA170, AA190-AA210, AA200-AA250, AA220-AA240, AA245-AA265, AA250-AA300, AA290-AA330, AA290-AA305, AA300-AA350, AA310-AA330, AA350-AA400, AA380-AA395, AA405-AA495, AA400-AA450, AA405-AA415, AA415-AA425, AA425-AA435, AA440-AA460, AA437-AA582.

9. Polypeptid nach einem der vorhergehenden Ansprüche, bei dem das Polypeptid immunologisch mit anti-HCV-Antikörpern reagiert.

10. Polypeptid, wie in einem der vorhergehenden Ansprüche definiert, gebunden an ein festes Substrat.

11. Immuntest-Kit, das ein Polypeptid gemäß einem der vorhergehenden Ansprüche in einem geeigneten Behälter umfasst.

12. Immuntest-Verfahren zum Detektieren von gegen ein Hepatitis C-Virus (HCV)-Polypeptid gerichtete Antikörpern, das die Schritte umfasst:
(a) Bereitstellen eines ersten Polypeptids, das immunologisch reaktiv mit anti-HCV-Antikörpern ist, bestimmt durch Bindungskonkurrenz mit einem zweiten Polypeptid, das eine HCV-antigenische Sequenz von mindestens 10 zusammenhängenden Aminosäuren aus dem Bereich der Aminosäurenummern 1-449 eines HCV-Polyproteins umfasst.
(b) Inkubieren des ersten Polypeptids mit einer biologischen Probe unter Bedingungen, welche die Bildung eines Antikörper-Polypeptid-Komplexes ermöglichen; und
(c) Detektieren jedes das Polypeptid umfassenden Antikörper-Polypeptid-Komplexes, wobei HCV **gekennzeichnet ist durch**:
ein RNA-Genom mit Positivstrangordnung;
wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

13. Immuntest nach Anspruch 12, bei dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

14. Immnuntest nach Anspruch 12, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

15. Immuntest nach Anspruch 12, bei dem das Polyprotein mindestens 90 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

16. Immuntest nach einem der Ansprüche 12 bis 15, bei dem die Aminosäuren aus dem Bereich der Aminosäurenummern 1-449 des HCV-Polyproteins aus Figur 17 ausgewählt sind.

17. Immuntest nach einem der Ansprüche 12 bis 16, bei dem das erste Polypeptid eine HCV-antigenische Sequenz aus dem Bereich der Aminosäurennummern 1-449 des HCV-Polyproteins aus Figur 17 umfasst.

18. Immuntest nach einem der Ansprüche 12 bis 17, bei dem die biologische Probe menschliches Blut, Serum oder Plasma ist.

19. Polynukleotid in im Wesentlichen isolierter Form, das eine HCV-genomische Sequenz oder deren Komplement umfasst, die selektiv an ein Polynukleotid hybridisierbar ist, das im Bereich der Nukleotide -319 bis 1348 eines HCV-Genoms oder dem Komplement davon vorkommt, wobei HCV **gekennzeichnet ist durch**:
ein RNA-Genom mit Positivstrangordnung;
wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

20. Polynukleotid nach Anspruch 19, bei dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

21. Polynukleotid nach Anspruch 19, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

22. Polynukleotid nach Anspruch 19, bei dem das Polyprotein mindestens 90 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

23. Polynukleotid nach einem der Ansprüche 19 bis 22, bei dem die HCV-genomische Sequenz oder das Komplement davon innerhalb der Nukleotide -319 bis 1348 oder innerhalb der Nukleotide -319 bis -1 eines HCV-Genoms vorkommt.

24. Polynukleotid nach einem der Ansprüche 19 bis 23, bei dem die HCV-genomische Sequenz oder das Komplement davon ausgewählt ist aus der Gruppe von Sequenzen, die innerhalb der Figuren 11, 12 und 17 gefunden wird.

25. Polynukleotid nach einem der Ansprüche 19 bis 24, das DNA ist.

26. Polynukleotid nach einem der Ansprüche 19 bis 25, das eine Sonde oder ein Primer für die DNA-Synthese ist.

27. Polynukleotid nach Anspruch 26, bei dem die Sonde markiert ist.

28. Kit zur Analyse biologischer Proben auf die Anwesenheit von HCV-Polynukleotiden, das ein Polynukleotid gemäß einem der Ansprüche 19 bis 27 in einem geeigneten Behälter umfasst.

29. Verfahren zum Detektieren von HCV-Nukleinsäuren in einer Probe, das die Schritte umfasst:
a. Bereitstellen eines Polynukleotids, wie es in einem der Ansprüche 19 bis 27 definiert ist;
b. Umsetzen des Polynukleotids mit der Probe unter Bedingungen, welche die selektive Bildung von Polynukleotidduplexen zwischen dem Polynukleotid und jeder HCV-Nukleinsäure oder HCV-cDNA in der Probe erlauben; und
c. Detektieren jedes der das Polynukleotid enthaltenden Polynukleotidduplexe.

30. Verfahren zum Amplifizieren eines Polynukleotids des HCV in einer Probe, das die Schritte umfasst:
a. Amplifizieren des Polynukleotids des HCV unter Verwendung einer DNA-Polymerase und mindestens eines in einem der Ansprüche 19 bis 27 definierten Polynukleotids; und
b. Detektieren des amplifizierten Polynukleotids.

31. Verfahren nach Anspruch 29 oder 30, bei dem die Probe menschliches Blut, Serum oder Plasma ist.

32. Rekombinanter Vektor, der eine Polynukleotidsequenz umfasst, die für ein Polypeptid wie in einem der Ansprüche 1 bis 9 definiert kodiert, wobei die Sequenz, die für das Polypeptid kodiert, funktional an eine Kontrollsequenz gekoppelt ist, die zur Expression des Polypeptids befähigt ist.

33. Rekombinanter Vektor nach Anspruch 32, bei dem das Polynukleotid DNA ist.

34. Wirtszelle, die mit dem Vektor gemäß Anspruch 32 oder 33 transformiert ist.

35. Verfahren zum Herstellen eines HCV-Polypeptids, das Inkubieren einer Wirtszelle gemäß Anspruch 34 unter Bedingungen umfasst, welche die Expression der Sequenz, die für ein Polypeptid kodiert, und Gewinnung des Polypeptids erlauben.

36. Verfahren zum Herstellen eines HCV-Polypeptids, das chemisches Synthetisieren eines Polypeptids gemäß einem der Ansprüche 1 bis 9 umfasst.

37. Pharmazeutische Zusammensetzung, die
a. ein Polynukleotid wie in einem der Ansprüche 19 bis 25 definiert; oder
b. ein Polypeptid wie in einem der Ansprüche 1 bis 9 definiert; und
c. einen pharmazeutisch verträglichen Träger
umfasst.

38. Verfahren zum Herstellen einer mit einem rekombinanten Vektor gemäß Anspruch 32 oder 33 transformierten Wirtszelle, das die Schritte umfasst:
a. Bereitstellen einer zur Transformation befähigten Wirtszelle;
b. Bereitstellen des rekombinanten Vektors; und
c. Inkubieren von (a) mit (b) unter Bedingungen, welche die Transformation der Wirtszelle mit dem Vektor ermöglichen.

39. Verfahren zum Herstellen eines rekombinanten Vektors gemäß Anspruch 32 oder 33, das die Schritte umfasst:
a. Bereitstellen einer mit einem rekombinanten Polynukleotid gemäß einem der Ansprüche 19 bis 27 transformierten Wirtszelle; und
b. Isolieren des Polynukleötids aus der Wirtszelle.

40. Verfahren zum Erzeugen einer Polynukleotidsonde, das chemisches Synthetisieren eines Polynukleotids gemäß einem der Ansprüche 19 bis 27 umfasst.

41. Verfahren zum Herstellen eines Vorrats von humanen biologischen Proben vor deren Verwendung, wobei das Verfahren die Schritte umfasst:
(a) Bereitstellen eines Vorrats von humanen biologischen Proben; und
(b) Entfernen solcher Proben aus dem Vorrat von humanen biologischen Proben, die:
(i) zur Detektierung in einem Verfahren gemäß Anspruch 29 oder 30 befähigte Polynukleotide;
(ii) zur Bildung detektierbarer Polypeptid-Antikörper-Komplexe in einem Immuntest gemäß einem der Ansprüche 12 bis 18 befähigte Antikörper
enthalten.

42. Verfahren nach Anspruch 41, bei dem die humanen biologischen Proben Blut, Plasma oder Seren sind.

43. Verwendung einer biologischen Probe, die gemäß dem Verfahren aus Anspruch 41 oder 42 hergestellt wurde, für die Herstellung von Produkten, die im Zusammenhang mit Blut stehen.

## Patentansprüche (Patentansprüche für folgende(n) Vertragsstaat(en): GR, SP)

1. Verfahren zum Herstellen eines Polypeptids in im Wesentlichen isolierter Form, wobei das Polypeptid eine zusammenhängende Sequenz eines Hepatitis C Virus (HCV)-Polyproteins von mindestens 10 Aminosäuren umfasst, wobei sich die zusammenhängende Sequenz im Bereich der Aminosäuren 1 bis 449 eines HCV-Polyproteins befindet, wobei HCV **gekennzeichnet ist durch**:
ein RNA-Genom mit Positivstrangordnung;
wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden,
**dadurch gekennzeichnet, daß** das Verfahren ausgewählt ist aus der Gruppe von Verfahren, die chemische Synthese, Expression eines rekombinanten Expressionssystems und Isolierung von HCV-Mutanten umfasst.

2. Verfahren nach Anspruch 1, bei dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

3. Verfahren nach Anspruch 1, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

4. Verfahren nach Anspruch 1, bei dem das Polyprotein mindestens 90 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

5. Verfahren nach einem der Ansprüche 1 bis 4, bei dem die zusammenhängende Sequenz ausgewählt ist aus dem Bereich der Aminosäuren 1 - 84 oder dem Bereich der Aminosäuren 9 - 177 eines HCV-Polyproteins.

6. Verfahren nach einem der Ansprüche 1 bis 5, bei dem sich die zusammenhängende Sequenz innerhalb der Sequenzen befindet, die aus der Gruppe ausgewählt sind bestehend aus Figur 4, 11, 12 und 17.

7. Verfahren nach einem der vorhergehenden Ansprüche, bei dem die zusammenhängende Sequenz mindestens 15 Aminosäuren umfasst.

8. Verfahren nach einem der Ansprüche 1 bis 7, bei dem die zusammenhängende Sequenz die Formel AAx-AAy hat, bei der x und y die in Figur 17 dargestellten Aminosäurenummern angeben:
AA1-AA25, AA1-AA50, AA1-AA84, AA9-AA177, AA1-AA10, AA5-AA20, AA35-AA45, AA50-AA100, AA40-AA90, AA45-AA65; AA65-AA75, AA80-AA90, AA99-AA120, AA95-AA110, AA105-AA120, AA100-AA150, AA150-AA200, AA155-AA170, AA190-AA210, AA200-AA250, AA220-AA240, AA245-AA265, AA250-AA300, AA290-AA330, AA290-AA305, AA300-AA350, AA310-AA330, AA350-AA400, AA380-AA395, AA405-AA495, AA400-AA450, AA405-AA415, AA415-AA425, AA425-AA435, AA440-AA460, AA437-AA582.

9. Verfahren nach einem der vorhergehenden Ansprüche, bei dem das Polypeptid immunologisch mit anti-HCV-Antikörpern reagiert.

10. Verfahren, wie in einem der vorhergehenden Ansprüche definiert, das ferner die Bindung des Polypeptids an ein festes Substrat umfasst.

11. Immuntest-Verfahren zum Detektieren von gegen ein Hepatitis C-Virus (HCV)-Polypeptid gerichtete Antikörpern, das die Schritte umfasst:
(a) Bereitstellen eines ersten Polypeptids, das immunologisch reaktiv mit anti-HCV-Antikörpern ist, bestimmt durch Bindungskonkurrenz mit einem zweiten Polypeptid, das eine HCV-antigenische Sequenz von mindestens 10 zusammenhängenden Aminosäuren aus dem Bereich der Aminosäurenummern 1-449 eines HCV-Polyproteins umfasst.
(b) Inkubieren des ersten Polypeptids mit einer biologischen Probe unter Bedingungen, welche die Bildung eines Antikörper-Polypeptid-Komplexes ermöglichen; und
(c) Detektieren jedes das Polypeptid umfassenden Antikörper-Polypeptid-Komplexe, bei denen HCV **gekennzeichnet ist durch**:
ein RNA-Genom mit Positivstrangordnung;
wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

12. Immuntest-Verfahren nach Anspruch 11, bei.dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

13. Immnuntest-Verfahren nach Anspruch 11, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

14. Immuntest-Verfahren nach Anspruch 11, bei dem das Polyprotein mindestens 90 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

15. Immuntest-Verfahren nach einem der Ansprüche 11 bis 14, bei dem die Aminosäuren aus dem Bereich der Aminosäurenummern 1-449 des HCV-Polyproteins aus Figur 17 ausgewählt sind.

16. Immuntest-Verfahren nach einem der Ansprüche 11 bis 15, bei dem das erste Polypeptid eine HCV-antigenische Sequenz aus dem Bereich der Aminosäurennummern 1-449 des HCV-Polyproteins aus Figur 17 umfasst.

17. Immuntest-Verfahren nach einem der Ansprüche 11 bis 16, bei dem die biologische Probe menschliches Blut, Serum oder Plasma ist.

18. Verfahren zum Herstellen eines Polynukleotids in im Wesentlichen isolierter Form, wobei das Polynukleotid eine HCV-genomische Sequenz oder deren Komplement umfasst, die selektiv an ein Polynukleotid hybridisierbar ist, das im Bereich der Nukleotide -319 bis 1348 eines HCV-Genoms oder dem Komplement davon vorkommt, wobei HCV **gekennzeichnet ist durch**:
ein RNA-Genom mit Positivstrangordnung;
wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden,
**dadurch gekennzeichnet, daß** das Verfahren ausgewählt ist aus der Gruppe von Verfahren, die chemische Synthese, DNA-Replikaiton, reverse Transkription und Transkription umfasst.

19. Verfahren nach Anspruch 18, bei dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

20. Verfahren nach Anspruch 18, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

21. Verfahren nach Anspruch 18, bei dem das Polyprotein mindestens 90 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

22. Verfahren nach einem der Ansprüche 18 bis 21, bei dem die HCV-genomische Sequenz oder das Komplement davon innerhalb der Nukleotide -319 bis 1348 oder innerhalb der Nukleotide -319 bis -1 eines HCV-Genoms vorkommt.

23. Verfahren nach einem der Ansprüche 18 bis 22, bei dem die HCV-genomische Sequenz oder das Komplement davon ausgewählt ist aus der Gruppe von Sequenzen, die innerhalb der Figuren 11, 12 und 17 gefunden wird.

24. Verfahren nach einem der Ansprüche 18 bis 23, bei dem das Polynukleotid DNA ist.

25. Verfahren nach einem der Ansprüche 18 bis 24, bei dem das Polynukleotid eine Sonde oder ein Primer für die DNA-Synthese ist.

26. Verfahren nach Anspruch 25, bei dem die Sonde markiert ist.

27. Verfahren zum Detektieren von HCV-Nukleinsäuren in einer Probe, das die Schritte umfasst:
a. Bereitstellen eines Polynukleotids, wie es in einem der Ansprüche 18 bis 26 definiert ist;
b. Reagieren des Polynukleotids mit der Probe unter Bedingungen, welche die selektive Bildung von Polynukleotidduplexen zwischen dem Polynukleotid und jeder HCV-Nukleinsäure oder HCV-cDNA in der Probe erlauben; und
c. Detektieren jedes das Polynukleotid enthaltenden Polynukleotidduplexe.

28. Verfahren zum Amplifizieren eines Polynukleotids des HCV in einer Probe, das die Schritte umfasst:
a. Amplifizieren des Polynukleotids des HCV unter Verwendung einer DNA-Polymerase und mindestens eines in einem der Ansprüche 18 bis 25 definierten Polynukleotids; und
b. Detektieren des amplifizierten Polynukleotids.

29. Verfahren nach Anspruch 27 oder 28, bei dem die Probe menschliches Blut, Serum oder Plasma ist.

30. Verfahren zum Herstellen eines rekombinanten Vektors, der eine Polynukleotidsequenz umfasst, die für ein Polypeptid wie in einem der Ansprüche 1 bis 9 definiert kodiert, wobei die Sequenz, die für das Polypeptid kodiert, funktional an eine Kontrollsequenz gekoppelt ist, die zur Expression des Polypeptids befähigt ist, **dadurch gekennzeichnet, daß** das Verfahren die Gewinnung von für das gewünschte Polypeptid kodierenden Fragmenten aus cDNA-Klonen durch Restriktionsverdau oder chemisches Synthetisieren der Polynukleotidsequenz und Ligieren der Polynukleotidsequenz in einen Vektor umfasst.

31. Verfahren nach Anspruch 30, bei dem das Polynukleotid DNA ist.

32. Verfahren zum Herstellen einer mit einem gemäß Anspruch 30 oder 31 hergestellten rekombinanten Vektor transformierten Wirtszelle, das die Schritte umfasst:
a. Bereitstellen einer zur Transformation befähigten Wirtszelle;
b. Bereitstellen des rekombinanten Vektors; und
c. Inkubieren von (a) mit (b) unter Bedingungen, welche die Transformation der Wirtszelle mit dem Vektor ermöglichen.

33. Verfahren zum Herstellen eines HCV-Polypeptids, das Inkubieren einer Wirtszelle gemäß Anspruch 32 unter Bedingungen umfasst, welche die Expression der Sequenz, die für ein Polypeptid kodiert, und Gewinnung des Polypeptids erlauben.

34. Verfahren zum Erzeugen einer Polynukleotidsonde, das chemisches Synthetisieren eines Polynukleotids gemäß einem der Ansprüche 18 bis 26 umfasst.

35. Verfahren zum Herstellen eines Vorrats von humanen biologischen Proben vor deren Verwendung, wobei das Verfahren die Schritte umfasst:
(a) Bereitstellen eines Vorrats von humanen biologischen Proben; und
(b) Entfernen solcher Proben aus dem Vorrat von humanen biologischen Proben, die:
(i) zur Detektierung in einem Verfahren gemäß Anspruch 27 oder 28 befähigte Polynukleotide;
(ii) zur Bildung detektierbarer Polypeptid-Antikörper-Komplexe in einem Immuntest-Verfahren gemäß einem der Ansprüche 11 bis 17 befähigte Antikörper
enthalten.

36. Verfahren nach Anspruch 35, bei dem die humanen biologischen Proben Blut, Plasma oder Seren sind.

37. Polypeptid in im Wesentlichen isolierter Form, das eine zusammenhängende Sequenz eines Hepatitis C Virus (HCV)-Polyproteins von mindestens 10 Aminosäuren umfasst, bei dem die zusammenhängende Sequenz im Bereich der Aminosäuren 1 bis 449 eines HCV-Polyproteins liegt, wobei HCV **gekennzeichnet ist durch**:
ein RNA-Genom mit Positivstrangordnung; wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

38. Polypeptid nach Anspruch 37, bei dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

39. Polypeptid nach Anspruch 37, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

40. Polypeptid nach Anspruch 37, bei dem das Polyprotein mindestens 90 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

41. Polypeptid nach einem der Ansprüche 37 bis 40, bei dem die zusammenhängende Sequenz ausgewählt ist aus dem Bereich der Aminosäuren 1 - 84 oder dem Bereich der Aminosäuren 9 - 177 eines HCV-Polyproteins.

42. Polypeptid nach einem der Ansprüche 37 bis 41, bei dem die zusammenhängende Sequenz innerhalb der Sequenzen liegt, die aus der Gruppe ausgewählt sind bestehend aus Figur 4, 11, 12 und 17.

43. Polypeptid nach einem der Ansprüche 37 bis 42, bei dem die zusammenhängende Sequenz mindestens 15 Aminosäuren umfasst.

44. Polypeptid nach einem der Ansprüche 37 bis 43, bei dem die zusammenhängende Sequenz die Formel AAx-AAy hat, bei der x und y die in Figur 17 dargestellten Aminosäurenummern angeben:
AA1-AA25, AA1-AA50, AA1-AA84, AA9-AA177, AA1-AA10, AA5-AA20, AA35-AA45, AA50-AA100, AA40-AA90, AA45-AA65, AA65-AA75, AA80-AA90, AA99-AA120, AA95-AA110, AA105-AA120, AA100-AA150, AA150-AA200, AA155-AA170, AA190-AA210, AA200-AA250, AA220-AA240, AA245-AA265, AA250-AA300, AA290-AA330, AA290-AA305, AA300-AA350, AA310-AA330, AA350-AA400, AA380-AA395, AA405-AA495, AA400-AA450, AA405-AA415, AA415-AA425, AA425-AA435, AA440-AA460, AA437-AA582.

45. Polypeptid nach einem der Ansprüche 37 bis 44, bei dem das Polypeptid immunologisch mit anti-HCV-Antikörpern reagiert.

46. Polypeptid, wie in einem der Ansprüche 37 bis 45 definiert, gebunden an ein festes Substrat.

47. Immuntest-Kit, das ein Polypeptid gemäß einem der Ansprüche 37 bis 46 in einem geeigneten Behälter umfasst.

48. Polynukleotid in im Wesentlichen isolierter Form, das eine HCV-genomische Sequenz oder deren Komplement umfasst, die selektiv an ein Polynukleotid hybridisierbar ist, das im Bereich der Nukleotide -319 bis 1348 eines HCV-Genoms oder dem Komplement davon vorkommt, wobei HCV **gekennzeichnet ist durch**:
ein RNA-Genom mit Positivstrangordnung;
wobei das Genom einen für ein Polyprotein kodierenden offenen Leserahmen (ORF) umfasst; und
die Gesamtheit des kodierten Polyproteins mindestens 40 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

49. Polynukleotid nach Anspruch 48, bei dem das Polyprotein mindestens 70 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

50. Polynukleotid nach Anspruch 48, bei dem das Polyprotein mindestens 80 % Homologie zu dem gesamten Polyprotein hat, das ausgewählt ist aus (i) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

51. Polynukleotid nach Anspruch 48, bei dem das Polyprotein mindestens 90 % Homologie zu dem gesamten Polyprotein hat, das ausgewählt ist aus (1) dem wie in Figur 17 gezeigten Polyprotein, oder (ii) dem Polyprotein eines viralen Isolates des Genoms, von dem die in einer Lambda gt-11 cDNA-Bibliothek bei der American Type Culture Collection (ATCC) mit der Zugangskodenummer 40394 hinterlegten cDNAs hergestellt wurden.

52. Polynukleotid nach einem der Ansprüche 48 oder 51, bei dem die HCV-genomische Sequenz oder das Komplement davon innerhalb der Nukleotide -319 bis 1348 oder innerhalb der Nukleotide -319 bis -1 eines HCV-Genoms vorkommt.

53. Polynukleotid nach einem der Ansprüche 48 bis 52, bei dem die HCV-genomische Sequenz oder das Komplement davon ausgewählt ist aus der Gruppe von Sequenzen, die innerhalb der Figuren 11, 12 und 17 gefunden wird.

54. Polynukleotid nach einem der Ansprüche 48 bis 53, das DNA ist.

55. Polynukleotid nach einem der Ansprüche 48 bis 54, das eine Sonde oder ein Primer für die DNA-Synthese ist.

56. Polynukleotid nach Anspruch 55, bei dem die Sonde markiert ist.

57. Kit zur Analyse biologischer Proben auf die Anwesenheit von HCV-Polynukleotiden, das ein Polynukleotid gemäß einem der Ansprüche 48 bis 56 in einem geeigneten Behälter umfasst.

58. Rekombinanter Vektor, der eine Polynukleotidsequenz umfasst, die für ein Polypeptid wie in einem der Ansprüche 37 bis 45 definiert kodiert, wobei die Sequenz, die für das Polypeptid kodiert, funktional an eine Kontrollsequenz gekoppelt ist, die zur Expression des Polypeptids befähigt ist.

59. Rekombinanter Vektor nach Anspruch 58, bei dem das Polynukleotid DNA ist.

60. Wirtszelle, die mit dem Vektor gemäß Anspruch 58 oder 59 transformiert ist.

61. Pharmazeutische Zusammensetzung, die
a. ein Polynukleotid wie in einem der Ansprüche 48 bis 54 definiert; oder
b. ein Polypeptid wie in einem der Ansprüche 37 bis 45 definiert; und
c. einen pharmazeutisch verträglichen Träger
umfasst.

## Revendications (Revendications pour l'(les) Etat(s) contractant(s) suivant(s): AT, BE, CH, DE, DK, FR, GB, IT, LI, LU, NL, SE)

1. Polypeptide sous forme essentiellement isolée comprenant une séquence contiguë issue d'une polyprotéine du virus de l'hépatite C (VHC) d'au moins 10 acides aminés, dans lequel ladite séquence contiguë est comprise entre les acides aminés 1 et 449 d'une polyprotéine du VHC, dans lequel le VHC se **caractérise par** :
un génome si le brin de polarité positive :
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

2. Polypeptide selon la revendication 1 dans lequel ladite polyprotéine a au moins 70 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

3. Polypeptide selon la revendication 1 dans lequel ledit polypeptide a au moins 80 % d'homologie avec la polyprotéine entière choisie à partir de (i) la polyprotéine illustrée en usure 17, ou (ii) la polyprotéine d'un isolat viral à partir du génome duquel ont été préparés les déposés dans une banque d'ADNc lambda gt-11 auprès de l'ATCC (American Type Culture Collection) sous le numéro d'accès 40394.

4. Polypeptide selon la revendication 1 dans lequel ledit polypeptide a au moins 90 % d'homologie la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

5. Polypeptide selon l'une quelconque des revendications 1 à 4 dans lequel ladite séquence contiguë est choisie entre les acides aminés 1 et 84 ou entre les acides aminés 9 et 177 d'une polyprotéine du VHC.

6. Polypeptide selon l'une quelconque des revendications 1 à 5 dans lequel ladite séquence contiguë est comprise dans les séquences sélectionnées dans le groupe constitué par les figures 4, 11; 12 et 17.

7. Polypeptide selon l'une quelconque des revendications précédentes dans lequel ladite séquence contiguë comprend au moins 15 acides aminés.

8. Polypeptide selon l'une quelconque des revendications 1 à 7 dans lequel ladite séquence contiguë est de formule AAx-AAy dans laquelle x et y désignent les numéros des acides aminés illustrés en ligure 17 :
AA1-AA25,AA1-AA50;AA1-AA84;AA9-AA177;AA1-AA10; AA5-AA20;AA35-AA45,AA50-AA100;AA40-AA90;AA45-AA65; AA65-AA75;AA80-AA90;AA99-AA120;AA95-AA110;AA105-AA120; AA100-AA150;AA150-AA200;AA155-AA170:AA190-AA210; AA200-A250;AA220-AA240;AA245-AA265;AA250-AA300; AA290-AA330;AA290-AA305;AA300-AA350;AA310-AA330; AA350-AA400;AA380-AA395;AA405-AA495;AA400-AA450; AA405-AA415;AA415-AA425-AA425-A435;AA440-AA460; AA437-AA582.

9. Polypeptide selon l'une quelconque des revendications précédentes dans lequel ledit polypeptide réagit immunologiquement avec des anticorps anti-VHC.

10. Polypeptide tel que défini selon l'une quelconque des revendications précédentes fixé à un substrat solide.

11. Kit d'essai immunologique qui comprend un polypeptide selon l'une quelconque des revendications précédentes dans un conteneur approprié.

12. Procédé d'essai immunologique de détection d'anticorps dirigés contre un polypeptide du virus de l'hépatite C (VHC) comprenant les étapes consistant à:
(a) fournir un premier polypeptide qui est immunologiquement réactif avec des anticorps anti-VHC comme déterminé par compétition avec la liaison d'un second polypeptide comprenant une séquence antigénique VHC d'au moins 10 acides aminés contigus comprise entre les acides aminés numéro 1 et 449 d'une polyprotéine du VHC.
(b) incuber ledit premier polypeptide avec un échantillon biologique dans des conditions qui permettent la formation d'un complexe anticorps-polypeptide; et
(c) détecter tout complexes anticorps-polypeptide comprenant ledit polypeptide, dans lequel le VHC se **caractérise par** :
un génome ARN simple brin de polarité positive ;
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

13. Essai immunologique selon la revendication 12 dans lequel ladite polyprotéine a au moins 70 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

14. Essai immunologique selon la revendication 12 dans lequel ladite polyprotéine a au moins 80 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une bibliothèque d'ADNc lambda gt-11 auprès de l'ATCC (American Type Culture Collection) sous le numéro d'accès 40394.

15. Essai immunologique selon la revendication 12 dans lequel ladite polyprotéine a au moins 90 % d'homologie avec la polyprotéine entière choisie à partir de (i) la polyprotéine illustrée en figure 17, ou (ii) la polyprotéine d'un isolat viral à partir du génome duquel ont étépréparés les ADNc déposés dans une banque d'ADNc lambda gt-11 auprès l'ATCC (American Type Culture Collection) sous le numéro d'accès 40394.

16. Essai immunologique selon l'une quelconque des revendications 12 à 15 dans lequel lesdits acides aminés sont choisis à partir des acides aminés 1 à 449 de la polyprotéine du VHC de la figure 17.

17. Essai immunologique selon l'une quelconque des revendications 12 à 16 dans lequel ledit premier polypeptide comprend une séquence antigénique du VHC comprise entre les acides aminés numéros 1 et 449 de la polyprotéine du VHC de la figure 17.

18. Essai immunologique selon l'une quelconque des revendications 12 à 17 dans lequel l'échantillon biologique est du sang, du sérum ou du plasma humain.

19. Polynucléotide sous forme essentiellement isolée qui comprend une séquence génomique du VHC ou le complément de celle-ci qui est sélectivement hybridable à un polynucléotide présent entre les nucléotides -319 et 1348 d'un génome du VHC ou du complément de celui-ci, dans lequel le VHC se **caractérise par** :
un génome ARN simple brin de polarité positive ;
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolai viral à partir du génome duquel ont été préparés les ADNc déposés dans une banque d'ADNc lambda gt-11 auprès de l'ATCC (American Type Culture Collection) sous le numéro d'accès 40394.

20. Polynucléotide selon la revendication 19 dans lequel ladite polyprotéine a au moins 70 % d'homologie avec la polyprotéine entière choisie à partir de (i) la polyprotéine illustrée en figure 17, ou (ii) la polyprotéine d'un isolai viral à partir du génome duquel ont été préparés les ADNc déposés dans une banque d'ADNc lambda gt-11 auprès de l'ATCC (American Type Culture Collection) sous le numéro d'accès 40394.

21. Polynucléotide selon la revendication 19 dans lequel ladite polyprotéine a au moins 80 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès l'ATCC (American Type Culture Collection) sous le numéro d'accès 40394.

22. Polynucléotide selon la revendication 19 dans lequel ladite pol^{y}protéine a au moins 90 % d'homologie avec la polyprotéine entière choisie à partir de (i) la polyprotéine illustrée en figure 17, ou (ii) la polyprotéine d'un isolat viral à partir du génome duquel étaient préparés les ADNc déposés dans une banque d'ADNc lambda gt-11 auprès de l'ATCC (American Type Culture Collection) sous le numéro d'accès 40394.

23. Polynucléotide selon l'une quelconque des revendications 19 à 22 dans lequel ladite séquence génomique du VHC ou le complément de celle-ci est compris(e) entre les nucléotides -319 et 1348 ou entre les nucléotides -319 et -1 d'un génome du VHC.

24. Polynucléotide selon l'une quelconque des revendications 19 à 23 dans lequel ladite séquence génomique du VHC ou le complément de celle-ci est choisi(e) à partir du groupe de séquences présent sur les figures 11, 12 et 17.

25. Polynucléotide selon l'une quelconque des revendications 19 à 24 qui est de l'ADN.

26. Polynucléotide selon l'une quelconque des revendications 19 à 25 qui est une sonde ou une amorce pour la synthèse d'ADN.

27. Polynucléotide selon la revendication 26 dans lequel la sonde est marquée.

28. Kit d'analyse d'échantillons biologiques pour détecter la présence de polynucléotides du VHC qui comprend un polynucléotide selon l'une quelconque des revendications 19 à 27 dans un conteneur approprié.

29. Procédé de détection d'acides nucléiques du VHC dans un échantillon qui comprend les étapes consistant à :
a. fournir un polynucléotide tel que défini selon l'une quelconque des revendications 19 à 27 ;
b. faire réagir ledit polynucléotide avec ledit échantillon dans des conditions qui permettent la formation sélective de duplexes polynucléotidiques entre ledit polynucléotide et tout acide nucléique du VHC ou tout ADNc du VHC dans ledit échantillon; et
c. détecter tout duplex polynucléotidique contenant ledit polynucléotide.

30. Procédé d'amplification d'un polynucléotide du VHC dans un échantillon qui comprend les étapes consistant à :
a. amplifier le polynucléotide du VHC en utilisant une ADN polymérase et au moins un polynucléotide défini selon l'une quelconque des revendications 19 à 27 ; et
b. détecter le polynucléotide amplifié.

31. Procédé selon la revendication 29 ou 30 dans lequel ledit échantillon est du sang, du sérum ou du plasma humain.

32. Vecteur recombinant comprenant une séquence polynucléotidique qui code un polypeptide tel que défini selon l'une quelconque des revendications 1 à 9 dans lequel ladite séquence qui code ledit polypeptide est liée de manière fonctionnelle à une séquence contrôle capable d'exprimer le polypeptide.

33. Vecteur recombinant selon la revendication 32 dans lequel ledit polynucléotide est de l'ADN.

34. Cellule hôte transformée par le vecteur selon la revendication 32 ou 33.

35. Procédé de fabrication d'un polypeptide du VHC qui comprend l'incubation d'une cellule hôte selon la revendication 34 dans des conditions qui permettent l'expression de ladite séquence qui code un polypeptide; et la récupération du polypeptide.

36. Procédé de fabrication d'un polypeptide du VHC comprenant la synthèse chimique d'un polypeptide selon l'une quelconque des revendications 1 à 9.

37. Composition pharmaceutique comprenant :
a. un polynucléotide tel que défini selon l'une quelconque des revendications 19 à 25 ; ou
b. un polypeptide tel que défini selon l'une quelconque des revendications 1 à 9 ; et
c. un véhicule pharmaceutiquenent acceptable.

38. Procédé de préparation d'une cellule hôte transformée par un vecteur recombinant selon l'une quelconque des revendications 32 ou 33 comprenant les étapes consistant à :
a. fournir une cellule hôte capable de transformation ;
b. fournir le vecteur recombinant ; et
c. incuber (a) avec (b) dans des conditions qui permettent la transformation de la cellule hôte par le vecteur.

39. Procédé de préparation d'une vecteur recombinant selon la revendication 32 ou 33 comprenant les étapes consistant à :
a. fournir une cellule hôte transformée par un polynucléotide recombinant selon l'une quelconque des revendications 19 à 27; et
b. isoler ledit polynucléotide à partir de ladite cellule hôte.

40. Procédé de production d'une sonde polynucléotidique comprenant la synthèse chimique d'un polynucléotide selon l'une quelconque des revendications 19 à 27.

41. Procédé de préparation d'un approvisionnement d'échantillons biologiques humains avant usage ledit procédé comprenant les étapes consistant à :
(a) fournir un approvisionnement d'échantillons biologiques humains ; et
(b) retirer dudit approvisionnement d'échantillons biologiques humains les échantillons contenant :
(i) des polynucléotides capables de détection dans un procédé selon l'une quelconque des revendications 29 ou 30 ;
ou (ii) des anticorps capables de produire des complexes polypeptide-anticorps détectables dans un essai immunologique selon l'une quelconque des revendications 12 à 18.

42. Procédé selon la revendication 41 dans lequel lesdits échantillons biologiques humains sont du sang, du plasma ou du sérum.

43. Utilisation d'un échantillon biologique préparé selon le procédé selon la revendication 41 ou 42 pour la prépararion de produits dérivés sanguins.

## Revendications (Revendications pour l'(les) Etat(s) contractant(s) suivant(s): SP, GR)

1. Procédé de préparation d'un polypeptide sous forme essentiellement isolée, ledit polypeptide comprenant une séquence contiguë issue d'une polyprotéine du virus de l'hépatite C(VHC) d'au moins 10 acides aminés, dans lequel ladite séquence contiguë est comprise entre les acides aminés 1 et 449 d'une polyprotéine du VHC, dans lequel le VHC se **caractérise par** :
un génome ARN simple brin de polarité positive ;
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394, **caractérisé en ce que** le procédé est choisi parmi le groupe de procédés comprenant la synthèse chimique, l'expression d'un système d'expression recombinant et l'isolation à partir d'un VHC mutant.

2. Procédé selon la revendication 1 dans lequel ladite polyprotéine a au moins 70 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

3. Procédé selon la revendication 1 dans lequel ladite polyprotéine a au moins 80 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

4. Procédé selon la revendication 1 dans lequel ledit polypeptide a au moins 90 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

5. Procédé selon l'une quelconque des revendications 1 à 4 dans lequel ladite séquence contiguë est choisie à partir des acides aminés 1 à 84 ou à partir des acides aminés 9 à 177 d'une polyprotéine du VHC.

6. Procédé selon l'une quelconque des revendications 1 à 5 dans lequel ladite séquence contiguë est comprise dans les séquences sélectionnées dans le groupe constitué par les figures 4, 11, 12 et 17.

7. Procédé selon l'une quelconque des revendications précédentes dans lequel ladite séquence contiguë comprend au moins 15 acides aminés.

8. Procédé selon l'une. quelconque des revendications 1 à 7 dans lequel ladite séquence contiguë est de formule AAx-AAy dans laquelle x et y désignent les numéros des acides aminés illustrés en figure 17 :
AA1-AA25,AA1-AA50;AA1-AA84;AA9-AA177;AA1-AA10; AA5-AA20;AA35-AA45;AA50-AA100;AA40-AA90;AA45-AA65; AA65-AA75;AA80-AA90;AA99-AA120;AA95-AA110;AA105-AA120; AA100-AA150;AA150-AA200;AA155-AA170;AA190-AA210; AA200-AA250;AA220-AA240;AA245-AA265;AA250-AA300; AA290-AA330;AA290-AA305;AA300-AA350;AA310-AA330; AA350-AA400;AA380-AA395;AA45-AA495;AA400-AA450; AA405-AA415;AA415-AA425;AA425-AA435;AA440-AA460; AA437-AA582.

9. Procédé selon l'une quelconque des revendications précédentes dans lequel ledit polypeptide réagit immunologiquement avec des anticorps anti-VHC.

10. Procédé tel que défini selon l'une quelconque des revendications précédentes comprenant en outre l'étape, consistant à fixer le polypeptide à un substrat solide.

11. Procédé d'essai immunologique de détection d'anticorps dirigés contre un polypeptide du virus de l'hépatite C (VHC) comprenant les étapes consistant à :
(a) fournir un premier polypeptide qui est immunologiquement réactif avec des anticorps anti-VHC comme déterminé par compétition avec la liaison d'un second polypeptide comprenant une séquence antigénique VHC d'au moins 10 acides aminés contigus comprise entre les acides aminés numéro 1 et 449 d'une polyprotéine du VHC.
(b) incuber ledit premier polypeptide avec un échantillon biologique dans des conditions qui permettent la formation d'un complexe anticorps-polypeptide ; et
(c) détecter tout complexe anticorps-polypeptide comprenant ledit polypeptide, dans lequel le VHC se **caractérise par** :
un génome ARN simple brin de polarité positive ;
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolai viral à partir du génome duquel ont été préparés les déposés dans une banque d'ADNc lambda gt-11 auprès de l'ATCC (American Type Culture Collection) sous le numéro d'accès 40394.

12. Procédé d'essai immunologique selon la revendication 11 dans lequel ladite polyprotéine a au moins 70 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

13. Procédé d'essai immunologique selon la revendication il dans lequel ladite polyprotéine a au moins 80 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

14. Procédé d'essai immunologique selon la revendication 11 dans lequel ladite polyprotéine a au moins 90 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

15. Procédé d'essai immunologique selon l'une quelconque des revendications 11 à 14 dans lequel lesdits acides aminés sont choisis à partir des acides aminés 1 à 449 de la polyprotéine du VHC de la figure 17.

16. Procédé d'essai immunologique selon l'une quelconque des revendications 11 à 15 dans lequel ledit premier polypeptide comprend une séquence antigénique du VHC choisie à partir des acides aminés numéros 1 à 449 de la polyprotéine du VHC de la figure 17.

17. Essai immunologique selon l'une quelconque des revendications 11 à 16 dans lequel l'échantillon biologique est du sang, du sérum ou du plasma humain.

18. Procédé de préparation d'un polynucléotide sous forme essentiellement isolée ledit polynucléotide comprenant une séquence génomique du VHC ou le complément de celle-ci qui est sélectivement hybridable à un polynucléotide présent entre les nucléotides -319 et 1348 d'un génome au VHC ou complément de celui-ci, dans lequel le VHC se **caractérise par** :
un génome ARN simple brin de polarité positive ;
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394, **caractérisé en ce que** le procédé est choisi parmi le groupe de procédés comprenant la synthèse chimique, la réplication d'ADN, la transcription inverse et la transcription.

19. Procédé selon la revendication 18 dans lequel ladite polyprotéine a au moins 70 % d'homologie avec la polyprotéine entière choisie à partir de (i) la polyprotéine illustrée en figure 17, ou (ii) la polyprotéine d'un isolat viral à partir du génome à partir duquel ont été préparés les ADNc déposés dans une banque d'ADNc lambda gt-11 auprès l'ATCC (American Type Culture Collection) sous le numéro d'accès 40394.

20. Procédé selon la revendication 18 dans lequel ladite polyprotéine a au moins 80 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

21. Procédé selon la revendication 18 dans lequel ladite polyprotéine a au moins 90 % d'homologie avec la polyprotéine entière choisie à partir de (i) la polyprotéine illustrée en figure. 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

22. Procédé selon l'une quelconque des revendications 18 à 21 dans lequel ladite séquence génomique du VHC ou le complément de celle-ci est compris(e) entre les nucléotides -319 et 1348 ou entre les nucléotides -319 et -1 d'un génome du VHC.

23. Procédé selon l'une quelconque des revendications 18 à 22 dans lequel ladite séquence génomique du VHC ou le complément de celle-ci est choisi(e) à partir du groupe de séquences présent sur les figures 11, 12 et 17.

24. Procédé selon l'une quelconque des revendications 18 à 23 dans lequel le polynucléotide est de l'ADN.

25. Procédé. selon l'une quelconque des revendications 18 à 24 dans lequel le polynucléotide est une sonde ou une amorce pour la synthèse d'ADN.

26. Procédé selon la revendication 25 dans lequel la sonde est marquée.

27. Procédé de détection d'acides nucléiques du VHC dans un échantillon qui comprend les étapes consistant à :
a. fournir un polynucléotide tel que défini selon l'une quelconque des revendications 18 à 26 ;
b. faire réagir ledit polynucléotide avec ledit échantillon dans des conditions qui permettent la formation sélective de duplexes polynucléotidiques entre ledit polynucléotide et tout acide nucléique du VHC ou tout ADNc du VHC dans ledit échantillon ; et
c. détecter tout duplex polynucléotidique contenant ledit polynucléotide.

28. Procédé d'amplification d'un polynucléotide du VHC dans un échantillon qui comprend les étapes consistant à :
a amplifier le polynucléotide du VHC en utilisant une ADN polymérase et au moins un polynucléotide défini selon l'une quelconque des revendications 18 à 25 ; et
b. détecter le polynucléotide amplifié.

29. Procédé selon la revendication 27 ou 28 dans lequel ledit échantillon est du sang, du sérum ou du plasma humain.

30. Procédé de préparation d'un vecteur recombinant comprenant une séquence polynucléotidique qui code un polypeptide tel que défini selon l'une quelconque des revendications 1 à 9 dans lequel ladite séquence qui code ledit polypeptide est liée de manière fonctionnelle à une séquence contrôle capable d'exprimer le polypeptide, **caractérisé en ce que** ledit procédé comprend l'étape consistant à obtenir des fragments codant le polypeptide souhaité à partir de clones d'ADNc par digestion de restriction ou synthèse chimique de ladite séquence polynucléotidique et à ligaturer ladite séquence polynucléotidique dans un vecteur.

31. Procédé selon la revendication 30 dans lequel ledit polynucléotide est de l'ADN.

32. Procédé de préparation d'une cellule hôte transformée par un vecteur recombinant comme préparé selon la revendication 30 ou 31 comprenant les étapes consistant à :
a. fournir une cellule hôte capable de transformation ;
b. fournir le vecteur recombinant ; et
c. incuber (a) avec (b) dans des conditions qui permettent la transformation de la cellule hôte par le vecteur.

33. Procédé de fabrication d'un polypeptide du VHC qui comprend l'incubation d'une cellule hôte préparée selon la revendication 32 dans des conditions qui permettent l'expression de ladite séquence qui code un polypeptide, et la récupération du polypeptide.

34. Procédé de production d'une sonde polynucléotidique comprenant la synthèse chimique d'un polynucléotide tel que défini selon l'une quelconque des revendications 18 à 26.

35. Procédé de préparation d'un approvisionnement d'échantillons biologiques humains avant usage ledit procédé comprenant les étapes consistant à :
(a) fournir un approvisionnement d'échantillons biologiques humains ; et
(b) retirer dudit approvisionnement d'échantillons biologiques humains les échantillons contenant :
(i) des polynucléotides capables de détection dans un procédé selon l'une quelconque des revendications 27 ou 28 ;
ou (ii) des anticorps capables de produire des complexes polypeptide-anticorps détectables dans un procédé d'essai immunologique selon l'une quelconque des revendications 11 à 17.

36. Procédé selon la revendication 35 dans lequel lesdits échantillons biologiques humains sont du sang, du plasma ou du sérum.

37. Polypeptide sous forme essentiellement isolée comprenant une séquence contiguë issue d'une polyprotéine du virus de l'hépatite C (VHC) d'au moins 10 acides aminés, dans lequel ladite séquence contiguë est comprise entre les acides aminés 1 et 449 d'une polyprotéine du VHC, dans lequel le VHC se **caractérise par** :
un génome simple brin de polarité positive ;
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

38. Polypeptide selon la revendication 37 dans lequel ladite polyprotéine a au moins 70 % d'homologie avec la polyprotéine entière choisie à partir de (i) la polyprotéine illustrée en figure 17, ou (ii) la polyprotéine d'un isolai viral à partir du génome duquel ont été préparés les ADNc déposés dans une banque d'ADNc lambda gt-11 auprès de l'ATCC (American Type Culture Collection) sous le numéro d'accès 40394.

39. Polypeptide selon la revendication 37 dans lequel ladite polyprotéine a au moins 80 % d'homologie avec la polyprotéine entière choisie à partir de (i) la polyprotéine illustrée en figure 17, ou (ii) la polyprotéine d'un isolai viral à partir du génome duquel ont été préparés les ADNc déposés dans une banque d'ADNc lambda gt-11 auprès de l'ATCC (American Type Culture Collection) sous le numéro d'accès 40394.

40. Polypeptide selon la revendication 37 dans lequel ladite polyprotéine a au moins 90 % d'homologie avec l'entière polyprotéine choisie à partir de (i) la polyprotéine illustrée en figure 17, ou (ii) la polyprotéine d'un isolat viral issu du génome à partir duquel étaient préparés les ADNc déposés dans une bibliothèque d'ADNc lambda gt-11 avec l'ATCC (American Type Culture Collection) sous le numéro d'accès 40394.

41. Polypeptide selon l'une quelconque des revendications 37 à 40 dans lequel ladite séquence contiguë est choisie à partir des acides aminés 1 à 84 ou à partir des acides aminés 9 à 177 d'une polyprotéine du VHC.

42. Polypeptide selon l'une quelconque des revendications 37 à 41 dans lequel ladite séquence contiguë est comprise dans les séquences choisies dans le groupe constitué par les figures 4, 11, 12 et 17.

43. Polypeptide selon l'une quelconque des revendications 37 à 42 dans lequel ladite séquence contiguë comprend au moins 15 acides aminés.

44. Polypeptide selon l'une quelconque des revendications 37 à 43 dans lequel ladite séquence contiguë est de formule AAx-AAy dans laquelle x et y désignent les numéros des acides aminés illustrés en figure 17 :
AA1-AA25,AA1-AA50;AA1-AA84;AA9-AA177;AA1-AA10; AA5-AA20;AA35-AA45;AA50-AA100;AA40-AA90;AA45-AA65; AA65-AA75;AA80-AA90;AA99-AA120;AA95-AA110;AA105-AA120; AA100-AA150;AA150-AA200;AA155-AA170;AA190-AA210; AA200-AA250;AA220-AA240;AA245-AA265;AA250-AA300; AA290-AA330;AA290-AA305;AA300-AA350;AA310-AA330; AA350-AA400;AA380-AA395;AA405-AA495;AA400-AA450; AA405-AA415;AA415-AA425;AA425-AA435;AA440-AA460; AA437-AA582.

45. Polypeptide selon l'une quelconque des revendications 37 à 44 dans lequel ledit polypeptide réagit immunoloaiquement avec des anticorps anti-VHC.

46. Polypeptide tel que défini selon l'une quelconque des revendications 37 à 45 fixé à un substrat solide.

47. Kit d'essai immunologique qui comprend un polypeptide selon l'une quelconque des revendications 37 à 46 dans un conteneur approprié.

48. Polynucléotide sous forme essentiellement isolée qui comprend une séquence génomique du VHC ou le complément de celle-ci qui est sélectivement hybridable à un polynucléotide présent entre les nucléotides -319 et 1348 d'un génome du VHC ou du complément de celui-ci, dans lequel le VHC se **caractérise par** :
un génome ARN simple brin de polarité positive ;
ledit génome comprenant un cadre ouvert de lecture (ORF) codant une polyprotéine ; et
l'intégralité de ladite polyprotéine codée ayant au moins 40 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

49. Polynucléotide selon la revendication 48 dans lequel ladite polyprotéine a au moins 70 % d'homologie avec la polyprotéine entière choisie à partir de (i) la polyprotéine illustrée en ligure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

50. Polynucléotide selon la revendication 48 dans lequel ladite polyprotéine a au moins 80 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

51. Polynucléotide selon la revendication 48 dans lequel ladite polyprotéine a au moins 90 % d'homologie avec la polyprotéine entière choisie à partir de (i) la polyprotéine illustrée en figure 17, ou (ii) la polyprotéine d'un isolat viral à partir du génome duquel ont été préparés les ADNc déposés dans une banque d'ADNc lambda gt-11 auprès de l'ATCC (American Type Culture Collection) sous le numéro d'accès 40394.

52. Polynucléotide selon la revendication 48 ou 51 dans lequel ladite séquence génomique du VHC ou le complément de celle-ci est présent(e) entre les nucléotides -319 et 1348 ou entre les nucléotides -319 et -1 d'un génome du VHC.

53. Polynucléotide selon l'une quelconque des revendications 48 à 52) dans lequel ladite séquence génomique du VHC ou le complément de celle-ci est choisi(e) à partir du groupe de séquences présent sur les figures 11, 12 et 17.

54. Polynucléotide selon l'une quelconque des revendications 48 à 53 qui est de l'ADN.

55. Polynucléotide selon l'une quelconque des revendications 48 à 54 qui est une sonde ou une amorce pour la synthèse. d'ADN.

56. Polynucléotide selon la revendication 55 dans lequel la sonde est marquée.

57. Kit d'analyse d'échantillons biologiques détectant la présence de polynucléotides du VHC qui comprend un polynucléotide selon l'une quelconque des revendications 48 à 56 dans un conteneur approprié.

58. Vecteur recombinant comprenant une séquence polynucléotidique qui code un polypeptide tel que défini selon l'une quelconque des revendications 37 à 45 dans lequel ladite séquence qui code ledit polypeptide est liée de manière fonctionnelle à une séquence contrôle capable d'exprimer le polypeptide.

59. Vecteur recombinant selon la revendication 58 dans lequel ledit polynucléotide est de l'ADN.

60. Cellule hôte transformée par le vecteur selon la revendication 58 ou 59.

61. Composition pharmaceutique comprenant :
a. un polynucléotide tel que défini selon l'une quelconque des revendications 48 à 54 ; ou
b. un polypeptide tel que défini selon l'une quelconque des revendications 37 à 45 ; et
c. un véhicule pharmaceutiquement acceptable.
